(19) Europäisches Patentamt
European Patent Office
Office européen des brevets

(11) **EP 3 588 508 A1**

(12) **EUROPEAN PATENT APPLICATION**

(43) Date of publication:
   **01.01.2020 Bulletin 2020/01**

(51) Int Cl.:
   **G16H 15/00** (2018.01)    **G16H 20/10** (2018.01)
   **G16H 70/40** (2018.01)

(21) Application number: **19182564.5**

(22) Date of filing: **26.06.2019**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**
   Designated Validation States:
   **KH MA MD TN**

(30) Priority: **29.06.2018 JP 2018125588**

(71) Applicant: SYSMEX CORPORATION
   **Kobe-shi**
   **Hyogo 651-0073 (JP)**

(72) Inventors:
   • **Watanabe, Reiko**
    **Hyogo, 651-0073 (JP)**
   • **Inoue, Fumio**
    **Hyogo, 651-0073 (JP)**
   • **Suzuki, Kenichiro**
    **Hyogo, 651-0073 (JP)**

(74) Representative: **Hoffmann Eitle**
   **Patent- und Rechtsanwälte PartmbB**
   **Arabellastraße 30**
   **81925 München (DE)**

(54) **ANALYSIS METHOD, INFORMATION PROCESSING APPARATUS, AND NON-TRANSITORY COMPUTER READABLE MEDIUM**

(57)    Disclosed is an analysis method for analyzing a nucleic acid sequence of a sample by using a computer, the analysis method including: detecting a predetermined mutation on the basis of sequence information having been read from the nucleic acid sequence; and creating, in accordance with a disease that corresponds to the sample, a report that includes information related to efficacy of a predetermined drug that corresponds to the predetermined mutation.

FIG. 1

**Description**

FIELD OF THE INVENTION

The present invention relates to an analysis method, an information processing apparatus, and a non-transitory computer readable medium.

BACKGROUND

In association with development of gene test technologies in recent years, there is increasing expectancy for individualized medical care in which gene sequences of each patient are analyzed so as to appropriately select a therapeutic strategy for the patient in consideration of characteristics such as the genetic background, the physiological condition, and the state of the disease of the patient.

In recent years, gene panel tests that can comprehensively check abnormalities in a plurality of genes by use of next-generation sequencers have been developed, and such gene panel tests are expected to play an important role in individualized medical care.

Furthermore, recently, companion diagnostics (abbreviated as "CDx") which predicts efficacy of certain pharmaceutical agents is attracting attention. It is also expected that CDx will be conducted by use of results obtained by comprehensively checking abnormalities in a plurality of genes in a gene panel test.

Japanese Laid-Open Patent Publication No. 2015-200678 describes a system that determines whether or not a gene, a gene expressed protein, or the like exhibits any change when compared with a normal reference, and that identifies a drug therapy capable of interacting with the gene, the gene expressed protein, or the like exhibiting the change.

However, in the case of the system described in Japanese Laid-Open Patent Publication No. 2015-200678, it is difficult for a medical institution and a medical professional to discern whether the results on the gene mutations provided from a test institution that has performed a gene panel test having been approved to be used for CDx are related to CDx.

In particular, in gene panel tests in recent years, abnormalities in a plurality of genes can be comprehensively checked as a batch, and thus, the result of the gene panel test includes a very large amount of information. However, all of the plurality of genes having been tested are not necessarily to be used for CDx. If a predetermined abnormality has been detected in a predetermined gene, the detected predetermined abnormality is to be used for CDx. In addition, even when such a gene abnormality has been detected, the detection result of the gene abnormality is not to be used for CDx, depending on the type of the disease. Therefore, if a medical professional viewing a gene panel test is to discern a gene mutation actually applicable to companion diagnostics from among a large number of pieces of gene information included in a test result, and decide a therapeutic strategy effective for the mutation and a pharmaceutical agent to be used, a very-high-level of expertise is required.

SUMMARY OF THE INVENTION

The scope of the present invention is defined solely by the appended claims, and is not affected to any degree by the statements within this summary.

In order to solve the above problem, an analysis method according to one aspect of the present invention is for analyzing a nucleic acid sequence of a sample by using a computer (information processing apparatus 1), and includes: detecting (specifying) a predetermined mutation on the basis of sequence information having been read from the nucleic acid sequence (step S1, S1112); and creating, in accordance with a disease that corresponds to the sample, a report that includes information related to efficacy of a predetermined drug that corresponds to the predetermined mutation (step S3, S1114).

According to the above configuration, a predetermined mutation is detected (specified) in a nucleic acid sequence of a sample, and a report that includes information related to efficacy of a predetermined drug that corresponds to a predetermined mutation is created in accordance with a disease that corresponds to the sample. Accordingly, for example, on the basis of a result of a panel test using a gene panel that allows batch analysis of a plurality of abnormalities being present in a plurality of genes and related to a plurality of diseases, it is possible to create a report that includes information related to the efficacy of a drug applicable to companion diagnostics (CDx). The report created in this manner allows easy discernment of the predetermined mutation applicable to CDx in the result of the gene panel test. If such a report is provided to a medical institution or the like, it is possible to make more effective the individualized medical care and the use of information related to the efficacy of the drug applicable to CDx based on detection of the predetermined mutation.

The analysis method may further include identifying the disease that corresponds to the sample, and the report that includes the information related to the efficacy of the predetermined drug that corresponds to the predetermined mutation may be created in accordance with identification of a predetermined disease.

The analysis method may further include identifying the disease that corresponds to the sample, and the report that includes the information related to the efficacy of the predetermined drug that corresponds to the predetermined mutation may be created in accordance with detection of the predetermined mutation in a predetermined disease.

The analysis method may further include determining presence or absence of the predetermined mutation related to the efficacy of the predetermined drug and another mutation not related to the efficacy of the predetermined drug, on the basis of the sequence information.

For example, when a predetermined mutation has been detected in a sample collected from a subject having cancer or the like, if this analysis method is employed, it is possible to create a report indicating information related to the efficacy of a drug that is applicable to CDx and that corresponds to the predetermined mutation.

The report according to the analysis method may be created such that the predetermined mutation related to the efficacy of the predetermined drug and another mutation not related to the efficacy of the predetermined drug may be distinct from each other.

The report according to the analysis method may be created such that the predetermined mutation and the information related to the efficacy of the predetermined drug are associated with each other.

The report according to the analysis method may be created so as to include a first region for showing the predetermined mutation related to the efficacy of the predetermined drug and a second region for showing another mutation not related to the efficacy of the predetermined drug.

In the report according to the analysis method, a first report that indicates the predetermined mutation related to the efficacy of the predetermined drug and a second report that indicates another mutation not related to the efficacy of the predetermined drug may be separately created.

The report according to the analysis method may be created such that an icon that indicates presence of relation to the efficacy of the predetermined drug is associated with the predetermined mutation.

The report according to the analysis method may include information indicating that the predetermined mutation is usable in selection of a subject to whom the predetermined drug has a possibility of being effective.

The report according to the analysis method may be created in accordance with a quality evaluation index satisfying a predetermined criterion.

The quality evaluation index according to the analysis method may indicate accuracy of reading of each base in the sequence information performed by a sequencer (2).

The quality evaluation index according to the analysis method may indicate a depth of the sequence information having been read from the nucleic acid sequence.

The quality evaluation index according to the analysis method may indicate variation in a depth of the sequence information having been read from the nucleic acid sequence.

The sequence information according to the analysis method may be information read from a cluster group of the nucleic acid sequence having been amplified on a flow cell, and the quality evaluation index may indicate a degree of closeness between clusters in the cluster group.

The report according to the analysis method may be created in accordance with the quality evaluation index not satisfying the predetermined criterion such that the report includes information indicating that an analysis result of the nucleic acid sequence is reference information.

In the analysis method, a report that does not include the information related to the efficacy of the predetermined drug may be created in accordance with the quality evaluation index not satisfying the predetermined criterion.

The report according to the analysis method may be created in accordance with the quality evaluation index not satisfying the predetermined criterion such that the report includes information indicating that the detected predetermined mutation is not usable in selection of a subject to whom the predetermined drug has a possibility of being effective.

In the analysis method, the predetermined mutation may be substitution, insertion, deletion, or polymorphism of nucleotide, copy number abnormality of genes or fusion gene.

In the analysis method, the sequence information may be read from an exon region of the nucleic acid sequence.

In the analysis method, the sequence information may be read from an exon region having at least 10 Mb (10 million bases) or greater.

The analysis method may obtain the sequence information via a communication line.

The analysis method may include obtaining information corresponding to the sample on the basis of an input from an operator of a computer (information processing apparatus 1).

The analysis method may include obtaining, on the basis of identification information related to the sample, information related to the disease that corresponds to the sample.

The analysis method may include obtaining, via a communication line, information related to the disease that corresponds to the sample.

The analysis method may include obtaining, via a communication line, information related to efficacy of a drug that corresponds to the predetermined mutation.

The analysis method may include reading the sequence information from at least one of (i) the sample corresponding to a predetermined disease and (ii) the sample corresponding to a disease other than the predetermined disease, wherein a presence of the predetermined mutation in the sample corresponding to the predetermined disease may indicate an effectiveness of treatment by the predetermined drug.

In the analysis method, in case where the predetermined mutation is detected in the sample corresponding to a predetermined disease, the report indicating that the detected predetermined mutation is indicative of an effectiveness of treatment by the predetermined drug may be created.

In the analysis method, in case where the predetermined mutation is detected in the sample corresponding to a disease other than the predetermined disease, the report indicating that the detected predetermined mutation is irrelevant to an effectiveness of treatment by the predetermined drug may be created.

In order to solve the above problem, an analysis method according to another aspect of the present invention is for analyzing a nucleic acid sequence of a sample by using a computer (information processing apparatus 1), and includes: detecting (specifying) a predetermined mutation on the basis of sequence information having been read from the nucleic acid sequence; identifying a disease that corresponds to the sample; and creating, in accordance with identification of a predetermined disease, a report that includes information related to efficacy of a predetermined drug.

In order to solve the above problem, an information processing apparatus (1) according to another aspect of the present invention is configured to analyze a nucleic acid sequence of a sample and includes a controller programmed to: obtain sequence information having been read from the nucleic acid sequence; detect a predetermined mutation in the nucleic acid sequence on the basis of the sequence information; identify a disease that corresponds to the sample; and create, in accordance with identification of a predetermined disease, a report that includes information related to efficacy of a predetermined drug that corresponds to the predetermined mutation.

In order to solve the above problem, a non-transitory computer readable medium according to another aspect of the present invention stores programs executable by a processor to: detect a predetermined mutation in a nucleic acid sequence of a sample, on the basis of sequence information having been read from the nucleic acid sequence; and create, in accordance with a disease that corresponds to the sample, a report that includes information related to efficacy of a predetermined drug that corresponds to the predetermined mutation.

In order to solve the above problem, an analysis method according to another aspect of the present invention is for analyzing a nucleic acid sequence of a sample by using a computer, and includes: detecting presence of a predetermined mutation in a predetermined set of genes on the basis of sequence information having been read from the nucleic acid sequence, wherein at least one of the genes correspond to the predetermined mutation to be used for indicating an effectiveness of treatment by a predetermined drug; and creating, if one or more of the predetermined mutations are detected in the sample corresponding to a predetermined disease, a report that includes information related to the effectiveness of treatment by the predetermined drug.

In order to solve the above problem, an analysis method according to another aspect of the present invention is for analyzing a nucleic acid sequence of a sample by using a computer, and includes: detecting presence of a predetermined mutation in a predetermined set of genes on the basis of sequence information having been read from the nucleic acid sequence, wherein at least one of the genes correspond to the predetermined mutation to be used for indicating an effectiveness of treatment by a predetermined drug; and creating a report selectively indicating (i) information of the presence of the predetermined mutation with a relation to the effectiveness of treatment by the predetermined drug, or (ii) information of the presence of the predetermined mutation without the relation to the effectiveness of treatment by the predetermined drug.

According to the above configuration, effects equivalent to those of the analysis method according to one aspect of the present invention are exhibited.

BRIEF DESCRIPTION OF THE DRAWINGS

FIG. 1 is a flow chart showing one example of the flow of a report creation process in an analysis method according to one embodiment of the present invention;

FIG. 2 is a diagram showing a configuration example of a system that includes: an information processing apparatus configured to create a report by the analysis method according to one embodiment of the present invention; and a sequencer configured to provide sequence information to the information processing apparatus;

FIG. 3 is a diagram showing a configuration example of a gene analysis system including the information processing apparatus according to one embodiment of the present invention;

FIG. 4 is a sequence diagram showing an example of major processes performed in the gene analysis system;

FIG. 5 is diagram showing an example of the structure of data stored in a management server;

FIG. 6 is a diagram showing an example of the configuration of the information processing apparatus;

FIG. 7 is a flow chart showing one example of the flow of a process for receiving an input of information related to a gene panel;

FIG. 8 is a diagram showing an example of a GUI used for inputting information related to a gene panel;

FIG. 9 is a diagram showing an example of a data structure of a gene-panel-related information database;

FIG. 10 is a diagram showing another example of a GUI used for inputting information related to a gene panel;

FIG. 11 is a diagram showing another example of a GUI used for inputting identification information for identifying a disease;

FIG. 12 is a diagram showing an example of a GUI used when an operator updates information related to a gene panel;

FIG. 13 is a diagram showing another example of a GUI used when an operator updates information related to a gene panel;

FIG. 14 is a flow chart showing one example of the flow of a process for analyzing gene sequences of a sample;

FIG. 15 is a flow chart describing one example of the procedure of pretreatment for analyzing base sequences of sample DNA by a sequencer;

FIG. 16 is a flow chart describing another example of the procedure of pretreatment for analyzing base sequences of sample DNA by a sequencer;

FIG. 17 is a flow chart describing another example of the procedure of pretreatment for analyzing base sequences of sample DNA by a sequencer;

FIG. 18 is a flow chart describing another example of the procedure of pretreatment for analyzing base sequences of sample DNA by a sequencer;

FIG. 19A is a diagram describing one example of a quality control sample;

FIG. 19B is a diagram describing one example of a quality control sample;

FIG. 19C is a diagram describing one example of a quality control sample;

FIG. 19D is a diagram describing one example of a quality control sample;

FIG. 20 is a diagram showing an example of a data structure of a gene-panel-related information database;

FIG. 21A is a diagram showing a specific example of a quality control sample;

FIG. 21B is a diagram showing a specific example of a quality control sample;

FIG. 22A is a diagram describing an example of a step of fragmenting a sample;

FIG. 22B is a diagram describing an example of a step of providing an index sequence and an adapter sequence;

FIG. 23 is a diagram describing one example of a hybridization step;

FIG. 24 is a diagram describing one example of a step of collecting DNA fragments to be analyzed;

FIG. 25 is a flow chart describing one example of the procedure of analyzing base sequences of sample DNA by a sequencer;

FIG. 26 is a diagram describing one example of a step of applying DNA fragments to a flow cell;

FIG. 27 is a diagram describing one example of a step of amplifying DNA fragments to be analyzed;

FIG. 28 is a diagram describing one example of a sequencing step;

FIG. 29 is a flow chart describing one example of the flow of analysis performed by the information processing apparatus;

FIG. 30 is a diagram showing one example of a file format for read sequence information;

FIG. 31A is a diagram describing alignment performed by a data adjustment unit;

FIG. 31B is a diagram showing one example of a format for a result of alignment performed by the data adjustment unit;

FIG. 32 is a diagram showing an example of the structure of a reference sequence database;

FIG. 33 is a diagram showing an example of known mutations to be incorporated into reference sequences (that do not indicate wild type sequences) included in the reference sequence database;

FIG. 34 is a flow chart describing in detail one example of a step of alignment;

FIG. 35A is a diagram showing one example of score calculation;

FIG. 35B is a diagram showing another example of score calculation;

FIG. 36 is a diagram showing one example of a format of a result file generated by a mutation identification unit;

FIG. 37 is a diagram showing one example of the structure of a mutation database;

FIG. 38 is a diagram showing in detail an example of the structure of mutation information in the mutation database;

FIG. 39A is a table showing correspondence relationship between genes to be analyzed and position information;

FIG. 39B is a diagram showing a state where mutations that do not correspond to information related to a gene panel are excluded from a result file;

FIG. 40 is a flow chart showing one example of a process in which a drug search unit generates a list of drugs related to mutations;

FIG. 41 is a diagram showing an example of a data structure of a drug database;

FIG. 42 is a diagram showing an example of a data structure of the drug database;

FIG. 43 is a flow chart showing one example of a process in which the drug search unit generates a list that includes information regarding drugs related to mutations;

FIG. 44 is a flow chart showing one example of a process in which, on the basis of information obtained by searching the drug database, the drug search unit determines the presence or absence of a drug having a possibility of off-label use and generates a list that includes the determination result;

FIG. 44A is a diagram showing an example of a label attached to a container storing a sample;

FIG. 44B is a diagram showing another example of a label attached to a container storing a sample;

FIG. 45 is a diagram showing an example of a data structure of the drug database;

FIG. 46 is a flow chart showing one example of a process in which the drug search unit generates a list that includes information regarding clinical trials of drugs;

FIG. 47 is a diagram showing an example of a data structure of a CDx information database;

FIG. 48 is a flow chart showing one example of a process in which a report creation unit creates a report that includes auxiliary information related to the efficacy of a predetermined drug;

FIG. 49 is a flow chart showing another example of a process in which the report creation unit creates a report that includes auxiliary information related to the efficacy of a predetermined drug;

FIG. 50 is a flow chart showing one example of a process in which the report creation unit creates a report that includes auxiliary information related to the efficacy for CDx in accordance with whether or not a gene panel used is a gene panel for CDx;

FIG. 51 is a flow chart showing another example of a process in which the report creation unit creates a report that includes auxiliary information related to the efficacy of a predetermined drug;

FIG. 52 is a diagram showing one example of a report to be created;

FIG. 53 is a diagram showing one example of a report to be created;

FIG. 54 is a diagram showing one example of a report to be created;

FIG. 55 is a diagram showing one example of a report to be created;

FIG. 56 is a diagram showing one example of a report to be created;

FIG. 57 is a diagram showing one example of a report to be created;

FIG. 58 is a diagram showing one example of a report to be created;

FIG. 59 is a diagram showing one example of a report to be created;

FIG. 60 is a diagram showing one example of a report to be created;

FIG. 61 is one example of a GUI to be displayed when a test institution logs in the gene analysis system;

FIG. 62 is a diagram showing one example of a report to be created when a contract of a test institution is of a type that does not include CDx usage; and

FIG. 63 is a diagram showing one example of a quality evaluation index.

DETAILED DESCRIPTION OF THE PREFERRED EMBODIMENTS

[Embodiment 1]

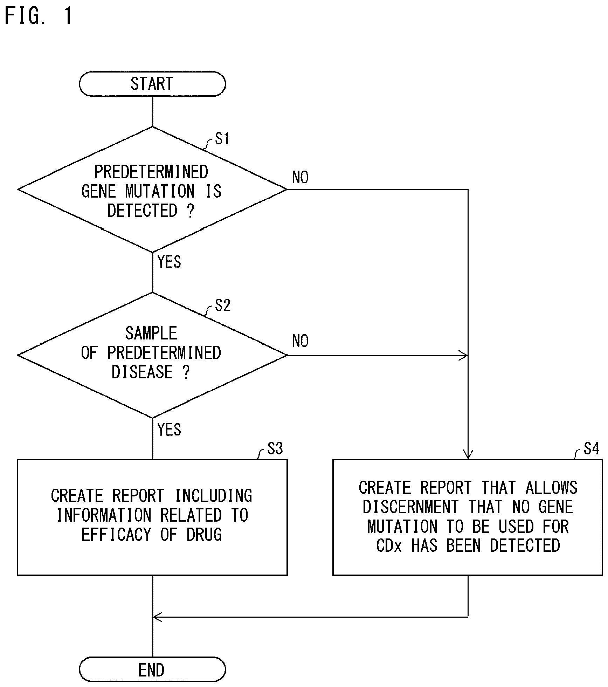

An analysis method according to one embodiment of the present disclosure is an analysis method for analyzing, by use of a computer, nucleic acid sequences of a sample collected from a subject. In this analysis method, on the basis of sequence information having been read from a nucleic acid sequence, a predetermined mutation (for example, a gene mutation to be used for CDx) is detected (specified), and in accordance with a disease corresponding to the sample, a report that indicates information related to the efficacy of a predetermined drug for the subject having the predetermined mutation is created. First, the outline of this analysis method is described with reference to FIG. 1. FIG. 1 is a flow chart showing the outline of the flow of a process by an information processing apparatus 1 performing an analysis method according to one embodiment of the present disclosure.

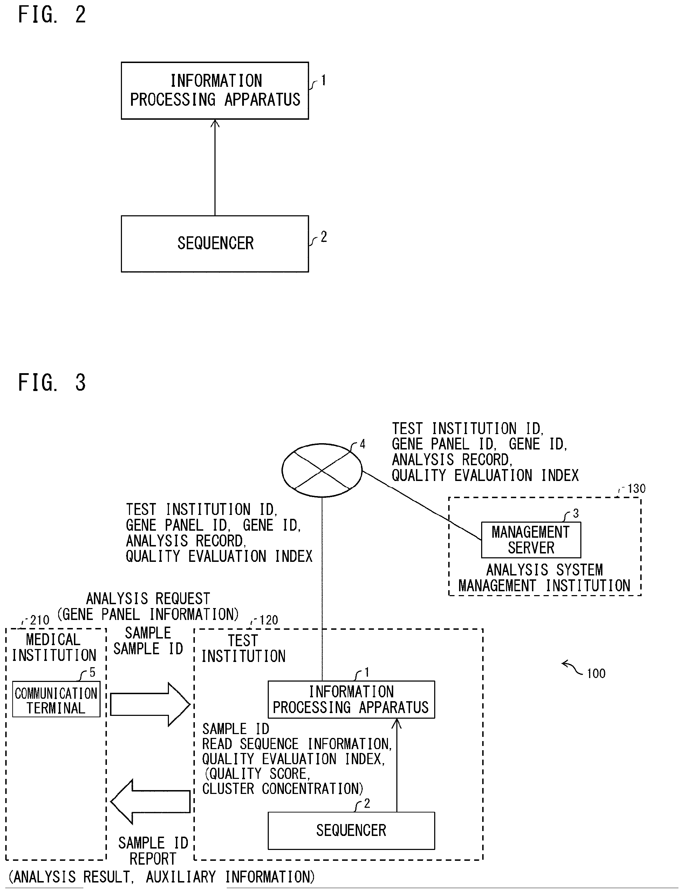

The information processing apparatus 1 is a computer that performs an analysis method according to one embodiment of the present disclosure. FIG. 2 is a diagram showing a configuration example of a system that includes: the information processing apparatus 1 which creates a report in accordance with an analysis method according to one embodiment of the present disclosure; and a sequencer 2 which provides sequence information to the information processing apparatus 1. As shown in FIG. 2, the information processing apparatus 1 analyzes sequence information generated by the sequencer 2, and analyzes the presence or absence of a gene abnormality. The sequence information generated by the sequencer 2 is stored in, for example, an information storage medium connected to the sequencer 2, and the information processing apparatus 1 obtains the sequence information from the information storage medium. The information processing apparatus 1 may receive, via a communication line, the sequence information having been read by the sequencer 2.

Here, "sequence information having been read" means a polynucleotide sequence obtained through sequencing, and "sequence information" means information of a read sequence outputted by the sequencer 2.

"Gene" includes a sequence on a genome from a start codon to a stop codon, mRNA generated from a sequence on the genome, a promoter region on the genome, and the like. "Gene mutation" means at least one of polymorphism, substitution, InDel, and the like of a gene. "InDel (Insertion and/or Deletion)" means a gene mutation that includes Insertion, Deletion, or both of Insertion and Deletion. "Polymorphism" of a gene includes SNV (single nucleotide variant, single nucleotide polymorphism), VNTR (variable nucleotide of tandem repeat, repeat sequence polymorphism), STRP (short tandem repeat polymorphism, microsatellite polymorphism), and the like.

First, in step S1, on the basis of sequence information of a nucleic acid sequence of a sample, the information processing apparatus 1 confirms whether or not a predetermined gene mutation to be used for CDx has been detected. Next, in step S2, the information processing apparatus 1 confirms whether or not the sample is a sample of a predetermined disease. For example, the information processing apparatus 1 confirms whether or not the disease associated with the sample is a predetermined disease targeted in CDx, in relation to the detected predetermined gene mutation. In the case of the sample corresponding to the predetermined disease (YES in step S2), the information processing apparatus 1 creates, in step S3, a report that includes information related to the efficacy, for the subject, of a predetermined drug associated with the predetermined gene mutation (hereinafter, also referred to simply as "auxiliary information"). Meanwhile, in the case of NO in step S1 and step S2, the information processing apparatus 1 creates, in step S4, a report that allows discernment that no gene mutation to be used for CDx has been detected. It should be noted that the processes of step S1 and step S2 are not necessarily performed in this order, and step S1 may be performed after step S2.

A "predetermined drug" means a drug or the like that is applicable to companion diagnostics (CDx). A drug applicable to CDx is defined as, in general, a drug for specifying a subject (for example, patient) for whom greater therapeutic effects are expected, a drug for specifying a subject who is more likely to have specific side effects, or a drug for appropriately conducting determination on optimization of usage/dosage or stop of administration.

By employing this analysis method, the information processing apparatus 1 can create a report that includes auxiliary information related to the efficacy of a predetermined drug, in accordance with detection of a predetermined gene mutation in a sample of a predetermined disease. For example, with respect to a result of a panel test using a gene panel that allows batch analysis of a plurality of abnormalities being present in a plurality of genes and related to a plurality of diseases, the information processing apparatus 1 creates a report that indicates information related to the efficacy of drugs applicable to companion diagnostics (CDx), in accordance with the type of each disease.

The report created in this manner allows easy discernment of gene mutations applicable to CDx, even in the case of a result of a gene panel test that comprehensively analyzes a plurality of gene abnormalities. If such a report is provided to a medical institution or the like, it is possible to make more effective the individualized medical care and the use of information related to the efficacy of drugs applicable to CDx based on detection of the gene mutations.

Hereinafter, one embodiment of the present disclosure is described in more detail.

(Application example of gene analysis system 100)

First, the outline of a gene analysis system 100 including the information processing apparatus 1 according to one embodiment of the present disclosure is described with reference to FIG. 3. FIG. 3 is a diagram showing a configuration example of the gene analysis system 100 including the information processing apparatus 1 according to one embodiment of the present disclosure.

The gene analysis system 100 is a system for analyzing gene sequence information, and includes the information processing apparatus 1, the sequencer 2, and a management server 3.

The gene analysis system 100 shown in FIG. 3 is applied in an analysis system management institution 130 which manages general analyses performed in a test institution 120. The gene analysis system 100 is also applied in the test institution 120 which analyzes a provided sample in response to an analysis request from a medical institution 210 and which provides an analysis result to the medical institution 210.

The test institution 120 tests/analyzes the sample provided from the medical institution 210, creates a report based on the analysis result, and provides the report to the medical institution 210. In the example shown in FIG. 3, the test institution 120 is provided with, but not limited to, the sequencer 2, the information processing apparatus 1, and the like. For example, the test institution 120 may include a facility in which the information processing apparatus 1 is installed, and another facility in which the sequencer 2 is installed.

The analysis system management institution 130 manages general analyses that are performed in each test institution 120 that uses the gene analysis system 100. The analysis system management institution 130 may be the same institution as the test institution 120.

The medical institution 210 is an institution in which doctors, nurses, pharmacists, and the like perform medical activities such as providing diagnosis, therapy, and dispensation to patients, and examples of the medical institution 210 include hospitals, clinics, and pharmacies.

Although FIG. 3 shows an example case where the test institution 120 and the medical institution 210 are separate institutions, the gene analysis system 100 can be suitably used in an integrated facility of the test institution 120 and the medical institution 210, such as a university hospital.

(Process in application example of gene analysis system 100)

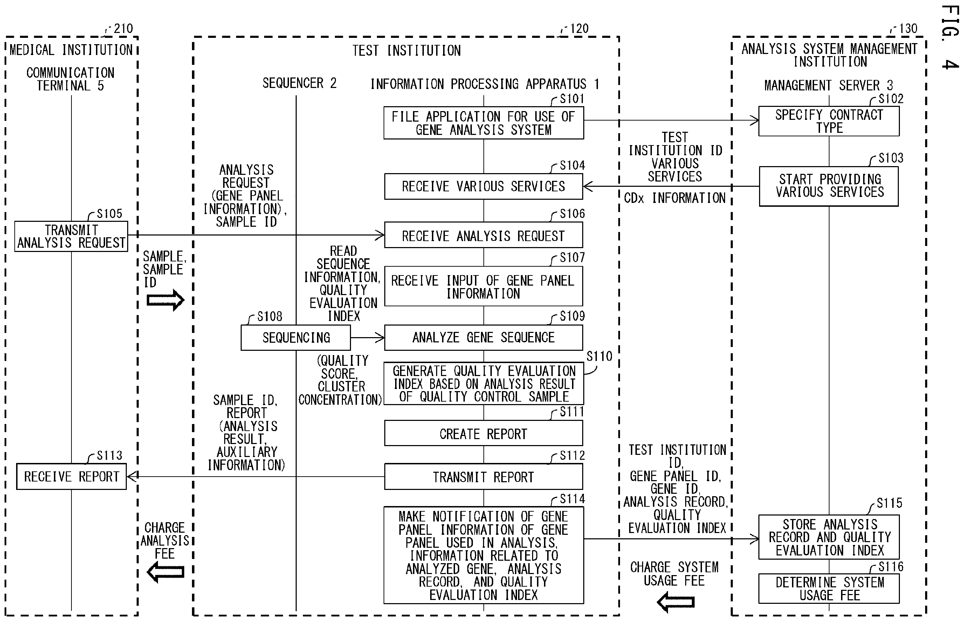

Next, the flow of processes performed in an application example of the gene analysis system 100 shown in FIG. 3 is more specifically described with reference to FIG. 4. FIG. 4 is a sequence diagram showing an example of major processes performed in the gene analysis system 100. The processes shown in FIG. 4 are only part of processes performed in each institution.

<Filing application for use of gene analysis system and start of use>

First, a test institution 120 that is going to use the gene analysis system 100 introduces the information processing apparatus 1. Then, the test institution 120 files an application for use of the gene analysis system 100 to the analysis system management institution 130 (step S101). S101 can be omitted. For example, in a case where the analysis system management institution 130 is identical to the test institution 120, S101 is omitted.

The test institution 120 and the analysis system management institution 130 can conclude in advance a desired contract with regard to use of the gene analysis system 100, from among a plurality of contract types. For example, service contents provided from the analysis system management institution 130 to the test institution 120, a method of determination of a system usage fee charged to the test institution 120 by the analysis system management institution 130, a method of payment for a system usage fee, and the like may be selected from a plurality of different contract types. The management server 3 of the analysis system management institution 130 specifies the content of the contract concluded with the test institution 120, in response to the application filed from the test institution 120 (step S102). S102 can be omitted. For example, in a case where the analysis system management institution 130 is identical to the test institution 120, S102 is omitted.

Next, the management server 3, managed by the analysis system management institution 130, provides a test institution ID to the information processing apparatus 1 of the test institution 120 having concluded the contract, and starts providing various types of services (step S103). S103 can be omitted. For example, in a case where the analysis system management institution 130 is identical to the test institution 120, S103 is omitted. In a case where the analysis system management institution 130 is identical to the test institution 120, the test institution ID and various types of services are managed by the test institution 120 itself.

The information processing apparatus 1 receives information, programs, and the like for controlling a gene sequence analysis result, a report based on the analysis result, and the like, from the management server 3. Accordingly, the test institution 120 becomes able to receive various services from the analysis system management institution 130. The information processing apparatus 1 can output an analysis result, a report, and the like that match inputted information related to a gene panel (hereinafter, also referred to as gene panel information). In a case where the analysis system management institution 130 is identical to the test institution 120, the test institution 120 itself manages the information, the programs, and the like for controlling the gene sequence analysis result, the report based on the analysis result, and the like.

In many cases, a gene panel includes a set of reagents such as a primer and a probe. The gene panel may be used for analyzing polymorphisms, such as mutation, single nucleotide polymorphism (SNP), and copy number variation (copy number abnormality) (CNV, Copy Number Polymorphism), that have occurred in gene sequences. The gene panel may be used for outputting information regarding the amount of mutations of the entire genes to be analyzed (also referred to as Tumor Mutation Burden, or the like), and for calculation of the methylation frequency.

Herein, a "gene panel" means a gene panel that allows batch analysis of a plurality of abnormalities in a plurality of genes, and that allows a test of a sample of a plurality of diseases. Such a gene panel is also referred to as "multi-panel" or a "large panel", and is used for analyzing genes that are related to a plurality of diseases. In such a gene panel, base sequences read from exon regions each having a base length of 10 Mb (10 million bases or greater) are to be analyzed.

<Analysis request to test institution 120>

In the medical institution 210, a doctor or the like collects a sample such as blood and a tissue of a lesion site of a subject as necessary. When analysis of the collected sample is requested to the test institution 120, an analysis request is transmitted from a communication terminal 5 provided in the medical institution 210, for example (step S105). When analysis of a sample is requested to the test institution 120, the medical institution 210 transmits an analysis request and provides the test institution 120 with a sample ID provided for each sample. The sample ID provided for each sample associates the sample with information regarding the subject from whom the sample has been collected (for example, patient ID), and identification information for identifying the disease of the subject (for example, disease name and disease ID). A subject ID, a disease ID, and the like may be transmitted, together with the sample ID, from the medical institution 210 to the test institution 120.

In the following, an example case in which the medical institution 210 requests a panel test analysis to the test institution 120 is described. The panel test is not limited to a laboratory test, but includes tests for research use.

Herein, a "subject" means a human subject. However, the concept of the present disclosure can be applied to a genome derived from an organism such as any animal other than human, and is useful also in the fields such as medical care, veterinary medicine, and zoological science.

When a gene panel test is requested from the medical institution 210, a desired gene panel may be designated. Therefore, gene panel information can be included in the analysis request transmitted from the medical institution 210 in step S105 shown in FIG. 4. Here, the gene panel information may be any information that can be used for specifying a gene panel, and may be, for example, the gene panel name, the names of genes to be analyzed in the panel test, and the like.

<Analysis in test institution 120>

The information processing apparatus 1 receives the analysis request from the medical institution 210 (S106). Further, the information processing apparatus 1 receives a sample from the medical institution 210, which is the transmission source of the analysis request.

There are a plurality of gene panels that can be used in analysis that the test institution 120 is requested to perform by the medical institution 210, and a gene group to be analyzed is fixed for each gene panel. The test institution 120 can selectively use a plurality of gene panels so as to suit the purpose of the analysis. That is, with respect to a first sample provided from the medical institution 210, a first gene panel can be used in order to analyze a first gene group to be analyzed, and with respect to a second sample, a second gene panel can be used in order to analyze a second gene group to be analyzed.

The information processing apparatus 1 receives, from an operator, an input of gene panel information of a gene panel to be used in order to analyze the sample (step S107).

In the test institution 120, the received sample is subjected to pretreatment using the gene panel, and sequencing is performed by use of the sequencer 2 (step S108).

In addition, in the test institution 120, separately from usual sample sequencing, a predetermined quality control sample corresponding to a gene panel is subjected to pretreatment using the gene panel, and sequencing is performed by use of the sequencer 2 (step S108), whereby accuracy control is performed.

The result obtained by subjecting the quality control sample to a gene test including pretreatment, sequencing, sequence analysis, and the like is used as a quality evaluation index of the panel test.

Each gene panel may be associated with one or a plurality of quality control samples. Alternatively, for example, for each gene panel, a corresponding quality control sample may be prepared in advance. Further, a quality control sample may be measured individually, or may be measured together with a sample provided from the medical institution 210.

Here, the quality control sample is a sample for quality control that is used in a gene test performed for a first type gene mutation and a second type gene mutation different from the first type gene mutation. The "quality control sample" is a preparation that includes a first standard gene including the first type gene mutation and a second standard gene including the second type gene mutation.

The pretreatment can include processes from fragmentation of genes such as DNA contained in the sample to collection of the fragmented genes. The sequencing includes a process for reading the sequence of one or a plurality of DNA fragments to be analyzed that have been collected in the pretreatment. The sequence information having been read in the sequencing by the sequencer 2 is inputted as read sequence information to the information processing apparatus 1. The information processing apparatus 1 may be configured to obtain the read sequence information from the sequencer 2 via a communication line. For example, the sequencer 2 may be installed in each of the medical institution 210 and the analysis system management institution 130, and the information processing apparatus 1 may obtain read sequence information read by each sequencer 2 via a communication line 4 (network). According to this configuration, even in a test institution 120 in which the sequencer 2 is not installed, it is possible to obtain read sequence information from outside and to create a report including auxiliary information which is information related to the efficacy of drugs for the subject having a gene mutation.

The pretreatment can include processes from fragmentation of genes such as DNA contained in the sample and the quality control sample to collection of the fragmented genes.

The read sequence means a polynucleotide sequence obtained by sequencing, and means a sequence to be outputted from the sequencer 2.

The sequencing includes a process for reading the sequence of one or a plurality of DNA fragments to be analyzed that have been collected in the pretreatment. The sequence information having been read in the sequencing by the sequencer 2 is inputted as read sequence information to the information processing apparatus 1.

The sequencer 2 may output, to the information processing apparatus 1, read sequence information that includes a quality score which is a quality evaluation index for the step of reading gene sequences. The sequencer 2 may output, to the information processing apparatus 1, a cluster concentration which is a quality evaluation index for a step of amplifying DNA fragments to be analyzed. The "quality score" and the "cluster concentration" are described later.

The information processing apparatus 1 obtains the read sequence information from the sequencer 2 and analyzes gene sequences (step S109).

The quality control sample is also processed in the same step performed in the panel test for the sample from the medical institution 210, and sequence information of genes of the quality control sample is analyzed. A quality evaluation index for evaluating the quality of the panel test is generated on the basis of the result of analyzing the quality control sample.

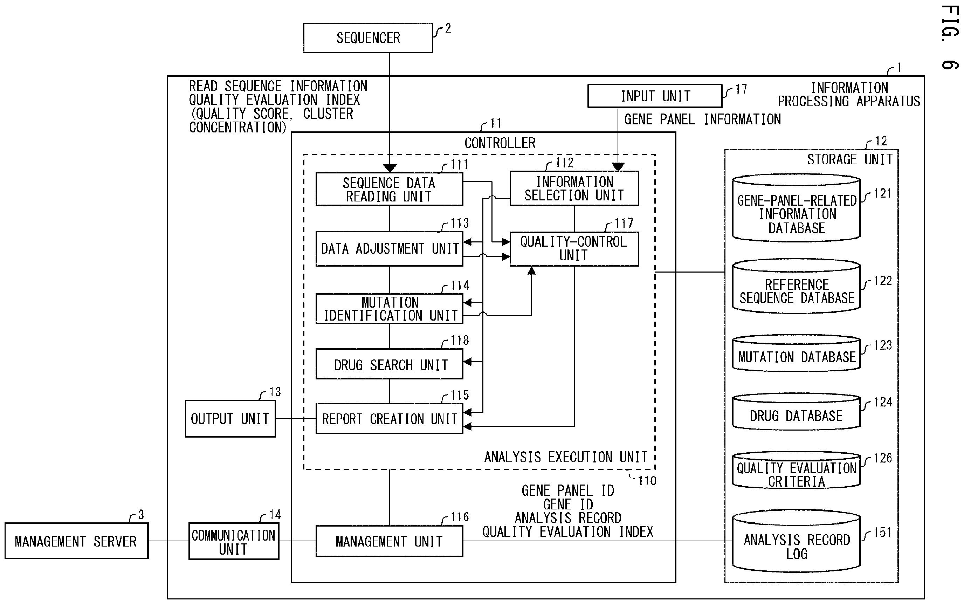

Next, the information processing apparatus 1 evaluates the quality of the panel test on the basis of the quality evaluation index generated by a quality-control unit 117 (step S110). Specifically, the information processing apparatus 1 can evaluate the quality of each panel test on the basis of a result of comparison between the generated quality evaluation index and an evaluation criterion set for each quality evaluation index stored in quality evaluation criteria 126 in FIG. 6.

The information processing apparatus 1 creates a report on the basis of the analysis result obtained in step S109, and the index generated on the basis of the result of analyzing the quality control sample (step S111), and transmits the created report to the communication terminal 5 (step S112). For example, the report may include: data of an alignment result of the read sequence information; data itself of the result of analysis by the information processing apparatus 1, such as data regarding identified gene mutations or the like; and information regarding the quality of the panel test.

The created report may be printed in the test institution 120. For example, the test institution 120 may send the created report in the form of a paper medium to the medical institution 210.

The information processing apparatus 1 of the test institution 120 that uses the gene analysis system 100 notifies the management server 3 of the gene panel information of the gene panel having been used in the analysis, information regarding the analyzed genes, an analysis record, the quality evaluation index generated for the gene test having been performed, and the like (step S114). S114 can be omitted. For example, in a case where the analysis system management institution 130 is identical to the test institution 120, S114 is omitted. In this case, the test institution 120 itself manages the analysis record, the quality evaluation index, and the like.

The management server 3 obtains a test institution ID, a gene panel ID, a gene ID, an analysis record, and the like, via, for example, the communication line 4 from the information processing apparatus 1 of each test institution 120 that uses the gene analysis system 100. The management server 3 stores the obtained test institution ID, gene panel ID, gene ID, analysis record, quality evaluation index, and the like so as to be associated with one another (step S115). S115 can be omitted. For example, in a case where the analysis system management institution 130 is identical to the test institution 120, S115 is omitted. In this case, the test institution 120 itself manages the analysis record, the quality evaluation index, and the like.

The test institution ID is information for specifying the test institution 120 that performs gene sequence analysis. The test institution ID may be an operator ID which is identification information provided to each operator who belongs to the test institution 120 that uses the information processing apparatus 1.

The gene panel ID is identification information provided for specifying a gene panel to be used in analysis of genes to be analyzed. The gene panel ID provided to the gene panel is associated with a gene panel name, the name of the company that provides the gene panel, and the like.

The gene ID is identification information provided to each gene for specifying a gene to be analyzed.

The analysis record is information regarding the analysis state of gene sequence information. For example, the analysis record may be the number of times of sequence analysis the analysis using a predetermined gene panel has been performed in the information processing apparatus 1, may be the number of genes that have been analyzed, or may be an accumulated total of the number of gene mutations that have been identified. Alternatively, the analysis record may be information regarding the amount of data that has been processed in the analysis.

The management server 3 aggregates, for each test institution 120, the analysis records in a predetermined period (for example, any period such as a day, week, month, or year) and determines a system usage fee in accordance with the aggregation result and the contract type (step S116). The analysis system management institution 130 may charge the determined system usage fee to the test institution 120, and request payment of the system usage fee to the analysis system management institution 130. S116 can be omitted. For example, in a case where the analysis system management institution 130 is identical to the test institution 120, S116 is omitted.

(Configuration example of gene analysis system 100)

The gene analysis system 100 is a system for analyzing gene sequence information, and includes at least the information processing apparatus 1 and the management server 3. The information processing apparatus 1 is connected to the management server 3 via the communication line 4 such as an intranet and the Internet.

(Sequencer 2)

The sequencer 2 is a base sequence analyzing apparatus that is used in order to read the base sequences of genes contained in a sample.

The sequencer 2 according to the present embodiment is preferably a next-generation sequencer that performs sequencing using a next-generation sequencing technology, or a third-generation sequencer. The next-generation sequencer denotes one of base sequence analyzing apparatuses which have been developed in recent years. The next-generation sequencer has a significantly improved analytical capability realized by performing, in a flow cell, parallel processing of a large amount of a single DNA molecule or a DNA template that has been clonally amplified.

Sequencing technology usable in the present embodiment can be a sequencing technology that obtains a plurality of reads by reading the same region multiple times (deep sequencing).

Examples of the sequencing technology usable in the present embodiment include sequencing technologies that can obtain a large number of reads per run, such as ionic semiconductor sequencing, pyrosequencing, sequencing-by-synthesis using a reversible dye terminator, sequencing-by-ligation, and sequencing that uses probe ligation of oligonucleotide. The present disclosure may be applied to whole genome sequencing which does not analyze the base sequences of a specific region but analyses the base sequences of the entire genome. The whole genome sequencing is applied to a gene panel to be used for analyzing genes related to a plurality of diseases, The whole genome sequencing can read base sequences from exon regions each having a base length of 10 Mb (10 million bases) or greater.

The sequencing primer to be used in sequencing is not limited in particular, and is set as appropriate on the basis of a sequence that is suitable for amplifying a target region. Reagents to be used in sequencing may also be suitably selected in accordance with the sequencing technology and the sequencer 2 to be used. The procedure from the pretreatment to the sequencing is described later by using a specific example.

(Management server 3)

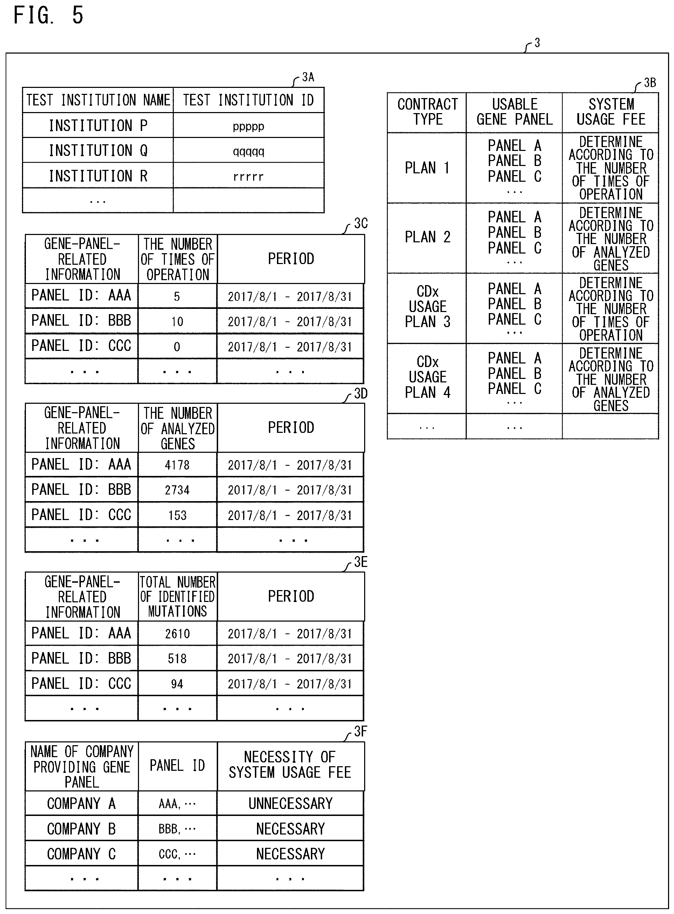

Next, data stored in the management server 3 is described with reference to FIG. 5. FIG. 5 is a diagram showing an example of the structure of data stored in the management server 3. On the basis of each piece of data shown in FIG. 5, the analysis system management institution 130 determines a system usage fee to be charged to each test institution. The management server 3 receives, from the information processing apparatus 1 via the communication line 4, information that includes: information for specifying a test institution 120 that performs gene sequence analysis (for example, test institution ID); gene panel information of the gene panel that has been used; and information regarding the state of gene sequence analysis (for example, analysis record). In FIG. 5, "gene panel A" is indicated as "Panel A", "gene panel B" is indicated as "Panel B", and so on. "Gene panel ID" is indicated as "panel ID".

In data 3A shown in FIG. 5, the name of a test institution that uses the gene analysis system 100, and a test institution ID provided to the test institution are associated with each other. In data 3B shown in FIG. 5, the type of contract concluded between the analysis system management institution 130 and a test institution 120, services to be provided to the test institution that has concluded the contract (for example, usable gene panel), and a system usage fee are associated with one another.

For example, in a case where a test institution "Institution P" has concluded a contract of "Plan 1" with the analysis system management institution 130, the analysis system management institution 130 charges the test institution P for a usage fee according to the number of times of operation. "The number of times of operation" is the number of times a panel test has been performed by the information processing apparatus 1, for example. When the test institution P starts using the gene analysis system 100, the test institution P logs in the gene analysis system 100 by using the test institution ID and a password of the test institution P. On the basis of the test institution ID inputted at the time of log-in, the management server 3 can specify the test institution name, the contract type, and the like.

"Plan 3" is a higher-order plan of "Plan 1". "Plan 3" is obtained by adding provision of auxiliary information usable for "CDx usage", to "Plan 1". Therefore, the cost for concluding a contract of "Plan 3" may be higher than the cost for concluding a contract of "Plan 1".

CDx information necessary for creating a report that includes auxiliary information related to the efficacy of drugs applicable to companion diagnostics (CDx) is provided to the test institution that has concluded the contract of "Plan 3" (see S104 in FIG. 4). For example, in a case where a test institution "Institution Q" has concluded the contract of "Plan 3" with the analysis system management institution 130, the management server 3 specifies the test institution name, the contract type, and the like on the basis of the test institution ID inputted at the time when the test institution Q has logged in the gene analysis system 100, and provides the test institution Q with auxiliary information related to the efficacy of drugs applicable to CDx. Thus, the test institution Q can provide the medical institution 210 with a report that includes auxiliary information related to the efficacy of drugs applicable to CDx.

Data 3C to 3E shown in FIG. 5 are analysis records regarding the number of times of operation that was performed, genes that were analyzed, and the total number of gene mutations that were identified, by the test institution using the gene analysis system 100 in a period from August 1, 2017 to August 31, 2017. These analysis records are transmitted from the information processing apparatus 1 to the management server 3, and are stored in the management server 3. On the basis of the data of these analysis records, the analysis system management institution 130 determines a system usage fee to be charged to each test institution. The record aggregation period is not limited to that mentioned above. The recodes may be aggregated in any period such as a day, week, month, or year.

When the analysis system management institution 130 determines a system usage fee, the system usage fee may be changed depending on whether the gene panel that was used in the test was from a company that provides (for example, produces or sells) the gene panel. In this case, it is sufficient that data 3F shown in FIG. 5 is stored in the management server 3. In data 3F shown in FIG. 5, the name of a company that provides gene panels, such as "Company A" or "Company B", a gene panel ID, and an agreement regarding the system usage fee (for example, whether a system usage fee is required or not) are associated with one another.

An example case in which "Institution P" concluded a contract of "Plan 1" with the analysis system management institution 130 and the analysis record are those shown in FIG. 5 is described. Institution P performed tests using a gene panel (gene panel ID "AAA") provided by Company A, five times, and tests using a gene panel (gene panel ID "BBB") provided by Company B, ten times. According to the data shown in FIG. 5, for the five tests using the gene panel provided by Company A, the system usage fee is not required. Therefore, for Institution P, the analysis system management institution 130 determines a system usage fee, excluding the number of times of test using the gene panel provided by Company A.

(Configuration example of information processing apparatus 1)

FIG. 6 is one example of the configuration of the information processing apparatus 1. The information processing apparatus 1 includes: a controller 11 which obtains read sequence information read by the sequencer 2, and gene panel information of a gene panel that includes a plurality of genes to be analyzed; and an output unit 13 which outputs a result of analysis, of the read sequence information, based on the gene panel information obtained by the controller 11. The information processing apparatus 1 can be configured by use of a computer. For example, the controller 11 is implemented by a processor such as a CPU (central processing unit), and a storage unit 12 is implemented by a hard disk drive.

In the storage unit 12, a program for sequence analysis, a program for generating a single reference sequence, and the like are also stored. The output unit 13 includes a display, a printer, a speaker, and the like. An input unit 17 includes a keyboard, a mouse, a touch sensor, and the like. A device may be used that has both of the functions of an input unit and an output unit, such as a touch panel in which a touch sensor and a display are integrated. A communication unit 14 is an interface that allows the controller 11 to communicate with an external apparatus.

The information processing apparatus 1 includes: the controller 11 which comprehensively controls the components of the information processing apparatus 1; the storage unit 12 which stores various kinds of data to be used by an analysis execution unit 110; the output unit 13; the communication unit 14; and the input unit 17. The controller 11 includes the analysis execution unit 110 and a management unit 116. Further, the analysis execution unit 110 includes a sequence data reading unit 111, an information selection unit 112, a data adjustment unit 113, a mutation identification unit 114, the quality-control unit 117, a drug search unit 118, and a report creation unit 115. The storage unit 12 stores a gene-panel-related information database 121, a reference sequence database 122, a mutation database 123, a drug database 124, and an analysis record log 151.

The information processing apparatus 1 creates a report that includes an analysis result corresponding to the gene panel having been used, even when a different gene panel is used for each analysis. The operator who uses the gene analysis system 100 can analyze the result of the panel test by a common analysis program irrespective of the type of the gene panel, and can create a report. Accordingly, when a panel test is performed, a bothersome operation, such as selecting an analysis program to be used for each gene panel and performing specific setting for the analysis program for each gene panel to be used, is omitted, and thus, convenience for the operator is improved.

When the operator of the information processing apparatus 1 has inputted gene panel information through the input unit 17, the information selection unit 112 refers to the gene-panel-related information database 121, and controls the algorithm of the analysis program such that the analysis program performs analysis of genes to be analyzed, in accordance with the inputted gene panel information.

Here, the gene panel information may be any information that can specify the gene panel that has been used in measurement performed by the sequencer 2. Examples of the gene panel information include the gene panel name, the names of genes to be analyzed with the gene panel, the gene panel ID, and the like.

On the basis of the gene panel information inputted through the input unit 17, the information selection unit 112 changes the analysis algorithm for performing analysis so as to correspond to the genes to be analyzed with the gene panel indicated by the gene panel information.

The information selection unit 112 outputs an instruction based on the gene panel information, to at least one of the data adjustment unit 113, the mutation identification unit 114, the drug search unit 118, and the report creation unit 115. Through this configuration, the information processing apparatus 1 can output a result of analysis of the read sequence information, on the basis of the inputted gene panel information.

That is, the information selection unit 112 is a function block that performs control so as to obtain gene panel information of a gene panel that includes a plurality of genes to be analyzed, and cause the output unit 13 to output the result of analysis of the read sequence information on the basis of the obtained gene panel information.

When genes contained in various samples are analyzed in the test institution 120 which performs panel tests, various gene panels are used in accordance with the gene groups, to be analyzed, for the respective samples.

That is, the information processing apparatus 1 can obtain first read sequence information read from a first sample by use of a first gene panel for analyzing a first gene group to be analyzed, and second read sequence information read from a second sample by use of a second gene panel for analyzing a second gene group to be analyzed.

Even when various combinations of genes to be analyzed have been analyzed by use of various gene panels, the information processing apparatus 1 can appropriately output results of analyses of read sequence information because the information processing apparatus 1 is provided with the information selection unit 112.

That is, if the operator merely selects gene panel information, without setting an analysis program to be used in analysis of read sequence information and performing analysis for each gene to be analyzed, a result of analysis of each piece of read sequence information can be appropriately outputted.

For example, when the information selection unit 112 outputs, to the data adjustment unit 113, an instruction based on the gene panel information, the data adjustment unit 113 performs an alignment process or the like reflecting the gene panel information.

In accordance with the gene panel information, the information selection unit 112 issues an instruction so that the reference sequence (reference sequence in which wild type genome sequences and mutation sequences are incorporated) to be used by the data adjustment unit 113 in mapping the read sequence information is limited only to the reference sequence for the genes that correspond to the gene panel information.

In this case, since the gene panel information has already been reflected in the result of the process performed by the data adjustment unit 113, the information selection unit 112 need not output an instruction based on the gene panel information to the mutation identification unit 114 which subsequently performs a process following the process performed by the data adjustment unit 113.

For example, in a case where the information selection unit 112 outputs an instruction based on the gene panel information to the mutation identification unit 114, the mutation identification unit 114 performs a process reflecting the gene panel information.

For example, in accordance with the gene panel information, the information selection unit 112 issues an instruction so that the region of the mutation database 123 referred to by the mutation identification unit 114 is limited to only mutations related to the genes that correspond to the gene panel information. Accordingly, the gene panel information is reflected in the result of the process performed by the mutation identification unit 114.

(Input of gene panel information)

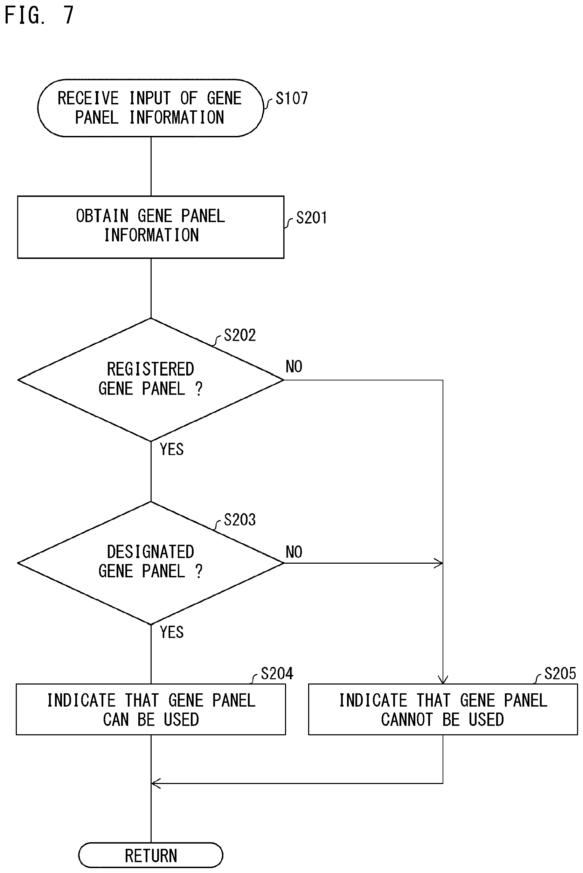

Here, a process for receiving an input of gene panel information shown in step S107 of FIG. 4 is described with reference to FIG. 7. FIG. 7 is a flow chart showing one example of the flow of a process for receiving an input of gene panel information.

Here, an example configuration is described in which the controller 11 causes the input unit 17 to display a GUI for inputting gene panel information, thereby allowing the operator to input gene panel information. Here, an example is described in which the input unit 17 is provided with a touch panel that allows the operator to perform an input on the presented GUI.

First, the controller 11 of the information processing apparatus 1 causes the input unit 17 to display a GUI for allowing the operator to select gene panel information. On the basis of the input operation onto the GUI by the operator, the gene panel information is obtained (step S201).

On the basis of information selected by the operator in the information displayed as the GUI, the information selection unit 112 searches the gene-panel-related information database 121 and reads gene panel information that corresponds to the selected information.

In addition, the information processing apparatus 1 reads gene panel information that is included in the analysis request received from the medical institution 210.

When a gene panel corresponding to the selected information is already registered in the gene-panel-related information database 121 (YES in step S202), and the gene panel matches the gene panel included in the analysis request received from the medical institution 210 (YES in step S203), the information selection unit 112 receives the input. Then, the information selection unit 112 causes the input unit 17 to display a message to the effect that the inputted gene panel can be used (step S204).

Meanwhile, when the gene panel corresponding to the selected information is not registered in the gene-panel-related information database 121, i.e., when an unregistered gene panel has been selected (NO in step S202), the information selection unit 112 causes the input unit 17 to display a message to the effect that the inputted gene panel cannot be used (step S205), and prohibits analysis from being performed by the information processing apparatus 1.

In this case, instead of the message to the effect that the gene panel cannot be used, a message that indicates an error may be displayed. The message may be, for example, "The selected gene panel is not registered." and may further include a message that urges re-input, such as "Please input gene panel information again".

When the gene panel corresponding to the selected information does not match the gene panel included in the analysis request received from the medical institution 210 (NO in step S203), the information selection unit 112 causes the input unit 17 to display a message to the effect that the inputted gene panel cannot be used (step S205), and prohibits analysis from being performed by the information processing apparatus 1.

Also in this case, instead of the message that the gene panel cannot be used, a message that indicates an error may be displayed. The message may be, for example, "The selected gene panel is different from that in the order." and may further include a message that urges re-input, such as "Please input gene panel information again".

This process can prevent performing sequencing by use of an inappropriate gene panel and performing unnecessary analysis operation, and can eliminate wasteful use of gene panels and wasteful operation of the gene analysis system 100.

(Example of GUI used for inputting gene panel information)

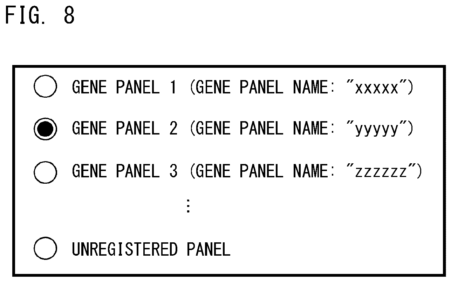

Next, some examples of the GUI for allowing the operator to input gene panel information are described with reference to FIG. 8. FIG. 8 is a diagram showing an example of a GUI to be used for inputting gene panel information.

As shown in FIG. 8, as gene panel information, a list of gene panel names such as "xxxxx" and "yyyyy" is displayed on the GUI, and the operator may be allowed to select a desired gene panel from among the gene panels in the list.

The list of gene panel names on the GUI is displayed on the basis of gene panel names of gene panels that are provided with gene panel IDs and that are already registered in the gene-panel-related information database 121.

In the GUI shown in FIG. 8, "gene panel 2 (gene panel name: "yyyyy") has been selected by the operator. Using the gene panel ID associated with the selected gene panel name "yyyyy" as a key, the information selection unit 112 searches the gene-panel-related information database 121, and obtains gene panel information that corresponds to the inputted gene panel name.

(Gene-panel-related information database 121)

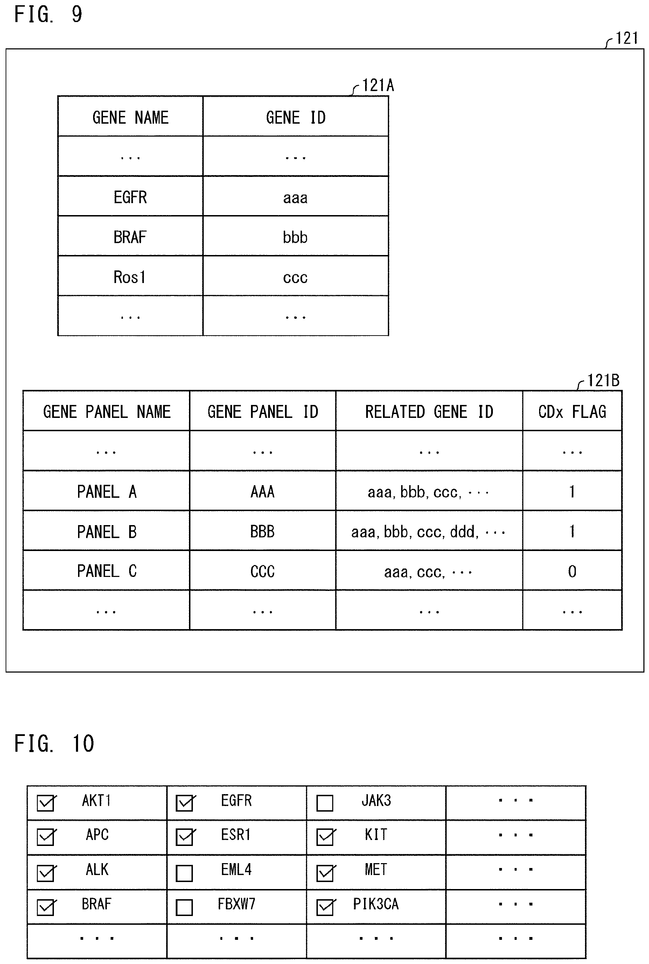

Next, data stored in the gene-panel-related information database 121 referred to by the information selection unit 112 when gene panel information has been inputted through the input unit 17 is described with reference to FIG. 9. FIG. 9 is a diagram showing an example of a data structure of the gene-panel-related information database 121.

In the gene-panel-related information database 121, as shown in data 121A in FIG. 9, the name of each gene that can be a gene to be analyzed and a gene ID provided to the gene are stored for each gene panel. Gene panels "Panel A", "Panel B", and "Panel C" are each a gene panel (so-called "large panel") that allows batch analysis of a plurality of abnormalities being present in a plurality of genes and related to a plurality of diseases.

In the gene-panel-related information database 121, as shown in data 121B in FIG. 9, the name of each selectable gene panel, a gene panel ID provided to the gene panel, gene IDs of genes to be analyzed with the gene panel (related gene ID), and a CDx flag are stored in association with one another. The CDx flag is a flag that indicates whether the gene panel is for CDx or not. The gene panel for CDx is a gene panel that allows detection of gene mutations to be used for CDx. Each gene panel may also be associated with information regarding whether or not use of the gene panel is already approved by a public institution (for example, Japanese Ministry of Health, Labour and Welfare).

As shown in FIG. 8, when the operator has selected a desired gene panel from among the gene panels presented on the GUI, the information selection unit 112 refers to the gene-panel-related information database 121 and extracts the gene panel ID and the related gene IDs that are associated with the selected gene panel name.

When genes to be analyzed have been selected from among the gene names presented on the GUI as shown in FIG. 10, the information selection unit 112 refers to the gene-panel-related information database 121 and extracts the gene IDs associated with the selected gene names, and the gene panel ID of the gene panel that includes these gene IDs as the related gene IDs.

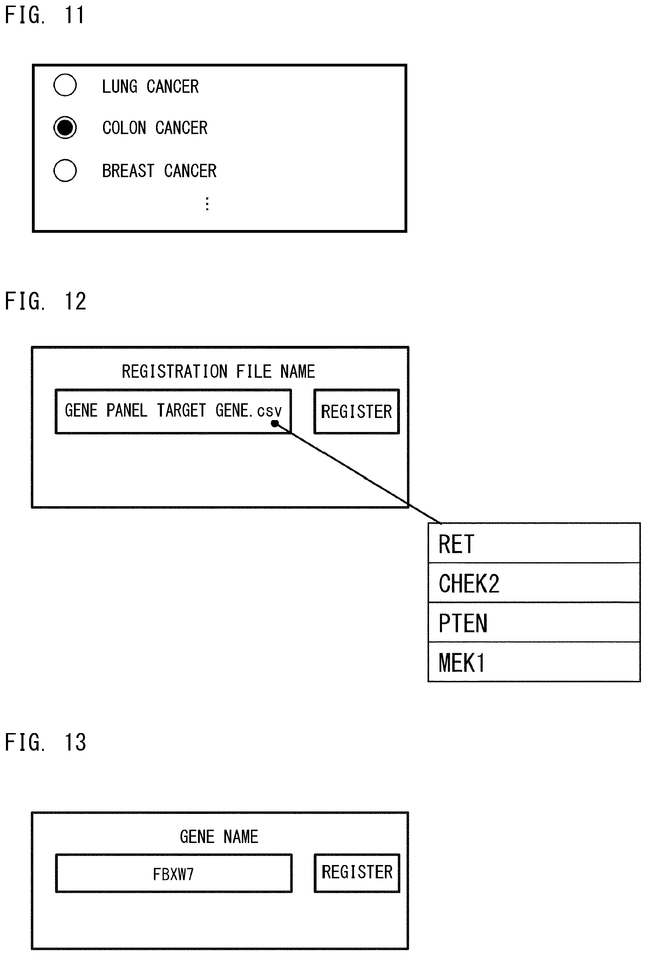

When performing a panel test using a gene panel that allows batch analysis of a plurality of abnormalities being present in a plurality of genes and related to a plurality of diseases, the disease to which each sample is related may be inputted. For example, as shown in FIG. 11, identification information for identifying a disease may be selected from a list of disease names presented on the GUI. The information selection unit 112 outputs the selected/inputted disease name (or disease ID) to the data adjustment unit 113, the mutation identification unit 114, the drug search unit 118, the quality-control unit 117, the report creation unit 115, and the like. On the basis of each sample ID, the information selection unit 112 may automatically obtain a disease name and a disease ID of the subject associated with the sample ID.

<Update of gene-panel-related information database 121>

Here, update of the information stored in the gene-panel-related information database 121 is described with reference to FIG. 12 and FIG. 13. FIG. 12 and FIG. 13 are each a diagram showing an example of a GUI to be used when the operator updates the gene-panel-related information database 121.

Update of the information stored in the gene-panel-related information database 121 can be performed by use of an update patch provided from the analysis system management institution 130 to the test institution 120.

Provision of the update patch from the analysis system management institution 130 may be targeted to test institutions 120 that have paid the system usage fee. For example, the analysis system management institution 130 may notify each test institution 120 that the condition for providing an update patch is existence of an update patch that can be provided and payment of the system usage fee. Such a notification can appropriately urge each test institution 120 to pay the system usage fee.

As shown in FIG. 12, when a plurality of genes are updated as a batch, a column for inputting a "registration file name" may be displayed, and the name of a file describing gene names, such as "gene panel target gene.csv", may be inputted in the column. In the example shown in FIG. 12, this "gene panel target gene.csv" includes a plurality of gene names of RET, CHEK2, PTEN, and MEK1.

When a "register" button is pressed after the file name has been inputted, a request for updating the information regarding the genes that correspond to the gene names included in the file is associated with the test institution ID, and is transmitted to the management server 3 via the communication unit 14. The generation of the update request and the association of the update request with the test institution ID may be performed by the controller 11 shown in FIG. 6, for example.

The analysis system management institution 130 permits the information processing apparatus 1 to download information that includes: the gene IDs provided to the gene names included in the update request received by the management server 3; and the gene panel ID provided to the gene panel for analyzing the genes.

Alternatively, as shown in FIG. 13, when the operator performs update by inputting a gene name individually, a column for inputting a "gene name" may be displayed, and a gene name such as "FBXW7" may be inputted in the column.

When a "register" button is pressed after the gene name has been inputted, a request for updating the information regarding the gene that corresponds to the gene name is associated with the test institution ID, and is transmitted to the management server 3 via the communication unit 14. The analysis system management institution 130 permits the information processing apparatus 1 to download information that includes: the gene ID provided to the gene name included in the update request received by the management server 3; and the gene panel ID provided to the gene panel for analyzing the gene.

The column for inputting a "registration file name" in FIG. 12 and the column for inputting a "gene name" in FIG. 13 may include a configuration for displaying input candidates as a suggestion.

For example, information of input candidates to be displayed is provided from the management server 3 to the information processing apparatus 1 in advance, and is stored in the storage unit 12. Then, when a click operation onto the GUI in the input column has been detected, all of the gene names that can be updated may be presented as input candidates to allow the operator to select therefrom, or a gene name that can be updated and that matches the character string inputted by the operator may be presented as an input candidate. Alternatively, for example, at the time point when the operator has inputted "E" in the column for inputting a "gene name" shown in FIG. 13, a list of gene names that can be updated such as "EGFR" and "ESR" may be displayed so as to allow the operator to select from the list. By presenting input candidates in this manner, it is possible to prevent the operator from making an erroneous input.

The gene-panel-related information database 121 may store each gene name, the gene ID of the gene, and the name of a protein coded by the gene in association with one another.

In this case, even when the inputted character string is not a gene name but a protein or the like coded by the gene, the information selection unit 112 can obtain a gene name and a gene ID that are associated with the inputted protein name, with reference to the gene-panel-related information database 121.

When a protein name has been inputted in the column for inputting a "gene name" and the register button has been pressed, a GUI may be displayed that shows a gene name associated with the protein name to allow the operator to confirm that the displayed gene name is the correct one.

(Management unit 116)

The management unit 116 stores, in the analysis record log 151, whenever necessary, an analysis record which includes the number of times of operation performed by the analysis execution unit 110, the number of analyzed genes, the total number of identified mutations, and the like, in association with the gene panel IDs and the gene IDs. At a desired frequency (for example, each day, each week, or each month), the management unit 116 reads data including the analysis record and the like from the analysis record log 151, and transmits the data in association with the test institution ID, to the management server via the communication unit 14.

(Communication unit 14)

The communication unit 14 allows the information processing apparatus 1 to communicate with the management server 3 via the communication line 4. Data transmitted from the communication unit 14 to the management server 3 can include the test institution ID, gene panel IDs, gene IDs, analysis records, update requests, and the like. Data received from the management server 3 can include gene panel information, gene names that can be updated, and the like.

(Flow of process for analyzing gene sequence of sample)

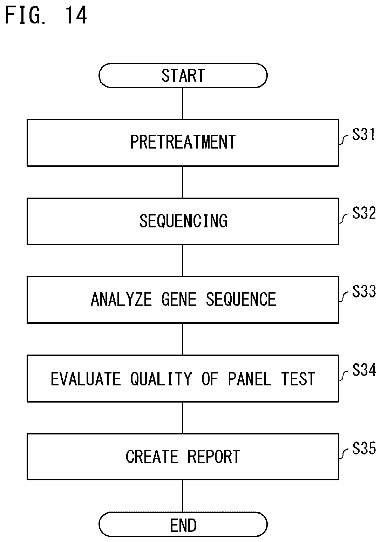

The flow of a process for analyzing gene sequences of a sample and a quality control sample is described with reference to FIG. 14. FIG. 14 is a flow chart showing one example of the flow of the process for analyzing gene sequences of a sample.

First, in step S31 in FIG. 14, pretreatment for analyzing a sequence of a gene to be analyzed is performed. The pretreatment includes processes from fragmentation of genes such as DNA included in a sample and a quality control sample to collection of the fragmented genes. When the sample provided from the medical institution 210 is, for example, a tissue and blood, a process for extracting genes (for example, DNA) from the tissue or blood is also included.

Next, in step S32, sequences of genes included in the sample and the quality control sample having been subjected to the pretreatment are read by the sequencer 2.

Specifically, step S32 is a step of reading the sequence of one or a plurality of DNA fragments, to be analyzed, which have been collected after the pretreatment. The read sequence information includes the gene sequence having been read in this step. One or a plurality of DNA fragments, to be analyzed, which have been collected after the pretreatment may also be referred to as a "library".

Subsequently, when the quality control sample has been measured, the information processing apparatus 1 analyzes the read gene sequence, and specifies the presence or absence of mutation in the sequence, the position of the mutation, the type of the mutation, and the like in step S33. By the read gene sequence being analyzed, the detected gene mutation is identified.

Next, in step S34, the quality-control unit 117 generates a quality evaluation index for evaluating the quality of the panel test. The information processing apparatus 1 may evaluate the quality of the panel test having been performed, on the basis of the generated quality evaluation index.

Lastly, the information processing apparatus 1 creates a report that includes an analysis result such as information related to the gene mutation identified in step S33, and information indicating the quality of the panel test, such as the quality evaluation index generated by the quality-control unit 117 in step S34. The created report is provided to the medical institution 210.

The type of the sequencer 2 that can be used in the present embodiment is not limited in particular, and any sequencer that can analyze a plurality of targets to be analyzed in one run can be suitably used. In the following, one example is described in which a sequencer of Illumina, Inc. (San Diego, CA) (for example, MySeq, HiSeq, NextSeq, or the like), or an apparatus that employs a similar method to that of the sequencer of Illumina, Inc. is used.

Through a combination of a Bridge PCR method and a Sequencing-by-synthesis technique, the sequencer of Illumina, Inc. can perform sequencing, with a target DNA amplified and synthesized to a huge number on a flow cell.

(a. pretreatment)

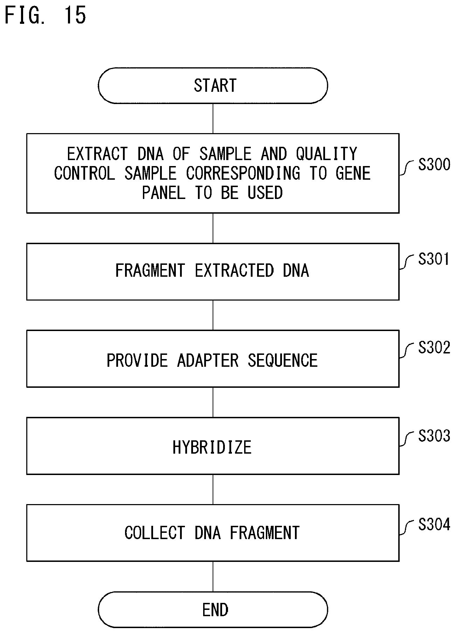

Next, the procedure of the pretreatment in step S31 in FIG. 14 is described with reference to the flow shown in FIGS. 15 to 18. FIGS. 15 to 18 are each a flow chart that describes one example of the procedure of the pretreatment for analyzing the base sequence of sample DNA by use of the sequencer 2.

When DNA is extracted from each of the sample and the quality control sample to perform sequence analysis, DNA is firstly extracted from the sample that includes genes to be analyzed, and the quality control sample that corresponds to the gene panel to be used (step S300 in FIGS. 15 to 18).

In this case, the DNA derived from the sample and the DNA derived from the quality control sample are each subjected to the process of step S301 and the subsequent steps.

Since the DNA extracted from the quality control sample is subjected to the same process as that for the DNA extracted from the sample, a quality evaluation index useful for evaluating the quality of the sequence analysis in the panel test can be generated.

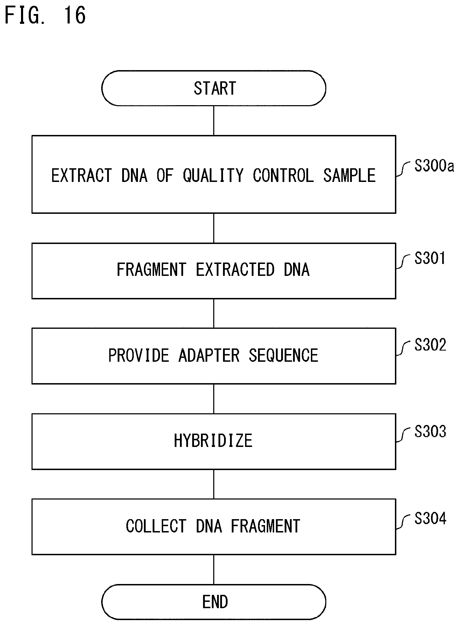

The usage of the quality control sample is not limited thereto. For example, as shown in FIG. 16, DNA of only the quality control sample may be extracted in step S300a, and subjected to the processes of step S301 and the subsequent steps.

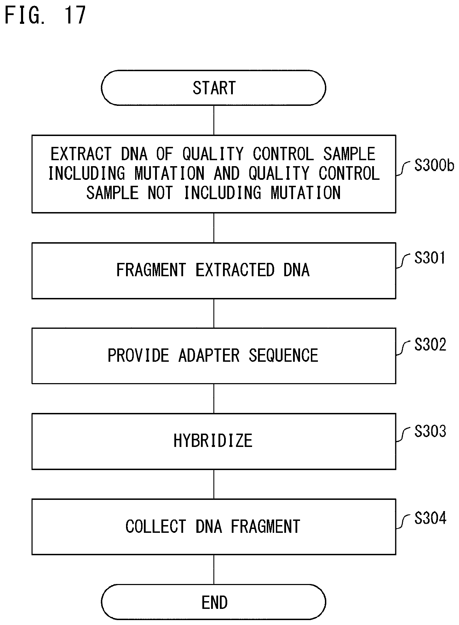

Alternatively, as shown in FIG. 17, a quality control sample that includes mutation and a quality control sample that does not include mutation are prepared as quality control samples, and DNA may be extracted therefrom (step S300b).

By comparison between a result of analysis of DNA derived from the quality control sample that includes mutation and a result of analysis of DNA derived from the quality control sample that does not include mutation, a quality evaluation index useful for evaluating the quality of the sequence analysis in the panel test can be generated.

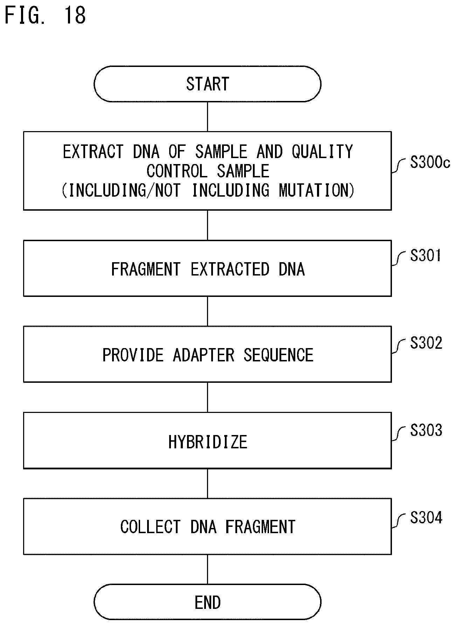

Furthermore, as shown in FIG. 18, DNA may be extracted from each of a sample that includes genes to be analyzed, a quality control sample that includes mutation, and a quality control sample that does not include mutation (step S300c). The sample that includes genes to be analyzed may be a combination of a blood sample and a tumor cell sample.

In the processes of step S301 and the subsequent steps, DNA derived from the sample and DNA derived from the quality control sample may be mixed to perform the processes of step S301 and the subsequent steps without individually processing the DNA derived from the sample and the DNA derived from the quality control sample. Accordingly, in all the processes of step S301 and the subsequent steps, the conditions for both of the samples are the same, and thus, a more accurate quality evaluation index can be generated. In addition, it is not necessary to use a part of the lanes in the flow cell used for the sequencer 2, only for the DNA fragments prepared from the quality control sample. Accordingly, the limited number of lanes can be effectively used for DNA fragments derived for the sample that includes genes to be analyzed.

In this case, (1) a reagent for appropriately fragmenting a standard gene included in the quality control sample and a gene to be analyzed in the panel test, to prepare a library, and (2) a reagent that contains RNA baits for appropriately capturing the respective DNA fragments after the standard gene included in the quality control sample and the gene to be analyzed in the panel test have been fragmented, are preferably used.

(Quality control sample)

In one embodiment, the quality control sample is a composition containing a plurality of standard genes. The quality control sample can be prepared by mixing a plurality of standard genes. A reagent obtained by these standard genes being mixed and stored in a single container can be provided as the quality control sample to the test institution 120. A plurality of standard genes that are stored in separate containers may be provided in the form of a kit as the quality control sample, to the test institution 120. The quality control sample may be in the form of a solution or may be in a solid (powder) state. When the quality control sample is provided in the form of a solution, an aqueous solvent, such as water or TE buffer, known to a person skilled in the art can be used as the solvent.

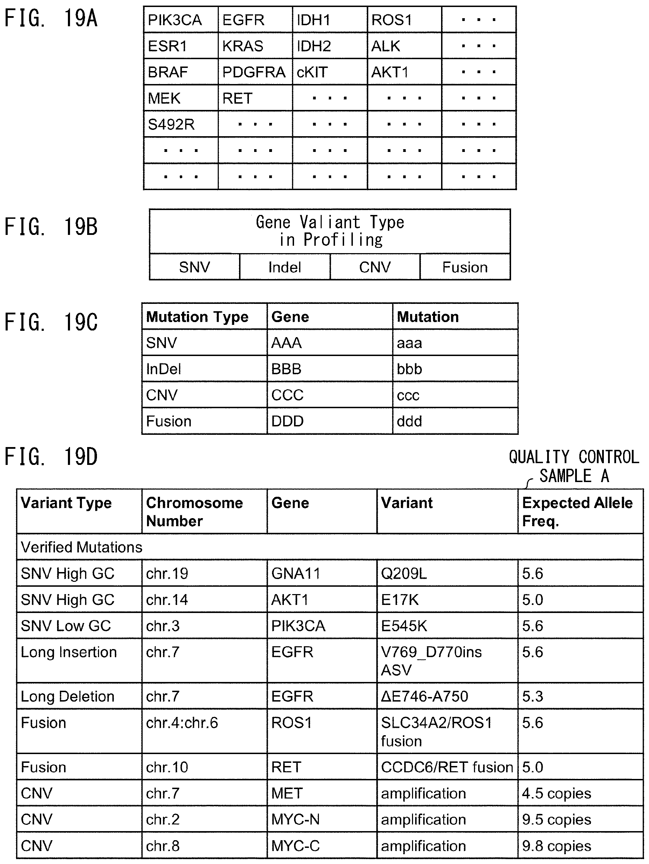

The quality control sample is described with reference to FIG. 19. FIG. 19 is a diagram describing one example of the quality control sample.

FIG. 19A shows a list of genes that can be genes to be analyzed in the panel test using the gene panel. One or a plurality of genes in this list are associated as gene to be analyzed in the gene panel (see data 121B in FIG. 9).

FIGS. 19B and 19C each show an example of types of mutations to be detected in the panel test. The types of mutations to be detected are "single nucleotide variant (SNV)", "Insertion" and "Deletion" (in the drawings, indicated as "InDel"), "copy number variation (CNV)", and "Fusion".

A quality control sample A1 corresponding to a gene panel A includes at least two of a standard gene that includes SNV, a standard gene that includes Insertion, a standard gene that includes Deletion, a standard gene that includes CNV, and a standard gene that includes Fusion. For example, the quality control sample A1 includes, as the standard gene, a partial sequence of gene A that includes "SNV" with respect to a wild type, and a partial sequence of gene B that includes "Insertion" with respect to a wild type.

FIG. 19D is an example of output of a result of analysis of the quality control sample and a result of analysis of the gene test using the gene panel A. In this example, as the analysis result of the gene panel A, SNV of GNA11, AKT1, and PIK3CA, Long insertion and Long deletion of EGFR, SLC34A2/ROS1 fusion gene, CCDC6/RET fusion gene, gene amplification of MET, gene amplification of MYC-N, and gene amplification of MYC-C are detected. The quality control sample of the gene panel A includes a standard gene that includes SNV of GNA11, a standard gene that includes SNV of AKT1, a standard gene that includes SNV of PIK3CA, a standard gene that includes Long insertion of EGFR, a standard gene that includes Long deletion of EGFR, a standard gene that includes SLC34A2/ROS1 fusion sequence, a standard gene that includes CCDC6/RET fusion sequence, a standard gene that includes gene amplification of MET, a standard gene that includes gene amplification of MYC-N, and a standard gene that includes gene amplification of MYC-C. In this example, the quality control sample includes 10 kinds of standard genes. However, the quality control sample is not limited to this example.

The first standard gene and the second standard gene included in the quality control sample may be different DNA molecules, or may be connected to each other. When the first standard gene and the second standard gene are connected to each other, the sequence of the first standard gene and the sequence of the second standard gene may be directly connected to each other, or a spacer sequence may intervene between the sequence of the first standard gene and the sequence of the second standard gene.

The spacer sequence is preferably a sequence that is less likely to be included in the sample subjected to the gene test. For example, the spacer sequence can be a sequence in which only a plurality (for example, 100) of adenine bases are consecutive.

The standard gene may be a gene that is included in the gene panel to be analyzed, or a gene that is not included in the gene panel to be analyzed. The standard gene may be a gene of a biological species for which the gene test is performed, or a gene of a different biological species. For example, when the gene test is performed for a human, the standard gene can be a gene of an animal other than a human, a plant, a bacterium, or the like.

A method for synthesizing the standard gene is not limited in particular. For example, the standard gene can be synthesized by a known DNA synthesizer. Alternatively, a gene derived from an organism, which serves as a template, is amplified by PCR and purified, whereby the standard gene may be obtained. Alternatively, PCR amplification is performed by using, as a template, a standard gene synthesized by a DNA synthesizer and purification is performed, whereby the standard gene may be obtained.

The length of the standard gene is not limited in particular. For example, the length of the standard gene can be 50 nucleotides or greater. In the case of amplification by PCR, amplification can be advantageously performed with ease if the length of the standard gene is 2000 nucleotides or less. When the standard gene is synthesized by a DNA synthesizer, up to several kbp of the standard gene can be synthesized.

The concentration of the standard gene in the quality control sample is not limited in particular. For example, the concentration of the standard gene can be approximately the same as a DNA concentration in the sample.

The standard gene in the quality control sample may be single-stranded or doublestranded. The standard gene may be linear or circular.

For example, (1) a standard gene that includes substitution mutation is prepared, (2) a standard gene that includes fusion mutation is prepared, and (3) the quality control sample and the sample are mixed together, whereby a sequence analysis sample is prepared. Next, (4) the standard genes and the sample-derived genomic DNA in the sequence analysis sample are subjected to the pretreatment (fragmentation, DNA concentration, PCR amplification using tag primer, and the like) and the sequence analysis, to obtain sequence information of the target gene. In the sequence analysis, an index for quality control is obtained, and the quality of the result of analysis of the target gene is evaluated on the basis of the index of sequence analysis of the standard DNA molecules. The operator is allowed to determine reliability of the result of analysis of the gene to be analyzed, on the basis of the result of the quality evaluation.

In the example above, in (3), the quality control sample and the sample derived from the subject are mixed together, but are not limited thereto. For example, the quality control sample and the sample may be separately subjected to the sequence analysis in (4) without mixing them.

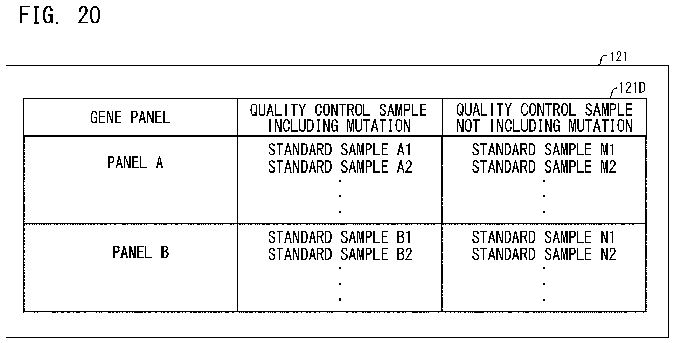

When the panel test using the same gene panel is repeatedly performed, the same quality control sample may be repeatedly used. As shown in data 121D in FIG. 20, a plurality of kinds of quality control samples including different types of mutations and different standard genes may be prepared as a plurality of quality control samples corresponding to each gene panel.

If a plurality of quality control samples having different combinations of standard genes are selectively used for each panel test, each week, or each month, the quality-control unit 117 can generate the quality evaluation index for evaluating the quality of the process for detecting mutations in the panel test, on the basis of detection of mutations of the increased number of kinds of standard genes. Therefore, the comprehensiveness of the quality control of the panel test is improved.

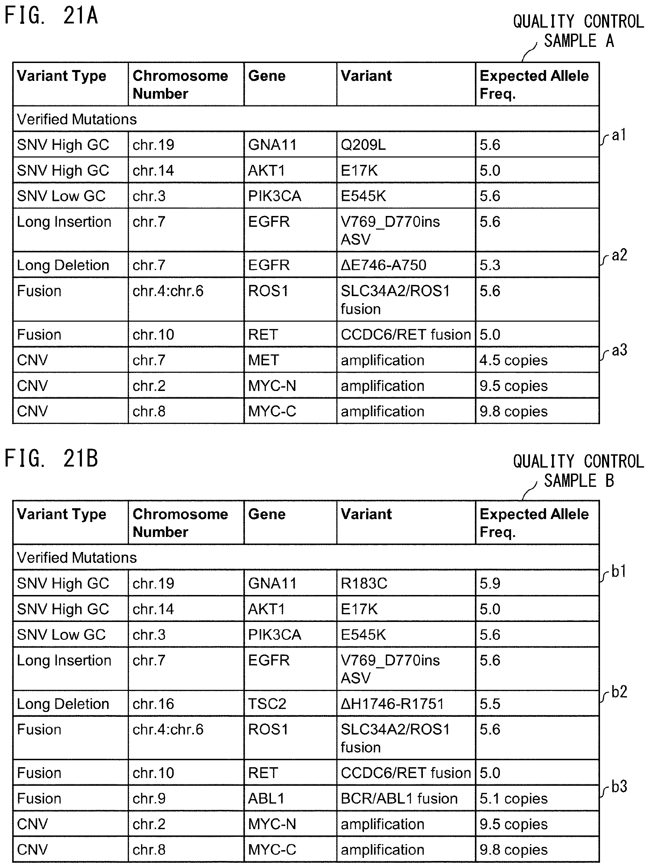

For example, FIGS. 21A and 21B show a quality control sample A and a quality control sample B which are quality control samples that correspond to a gene panel A. A standard gene a1, a standard gene a2, and a standard gene a3 included in the quality control sample A are respectively changed to a standard gene b1, a standard gene b2, and a standard gene b3, in a quality control sample B.

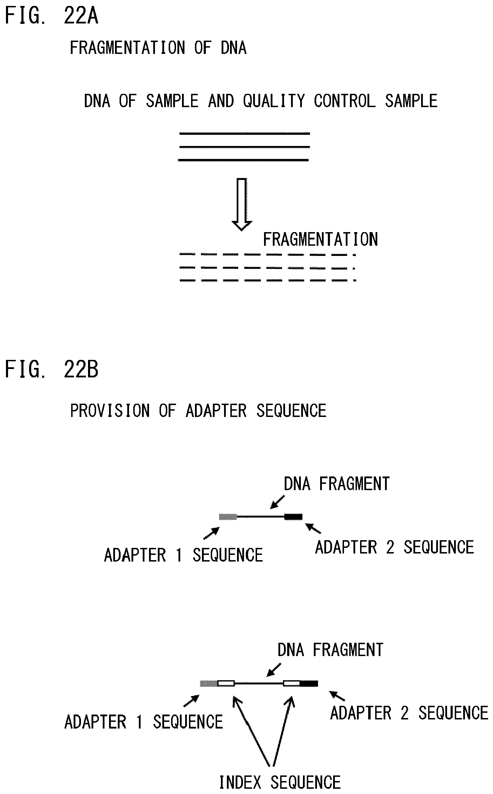

Next, as shown in FIG. 22A, a sample (genomic DNA derived from the sample, and/or standard gene) is fragmented so as to have a length with which the sequencer 2 reads the sequence (step S301 in FIG. 15 to FIG. 18). The sample DNA can be fragmented by a known method such as ultrasonication and a process using a reagent that fragments nucleic acid. Each obtained DNA fragment (nucleic acid fragment) can have a length of, for example, several tens of bp or several hundred bp.

Next, as shown in FIG. 22B, adapter sequences according to the type of the sequencer 2 and the sequencing protocol that are used are added to both ends (3' end and 5' end) of the DNA fragment obtained in step S301 (step S302 in FIG. 15 to FIG. 18). This step is indispensable when the sequencer 2 is a sequencer of Illumina, Inc. or an apparatus that employs a similar method to that of the sequencer of Illumina, Inc. However, when another type of sequencer 2 is used, this step may be omitted in some cases.

The adapter sequence is a sequence to be used for performing sequencing in the following steps. According to one embodiment, in Bridge PCR method, the adapter sequence can be a sequence that is hybridized with oligo DNA immobilized on a flow cell.

In one aspect, as shown in the upper part of FIG. 22B, the adapter sequences (for example, adapter 1 sequence and adapter 2 sequence in FIGS. 22A and 22B) may be added directly to both ends of the DNA fragment. The adapter sequences can be added to the DNA fragment by using a known technique in this technical field. For example, the DNA sequence may be blunted and ligated with the adapter sequences.

The adapter sequences may be added to the DNA fragment by using a known technique in this technical field. For example, the DNA fragment may be blunted and ligated with an index sequence, and thereafter, may be further ligated with the adapter sequences.

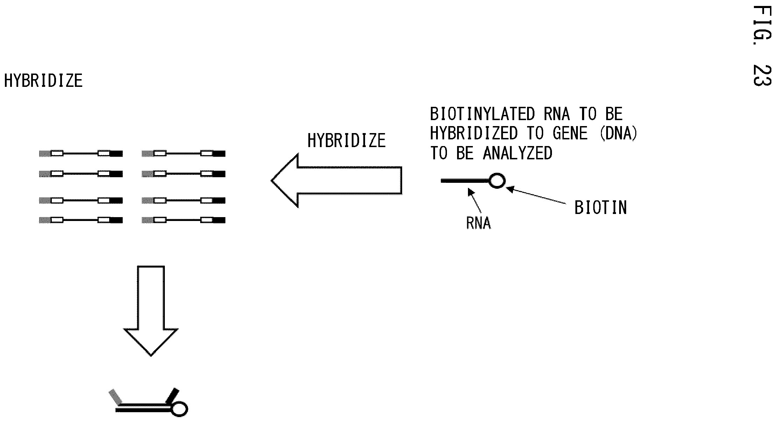

Next, as shown in FIG. 23, a biotinylated RNA bait library is hybridized with the DNA fragment to which the adapter sequences have been added (step S303 in FIG. 15 to FIG. 18).

The biotinylated RNA bait library is composed of biotinylated RNAs (hereinafter, referred to as RNA bait) that are hybridized with genes to be analyzed. The RNA bait may have any length. For example, long oligo RNA bait having about 120 bp may be used in order to enhance specificity.

In the panel test using the sequencer 2 in the present embodiment, a large number of genes (for example, 100 or more) are to be analyzed.

The reagent to be used in the panel test includes a set of RNA baits that respectively correspond to the large number of genes. When the panel is different, the number and the kinds of genes to be tested are different, and thus, the set of RNA baits included in the reagent to be used in the panel test is also different. When a gene different from a gene to be analyzed is used as a standard gene, a bait that binds to the standard gene needs to be prepared.

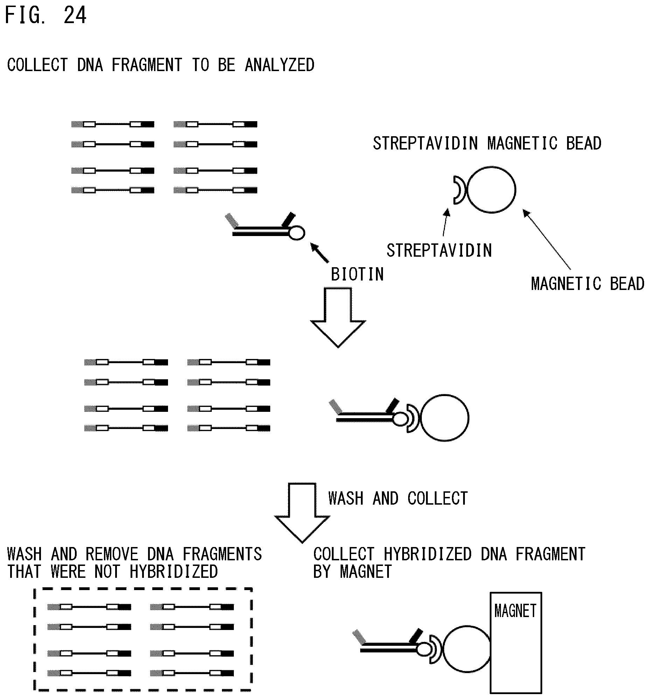

As shown in FIG. 24, DNA fragments to be analyzed are collected (step S304 in FIG. 15 to FIG. 18). Specifically, as shown in the upper part of FIG. 24, the DNA fragments hybridized with the biotinylated RNA bait library are mixed with streptavidin magnetic beads which are each composed of streptavidin and a magnetic bead bound to each other.

Accordingly, as shown in the middle part of FIG. 24, the streptavidin part of the streptavidin magnetic bead and the biotin part of the RNA bait are bound to each other. Then, as shown in the lower part of FIG. 24, the streptavidin magnetic beads are collected by a magnet, and the fragments that are not hybridized with the RNA baits (i.e., DNA fragments that are not to be analyzed) are removed by washing.

Accordingly, the DNA fragments hybridized with the RNA baits, i.e., the DNA fragments to be analyzed, can be selected and concentrated. The sequencer 2 reads the nucleic acid sequences of the DNA fragments selected by using a plurality of RNA baits, thereby obtaining a plurality of read sequences.

(Reading of read sequences by sequencer 2)

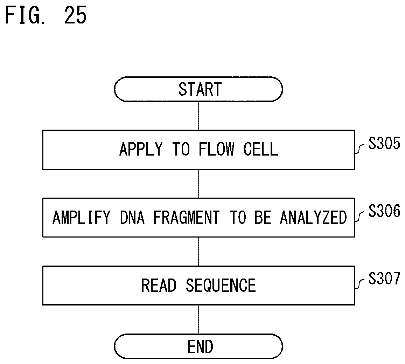

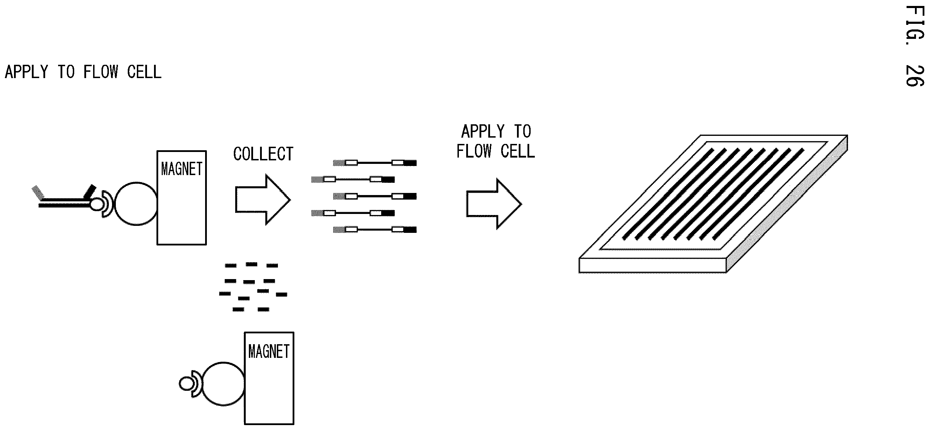

Next, with reference FIG. 26 to FIG. 28 as appropriate, the procedure of step S32 shown in FIG. 14 is described on the basis of the flow shown in FIG. 25. FIG. 25 is a flow chart describing one example of the procedure of analyzing a base sequence of sample DNA by using the sequencer 2.

As shown in FIG. 26 from the left part to the center part, the streptavidin magnetic beads and the RNA baits are removed from the concentrated DNA fragments, and the resultant DNA fragments are amplified through PCR, whereby the pretreatment is completed.

First, as shown in the right part of FIG. 26, the sequences of the amplified DNA fragments are applied to a flow cell (step S305 in FIG. 25).

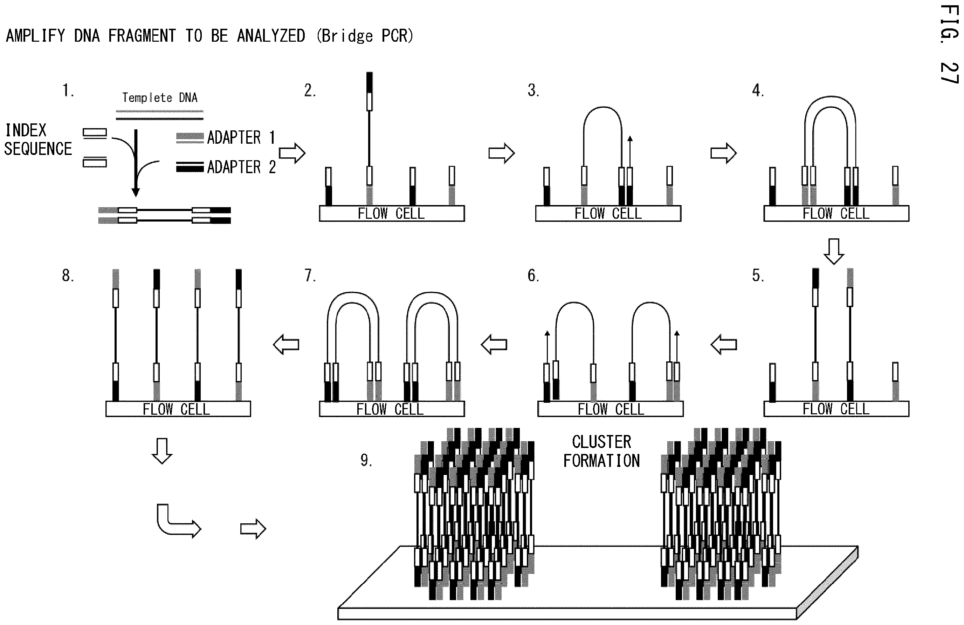

Subsequently, as shown in FIG. 27, the DNA fragments to be analyzed are amplified on the flow cell through Bridge PCR (step S306 in FIG. 25).

That is, each DNA fragment to be analyzed (for example, Template DNA in FIG. 27) is in a state where both ends of the DNA fragment have two different kinds of adapter sequences (for example, adapter 1 sequence and adapter 2 sequence in FIG. 27) added thereto through the pretreatment described above (" 1" in FIG. 27). This DNA fragment is separated into single strands, and the adapter 1 sequence at the 5' end side is immobilized on the flow cell ("2" in FIG. 27).

On the flow cell, the adapter 2 sequence on the 3' end side is immobilized in advance, and the adapter 2 sequence on the 3' end side of the DNA fragment is bound to the adapter 2 sequence on the 3' end side on the flow cell to produce a bridge-like state, whereby a bridge is formed ("3" in FIG. 27).

When DNA elongation is caused by DNA polymerase in this state ("4" in FIG. 27), and denaturation is caused, two single-stranded DNA fragments are obtained ("5" in FIG. 27).

Through repetition of the bridge formation, the DNA elongation, and the denaturation in this order, a large number of single-stranded DNA fragments are locally amplified and immobilized, whereby clusters can be formed ("6" to "9" in FIG. 27).

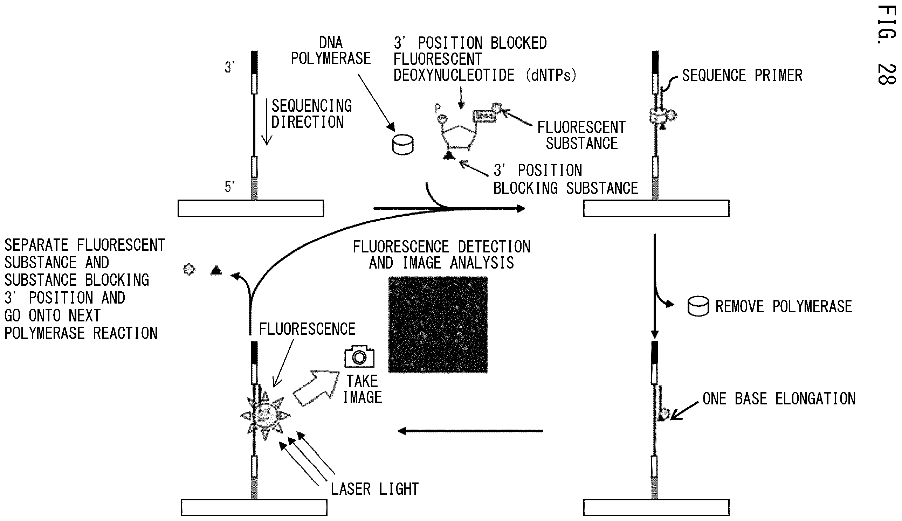

Then, as shown in FIG. 28, the single-stranded DNA forming the cluster is used as a template, and the sequence is read by sequencing-by-synthesis (step S307 in FIG. 25).

First, to the single-stranded DNA immobilized on the flow cell (the upper left part of FIG. 28), a DNA polymerase and dNTP that is labeled with fluoresce and that has the 3' end side blocked are added, (the upper center part of FIG. 28), and a sequence primer is further added thereto (the upper right part of FIG. 28).

The sequence primer may be any sequence primer that is designed so be hybridized to a part of the adapter sequence, for example. In other words, it is sufficient that the sequence primer is designed to amplify the DNA fragment derived from the sample DNA. In a case where an index sequence is added, it is sufficient that the sequence primer is designed to further amplify the index sequence.

After the sequence primer is added, one base elongation is caused, by the DNA polymerase, for dNTP labeled with fluorescence and having the 3' end blocked. Since dNTP having the 3' end side blocked is used, polymerase reaction stops when one base elongation has been realized. Then, the DNA polymerase is removed (the right middle part of FIG. 28), laser light is applied to the single-stranded DNA elongated by one base (the lower right part of FIG. 28) to excite the fluorescent substance bound to the base, and a photograph of light generated at this time is taken and recorded (the lower left part of FIG. 28).

In order to determine four kinds of bases, the photographs are taken by a fluorescence microscope for the fluorescent colors respectively corresponding to A, C, G, and T, while a wavelength filter is changed. After all the photographs have been obtained, bases are determined from the photograph data. Then, the fluorescent substance and the protecting group blocking the 3' end side are removed, and the reaction goes onto the next polymerase reaction. With this flow assumed as one cycle, the second cycle, the third cycle, and so on are performed, whereby sequencing of the entire length can be performed.

According to the technique described above, the length of the chain that can be analyzed reaches 150 bases × 2, and analysis in a unit much smaller than the unit of a picotiter plate can be performed. Thus, due to the high density, a huge amount of sequence information of 40 to 200 Gb can be obtained in one analysis.

(Gene panel)

The gene panel used for reading the read sequences by the sequencer 2 means an analysis kit for analyzing a plurality of targets to be analyzed in one run as described above. In one embodiment, the gene panel can be an analysis kit for analyzing a plurality of gene sequences related to a plurality of diseases.

When used herein, the term "kit" is intended to mean a package that includes containers (for example, bottle, plate, tube, and dish) each containing a specific material. Preferably, the kit includes instructions for using each material. When used in a context of a kit herein, "include" (is included) is intended to mean a state of being included in any of individual containers that form a kit. The kit can be a package in which a plurality of different compositions are packed into one, and the forms of the compositions can be as described above. In the case of a solution form, the solution may be contained in a container.

The kit may include a substance A and a substance B that are mixed in one container or that are in separate containers. The "instructions" indicate the procedure of applying each component in the kit to a therapy and/or diagnosis. The "instructions" may be written or printed on paper or any other medium, or may be stored in an electronic medium such as a magnetic tape, a computer readable disk or tape, or a CD-ROM. The kit can include a container that contains a diluent, a solvent, a washing liquid, or another reagent. Further, the kit may also include an apparatus that is necessary for the kit to be applied to a therapy and/or diagnosis.

In one embodiment, the gene panel may be provided with one or more of the quality control sample, reagents such as the reagent for fragmenting nucleic acid, the reagent for ligation, the washing liquid, and the PCR reagent (dNTP, DNA polymerase, etc.), and the magnetic beads, as described above. The gene panel may be provided with one or more of oligonucleotides for adding the adapter sequences to the fragmented DNA, oligonucleotides for adding the index sequence to the fragmented DNA, the RNA bait library, and the like.

In particular, the index sequence provided to each gene panel can be a sequence that is unique to the gene panel and that identifies the gene panel. The RNA bait library provided to each gene panel can be a library that is unique to the gene panel and that includes RNA baits corresponding to test genes of the gene panel.

(Sequence data reading unit 111, data adjustment unit 113, and mutation identification unit 114)

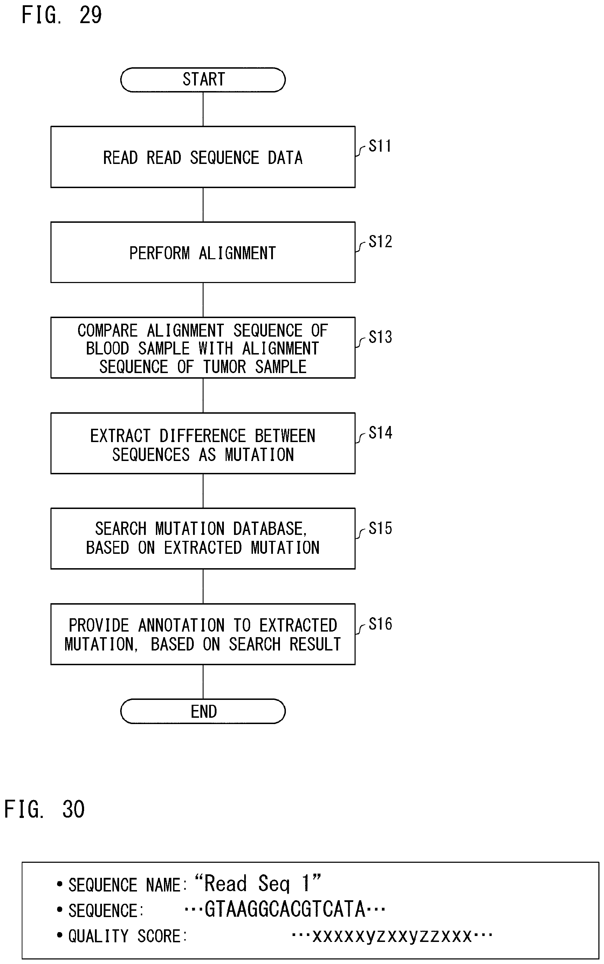

Next, the processes performed by the sequence data reading unit 111, the data adjustment unit 113, and the mutation identification unit 114 of the analysis execution unit 110 are described on the basis of the flow of the process shown in FIG. 29, with reference to FIG. 30 to FIG. 38 as appropriate.

FIG. 29 is a flow chart describing one example of the flow of analysis performed by the information processing apparatus 1. The process shown in FIG. 29 corresponds to step S109 shown in FIG. 4.

<Sequence data reading unit 111>

First, in step S11 shown in FIG. 29, the sequence data reading unit 111 reads the read sequence information provided from the sequencer 2. The sequence data reading unit 111 may obtain read sequence information read from an exon region of a nucleic acid sequence, or may obtain read sequence information read from an exon region having at least 10 Mb (10 million bases) or greater.

The read sequence information is data indicating a base sequence read by the sequencer 2. The sequencer 2 performs sequencing on a large number of nucleic acid fragments obtained by use of a specific gene panel, reads sequence information thereof, and provides the information processing apparatus 1 with the sequence information as read sequence information.

In one aspect, the read sequence information may include a quality score of each base in the sequence as well as the sequence having been read. Both the read sequence information obtained by subjecting, to the sequencer 2, an FFPE sample collected from a lesion site of a subject and the read sequence information obtained by subjecting a blood sample of the subject to the sequencer 2 are inputted to the information processing apparatus 1.

FIG. 30 is a diagram showing one example of a file format for the read sequence information. In the example shown in FIG. 30, the read sequence information includes a sequence name, a sequence, and a quality score. The sequence name may be a sequence ID or the like provided to the read sequence information outputted by the sequencer 2. The sequence indicates the base sequence read by the sequencer 2. The quality score indicates the probability of incorrect base assignment performed by the sequencer 2. Any base sequence quality score (Q) is represented by the following equation.

$$Q = -10\log_{10}E$$

In this equation, E represents an estimated value of the probability of incorrect base assignment. The greater the value of Q is, the lower the probability of the error is. The smaller the value of Q is, the greater the portion of the read that cannot be used is.

In addition, false-positive mutation assignment also increases, which could result in a lowered accuracy of the result. "False-positive" means that the read sequence is determined as having mutation although the read sequence does not have true mutation to be determined.

"Positive" means that the read sequence has true mutation to be determined, and "negative" means that the read sequence does not have mutation to be determined. For example, if the quality score is 20, the probability of error is 1/100. This means that the accuracy (also referred to as "base call accuracy") of each base in the gene sequence having been read is 99%.

<Data adjustment unit 113>

Subsequently, in step S12 shown in FIG. 29, on the basis of the read sequence information read by the sequence data reading unit 111, the data adjustment unit 113 performs alignment of the read sequence of each nucleic acid fragment included in the read sequence information.

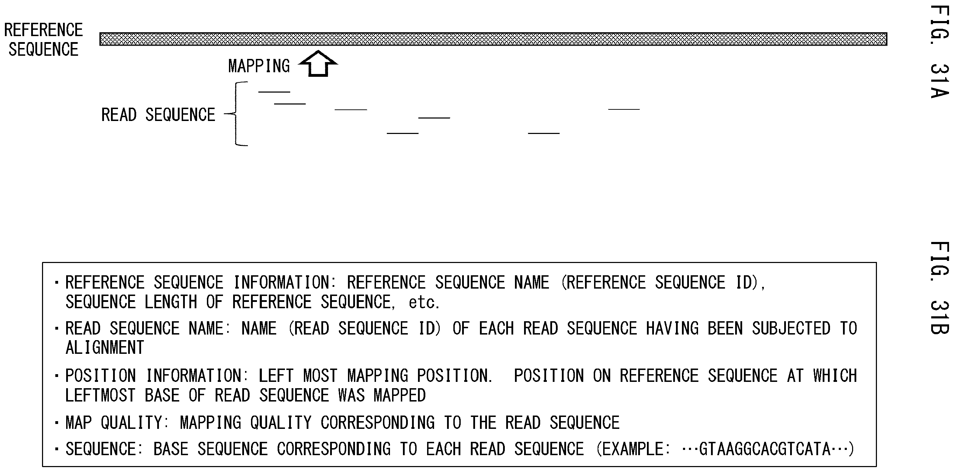

FIG. 31A is a diagram describing alignment performed by the data adjustment unit 113. The data adjustment unit 113 refers to reference sequences stored in the reference sequence database 122, and performs mapping of the read sequence of each nucleic acid fragment, to the reference sequence to be compared with the read sequence information, thereby performing alignment. In one aspect, a plurality of kinds of reference sequences that respectively correspond to the genes to be analyzed are stored in the reference sequence database 122.

The data adjustment unit 113 performs alignment for both the read sequence information obtained by subjecting an FFPE sample collected from a lesion site of the subject to the sequencer 2, and the read sequence information obtained by subjecting a blood sample of the subject to the sequencer 2.

FIG. 31B is a diagram showing one example of a format for a result of alignment performed by the data adjustment unit 113. The format for the result of alignment is not limited in particular, and may be any format that can specify the read sequence, the reference sequence, and the mapping position. As shown in FIG. 31B, the format may include reference sequence information, read sequence name, position information, map quality, and sequence.

The reference sequence information indicates the reference sequence name (reference sequence ID), the sequence length of the reference sequence, and the like in the reference sequence database 122. The read sequence name is information that indicates the name (read sequence ID) of each read sequence for which alignment has been performed. The position information indicates the position (Leftmost mapping position) on the reference sequence at which the leftmost base of the read sequence has been mapped. The map quality is information that indicates the quality of mapping corresponding to the read sequence. The sequence is information that indicates the base sequence (example: ...GTAAGGCACGTCATA...) corresponding to each read sequence.

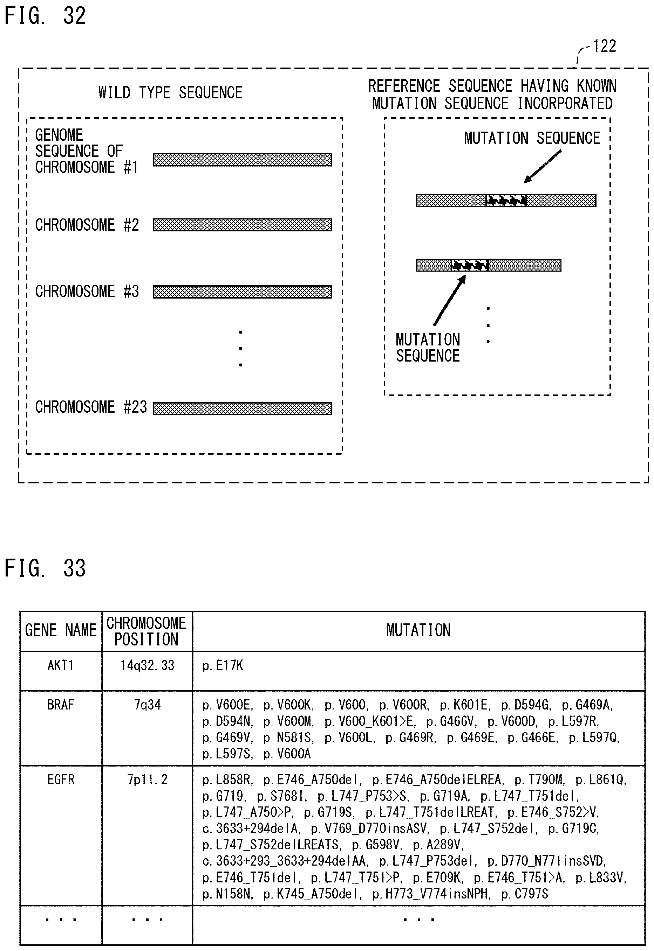

FIG. 32 is a diagram showing an example of the structure of the reference sequence database 122. As shown in FIG. 32, the reference sequence database 122 stores reference sequences (for example, genome sequences of chromosomes #1 to 23) indicating wild type sequences, and reference sequences in which known mutations are incorporated in the wild type sequences.

Further, each reference sequence in the reference sequence database 122 is provided with metadata which indicates gene panel information. For example, the gene panel information provided to each reference sequence can directly or indirectly indicate the gene, to be analyzed, that corresponds to the reference sequence.

In one embodiment, the information selection unit 112 may perform control such that, when the data adjustment unit 113 obtains a reference sequence from the reference sequence database 122, the data adjustment unit 113 refers to the inputted gene panel information and the metadata of each reference sequence, and selects a reference sequence that corresponds to the gene panel information.

For example, in one aspect, the information selection unit 112 may control the data adjustment unit 113 so as to select a reference sequence that corresponds to the gene, to be analyzed, that is specified by the inputted gene panel information. This allows the data adjustment unit 113 to perform mapping only on the reference sequence related to the gene panel having been used, and thus efficiency of the analysis can be improved.

In another embodiment, the information selection unit 112 need not perform the above-described control. In this case, the information selection unit 112 merely controls the mutation identification unit 114 or the report creation unit 115 as described later.

FIG. 33 is a diagram showing an example of known mutations to be incorporated into reference sequences (that do not indicate wild type sequences) included in the reference sequence database 122. The known mutations are gene mutations registered in external databases (for example, COSMIC, ClinVar, etc.), and as shown in FIG. 33, the chromosome positions, the gene names, and the mutations are specified. In the example shown in FIG. 33, mutations of amino acid are specified. However, mutations of nucleic acid may be specified. The types of mutations are not limited in particular, and the mutations may be various mutations such as substitution, insertion, and deletion, or the mutation may be a mutation in which a sequence of a part of another chromosome or reverse complement sequence is bound.

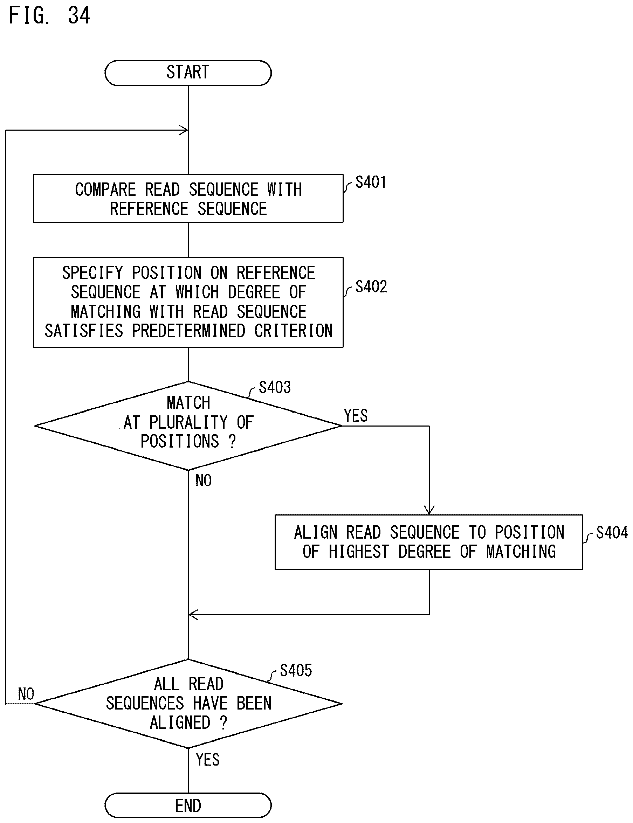

FIG. 34 is a flow chart describing in detail one example of a step of alignment in step S12 shown in FIG. 29. In one aspect, the alignment in step S12 shown in FIG. 29 is performed in steps S401 to S405 shown in FIG. 34.

In step S401 shown in FIG. 34, the data adjustment unit 113 selects a read sequence that has not been subjected to alignment from among the read sequences of nucleic acid fragments included in the read sequence information obtained by the sequence data reading unit 111, and compares the selected read sequence with a reference sequence obtained from the reference sequence database 122. In step S402, the data adjustment unit 113 specifies a position, on the reference sequence, at which the degree of matching with the read sequence satisfies a predetermined criterion. The degree of matching is a value that indicates how much the obtained read sequence information and the reference sequence match each other. Examples of the degree of matching include the number or proportion of bases that match each other.

In one aspect, the data adjustment unit 113 calculates a score that indicates the degree of matching between the read sequence and the reference sequence. The score indicating the degree of matching can be, for example, a percentage identity between two sequences. For example, the data adjustment unit 113 specifies positions at which bases of the read sequence and bases of the reference sequence are the same, obtains the number of the positions, and divides the number of the positions at which the bases are the same, by the number of bases (the number of bases in the comparison window) of the read sequence compared with the reference sequence, to calculate the percentage.

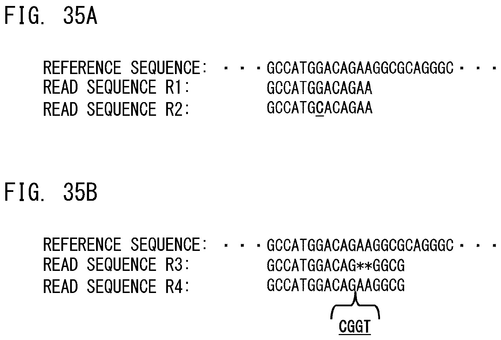

FIG. 35A is a diagram showing one example of score calculation. In one aspect, at the position shown in FIG. 35A, the score of the degree of matching between the read sequence R1 and the reference sequence is 100% because 13 bases among 13 bases of the read sequence match the bases of the reference sequence. The score of the degree of matching between the read sequence R2 and the reference sequence is 92.3% because 12 bases among 13 bases of the read sequence match the bases of the reference sequence.

In the calculation of the score indicating the degree of matching between a read sequence and a reference sequence, the data adjustment unit 113 may perform calculation such that, when the read sequence includes a predetermined mutation (for example, InDel: Insertion/Deletion) with respect to the reference sequence, a score lower than that calculated in the normal calculation is obtained.

In one aspect, for a read sequence that includes at least one of insertion and deletion with respect to the reference sequence, the data adjustment unit 113 may correct the score by, for example, multiplying the score calculated in the above-described normal calculation, by a weighting factor according to the number of bases that correspond to the insertion/deletion. The weighting factor $W$ may be calculated as, for example, $W=\{1-(1/100)\times(\text{the number of bases corresponding to insertion/deletion})\}$.

FIG. 35B is a diagram showing another example of score calculation. In one aspect, at the positions shown in FIG. 35B, the score of the degree of matching between the read sequence R3 and the reference sequence is 88% in the normal calculation because 15 bases among 17 bases of the read sequence (the symbol * indicating a deletion is calculated as one base) match the bases of the reference sequence, and the corrected score is $86\%=88\%\times0.98$. The score of the degree of matching between the read sequence R4 and the reference sequence is 81% in the normal calculation because 17 bases among 21 bases of the read sequence match the bases of the reference sequence, and the corrected score is $77.8\%=81\%\times0.96$.

The data adjustment unit 113 calculates the score of the degree of matching while changing the mapping position of the read sequence with respect to each reference sequence, thereby specifying a position on the reference sequence at which the degree of matching with the read sequence satisfies a predetermined criterion. At this time, an algorithm known in this technical field, such as dynamic programming, the FASTA method, and the BLAST method, may be used.

With reference back to FIG. 34, next, when the degree of matching with the read sequence satisfies the predetermined criterion at a single position on the reference sequence (NO in step S403), the data adjustment unit 113 performs alignment of the read sequence at the position, and when the degree of matching with the read sequence satisfies the predetermined criterion at a plurality of positions on the reference sequence (YES in step S403), the data adjustment unit 113 performs alignment of the read sequence at the position at which the degree of matching is highest (step S404).

When alignment of all the read sequences included in the read sequence information obtained by the sequence data reading unit 111 has not been performed (NO in step S405), the data adjustment unit 113 returns to step S401. When alignment of all the read sequences included in the read sequence information has been performed (YES in step S405), the data adjustment unit 113 completes the process of step S12.

<Mutation identification unit 114>

With reference back to FIG. 29, subsequently, in step S13, the mutation identification unit 114 compares the sequence (alignment sequence) of the reference sequence with which the read sequence obtained from the sample collected from the lesion site of the subject has been aligned, with the sequence of the reference sequence with which the read sequence obtained from a blood sample of the subject has been aligned.

In step S14 shown in FIG. 29, a difference between the alignment sequences is extracted as mutation. For example, if, at the same position of the same gene to be analyzed, the alignment sequence derived from the blood sample is ATCGA and the alignment sequence derived from the tumor tissue is ATCCA, the mutation identification unit 114 extracts the difference of G and C as a mutation.

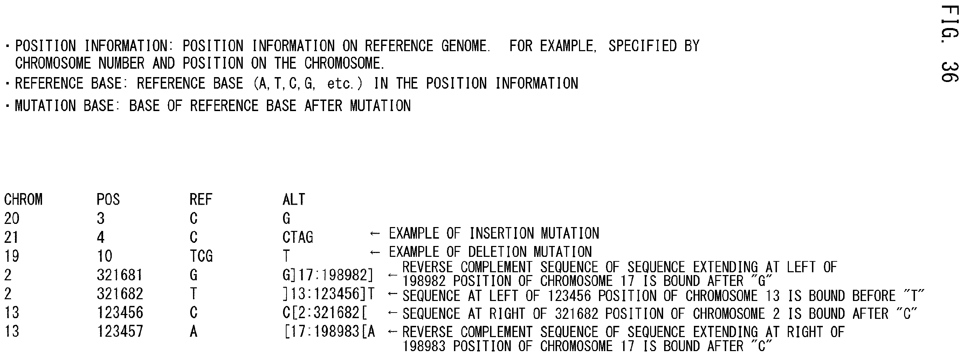

In one aspect, the mutation identification unit 114 generates a result file on the basis of the extracted gene mutation. FIG. 36 is a diagram showing one example of a format of the result file generated by the mutation identification unit 114. The format can be, for example, based on Variant Call Format (VCF).

As shown in FIG. 36, the result file contains position information, reference base, and mutation base for each extracted gene mutation. The position information indicates the position on the reference genome, and includes the chromosome number and the position on the chromosome, for example. The reference base indicates the reference base (such as A, T, C, G) at the position indicated by the position information. The mutation base indicates the base of the reference base which is present after the mutation. The reference base is the base on the alignment sequence derived from the blood sample. The mutation base is the base on the alignment sequence derived from the tumor tissue.

In FIG. 36, the mutation in which the reference base is C and the mutation base is G is an example of substitution mutation, the mutation in which the reference base is C and the mutation base is CTAG is an example of insertion mutation, and the mutation in which the reference base is TCG and the mutation base is T is an example of deletion mutation. The mutation in which the mutation base is G]17:198982], ]13:123456]T, C[2:321682[, or [17:198983[ A is an example of mutation in which a sequence of a part of another chromosome or reverse complement sequence is bound.

With reference back to FIG. 29, subsequently, in step S15, the mutation identification unit 114 searches the mutation database 123. In step S16, the mutation identification unit 114 refers to mutation information in the mutation database 123 and provides annotation to the mutation included in the result file, to identify the mutation.

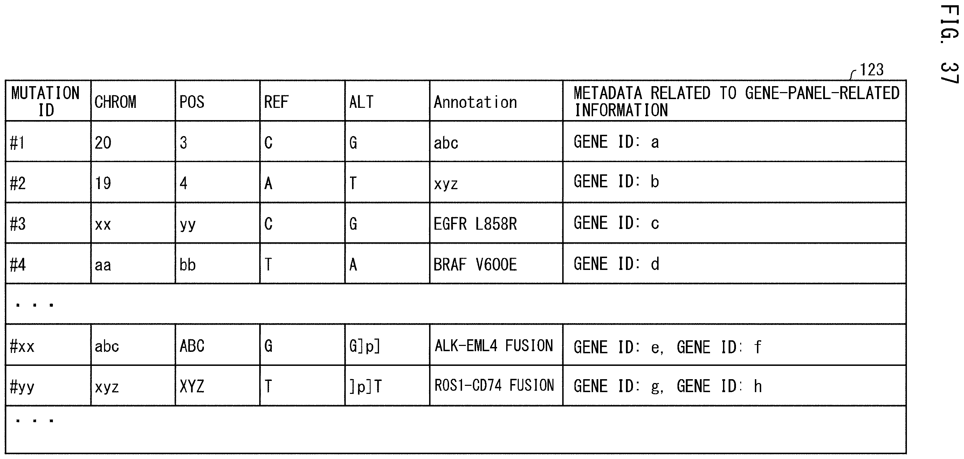

FIG. 37 is a diagram showing one example of the structure of the mutation database 123. The mutation database 123 is constructed on the basis of an external database such as COSMIC or ClinVar, for example. In one aspect, metadata related to gene panel information is provided to each piece of mutation information in the database. In the example shown in FIG. 37, a gene ID of a gene to be analyzed is provided as metadata to each piece of mutation information in the database.

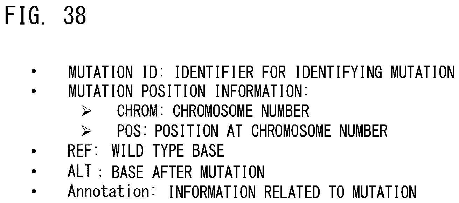

FIG. 38 is a diagram showing in detail an example of the structure of mutation information in the mutation database 123. As shown in FIG. 38, in one aspect, the mutation information included in the mutation database 123 may include mutation ID, mutation position information (for example, "CHROM" and "POS"), "REF", "ALT", and "Annotation". The mutation ID is an identifier for identifying the mutation.

In the mutation position information, "CHROM" indicates the chromosome number, and "POS" indicates the position at the chromosome number. "REF" indicates the base in the wild type, and "ALT" indicates the base that is present after the mutation. "Annotation" indicates information related to mutation. "Annotation" may be information indicating mutation of amino acid such as "EGFR C2573G" or "EGFR L858R", for example. For example, "EGFR C2573G" indicates mutation in which cysteine at the 2573-th residue of protein "EGFR" is substituted with glycine.

As in the above-described example, "Annotation" of the mutation information may be information for converting mutation based on the base information to mutation based on amino acid information. In this case, the mutation identification unit 114 can convert the mutation based on the base information to the mutation based on the amino acid information, according to the information of "Annotation" which has been referred to.

The mutation identification unit 114 searches the mutation database 123 by using, as a key, information (for example, base information corresponding to mutation position information and mutation) that specifies the mutation included in the result file. For example, the mutation identification unit 114 may search the mutation database 123 by using, as a key, information of any of "CHROM", "POS", "REF", and "ALT". When the gene mutation extracted by comparison between the alignment sequence derived from the blood sample and the alignment sequence derived from the lesion site is already registered in the mutation database 123, the mutation identification unit 114 identifies the mutation as a mutation existing in the sample, and provides annotation (for example, "EGFR L858R", "BRAF V600E", or the like) to the mutation included in the result file.

In one embodiment, before the mutation identification unit 114 searches the mutation database 123 on the basis of the result file, the information selection unit 112 may cause mutations that do not correspond to the gene panel information having been inputted to the mutation identification unit 114, to be masked in (excluded from) the result file.

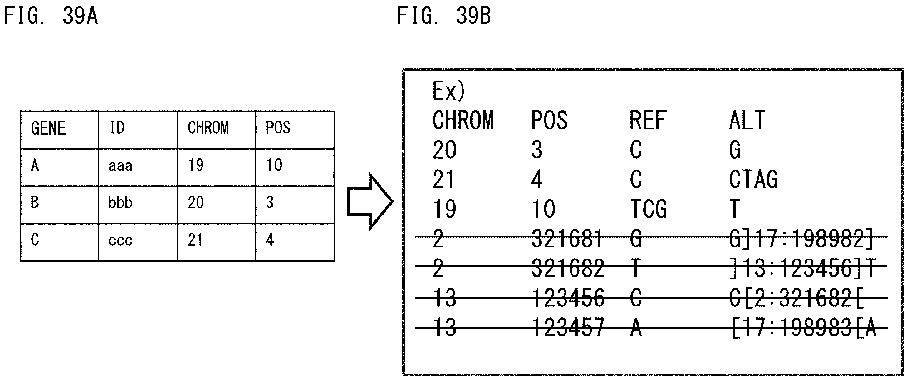

For example, in one aspect, the mutation identification unit 114 which has been notified of the gene panel information from the information selection unit 112 may refer to a table indicating the correspondence relationship between each gene to be analyzed and position information (for example, "CHROM" and "POS") as shown in FIG. 39A, may specify the position of mutation corresponding to the gene, to be analyzed, specified by the notified gene panel information, and may mask (exclude) mutations at the other positions in the result file as shown in FIG. 39B. Accordingly, the mutation identification unit 114 only has to provide annotation to the mutations, in the result file, that are related to the gene panel having been used, and thus, the mutation identifying and specifying efficiency can be improved.

(Drug search unit 118)

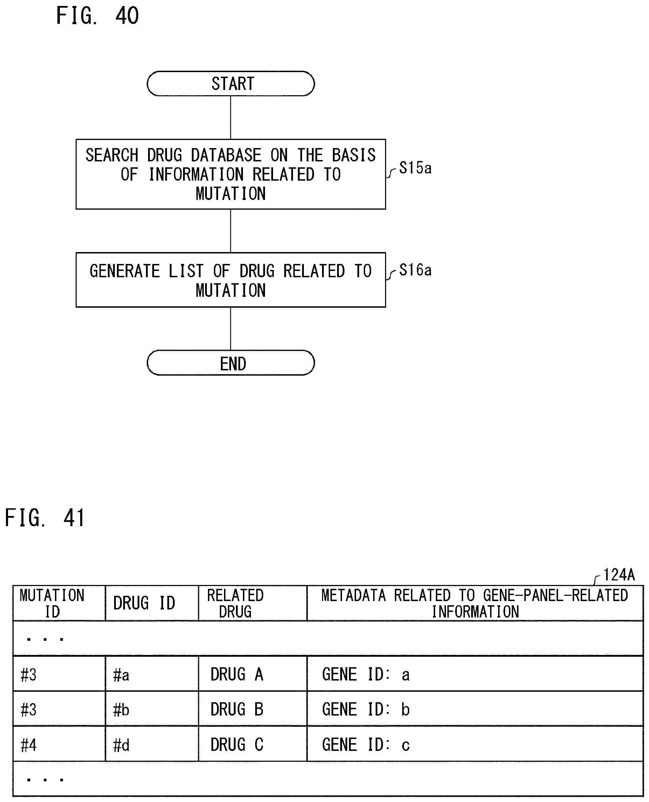

The flow of a process in which the drug search unit 118 generates a list including information related to drugs is described with reference to FIG. 40. FIG. 40 is a flow chart showing one example of the process in which the drug search unit 118 generates a list of drugs related to mutations.

The drug search unit 118 searches the drug database 124 by using, as a key, the mutation ID provided to each gene mutation identified by the mutation identification unit 114 (step S15a). On the basis of the search result, the drug search unit 118 generates a list including information regarding drugs related to the mutations (step S16a). The generated list is incorporated into the report created by the report creation unit 115.

(Drug database 124)

Data 124A stored in the drug database 124 and used when the drug search unit 118 searches the drug database 124 and generates a drug list is described with reference to FIG. 41. FIG. 41 is a diagram showing an example of a data structure of the drug database 124.

As shown in FIG. 41, a mutation ID provided to each mutation, a related drug name, and a drug ID provided to each drug are stored in association with one another in the drug database 124. With reference to data 124A of FIG. 41, "drug A" and "drug B" are associated with mutation ID "#3". Similar to this, each mutation ID may have a plurality of related drugs associated therewith.

Each mutation ID in the drug database 124 may be provided with "metadata related to gene-panel-related information", which is metadata related to gene panel information. The drug search unit 118 refers to the "metadata related to gene-panel-related information" in accordance with an instruction from the information selection unit 112.

The drug search unit 118 changes the range in which the drug database 124 is searched, to a range indicated by the metadata. Accordingly, in accordance with the "metadata related to gene-panel-related information" provided to each drug and the inputted gene panel information, the drug search unit 118 can narrow the drugs that should be referred to in the drug database, and can generate a list that includes information regarding drugs according to the gene panel information.

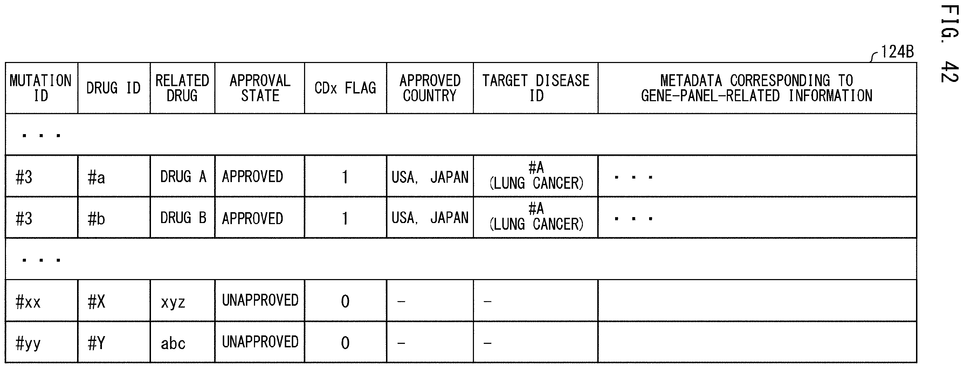

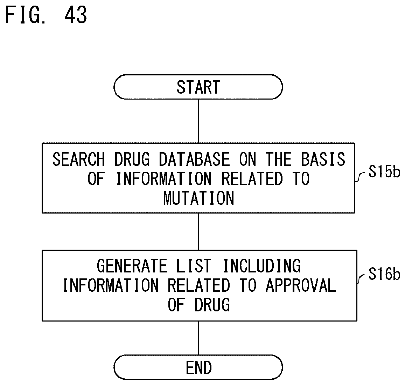

The drug search unit 118 may search the drug database 124 having a data structure shown in FIG. 42, and generate a list that includes another type of information regarding drugs related to mutations. This is described with reference to FIG. 43. FIG. 43 is a flow chart showing one example of a process in which the drug search unit 118 generates a list that includes information regarding drugs related to mutations.

The drug search unit 118 searches the drug database 124 which stores data 124B shown in FIG. 42, as to whether or not the related drug has been approved by an authority (FDA, PMDA, or the like). Specifically, for example, by using information related to a mutation such as "mutation ID" as a key, the drug search unit 118 searches for "approval state" which indicates whether the related drug corresponding to the mutation has been approved by the authority, and "approved country" which indicates which country's authority has provided its approval (step S15b).

On the basis of the search result, the drug search unit 118 generates a list that includes the mutation, the related drug that corresponds to the mutation, and information regarding the approval of the related drug (step S16b).

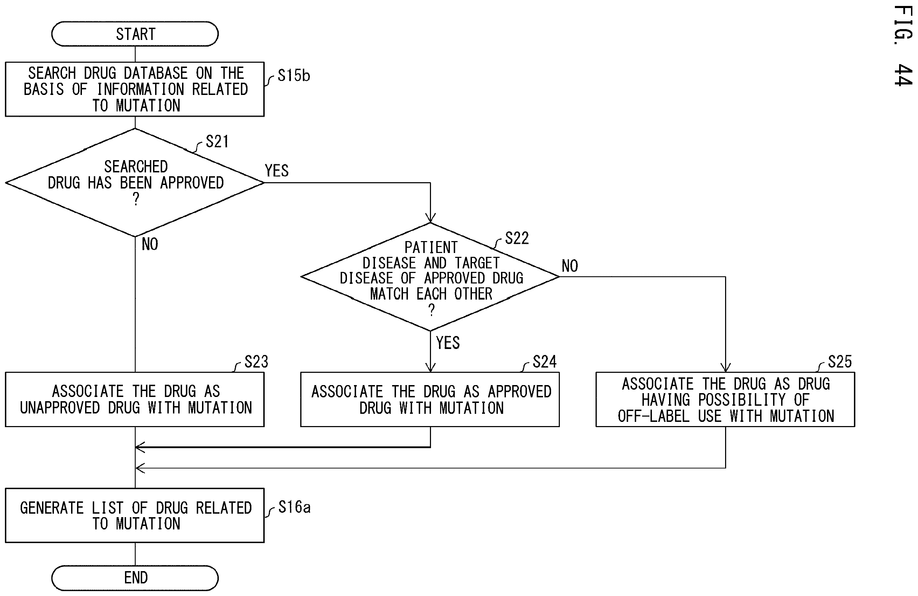

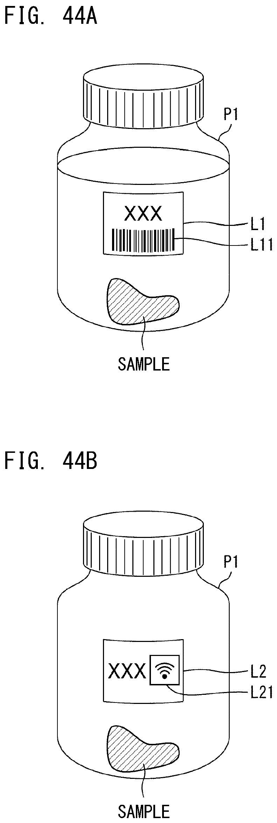

The drug search unit 118 may search the drug database 124 having a data structure shown in FIG. 42 and generate a list that includes still another type of information regarding drugs related to mutations. This is described with reference to FIG. 44. FIG. 44 is a flow chart showing one example of a process in which, on the basis of information obtained by searching the drug database 124, the drug search unit 118 determines the presence or absence of a drug having a possibility of off-label use and generates a list that includes the determination result.

The drug search unit 118 searches the drug database 124 which stores the data 124B shown in FIG. 42, as to whether or not the related drug has been approved by an authority (FDA, PMDA, or the like) (step S15b). When the searched drug has not been approved (NO in step S21), the drug search unit 118 associates the drug, as an unapproved drug, with the mutation (step S23), and creates a report of drugs related to the mutations (step S16a).

When the searched drug has been approved (YES in step S21), the drug search unit 118 determines whether or not the disease (disease name or disease ID) of the subject from whom the sample has been collected, and the disease (for example, disease name or disease ID of "target disease" shown in FIG. 42) that corresponds to the related drug searched from the drug database 124 match each other (step S22).

When the disease of the subject and the "target disease" match each other (YES in step S22), the drug search unit 118 associates the drug of the search result, as an approved drug, with the mutation (step S24), and generates a list that includes the mutation, the related drug corresponding to the mutation, information regarding the approval of the related drug, and the like (step S16a).

Meanwhile, when the disease of the subject and the "target disease" are different from each other (NO in step S22), the drug search unit 118 determines that the searched related drug is a drug having a possibility of off-label use, associates the determination result with the mutation (step S25), and generates a list that includes the mutation, the related drug corresponding to the mutation, and information regarding the approval of the related drug, and the like (step S16a).

The identification information (for example, disease name, disease ID, or the like) for identifying the disease of the subject can be inputted through the input unit 17 by an operator or the like when performing gene analysis, for example. In this case, the information selection unit 112 obtains information related to the disease corresponding to the sample inputted by the operator, and identifies the disease. Alternatively, as shown in FIG. 44A, a label L1 indicating a subject ID, a sample ID, and the like is attached to each container P1 which stores a sample, and a recording means such as a bar code L11 indicated on the label L1 is read, whereby the disease ID which is identification information of the disease of the subject may be obtained. Alternatively, as shown in FIG. 44B, a label L2 indicating a subject ID, a sample ID, and the like is attached to each container P1 which stores a sample, and a recording means such as an RFID tag L21 attached to the label L2 is read, whereby the disease ID which is identification information of the disease of the subject may be obtained. Alternatively, in the test institution 120, a sample ID and a subject ID are managed so as to be associated with a disease ID, and the information selection unit 112 may obtain a disease ID that corresponds to a sample, on the basis of the subject ID or the sample ID. For example, the information selection unit 112 may obtain, via a communication line, a disease ID associated with a subject ID (or sample ID) obtained by reading a recording means of a label attached to each container which stores a sample. The disease ID may be included in a header region of read sequence information and the information selection unit 112 may obtain the disease ID.

As in the data 124B shown in FIG. 42, the drug database 124 may have a "CDx flag" which indicates whether or not each drug in the database is a drug related to CDx in terms of the relationship of a predetermined gene mutation and a predetermined disease. When the drug search unit 118 has retrieved a drug whose CDx flag is "1" ("drug A" and "drug B" in FIG. 42), the drug search unit 118 may generate a list that includes auxiliary information that indicates the detection result of the predetermined gene mutation in the predetermined disease is applicable to the CDx for the retrieved drug. In accordance with the fact that a predetermined gene mutation has been detected in the sample collected from the subject having a predetermined disease (for example, cancer), the drug search unit 118 may create a list that includes information that the detected gene mutation and the drug corresponding to the gene mutation are related to CDx, and auxiliary information related to the efficacy of the drug.

In this manner, the drug search unit 118 searches the drug database 124 in which gene mutations, target diseases, and drugs are stored in association with one another, and checks a detected gene mutation against a disease specified by the information selection unit 112, thereby being able to create a list according to the disease corresponding to the sample. The report creation unit 115 creates a report by use of the list created by the drug search unit 118.

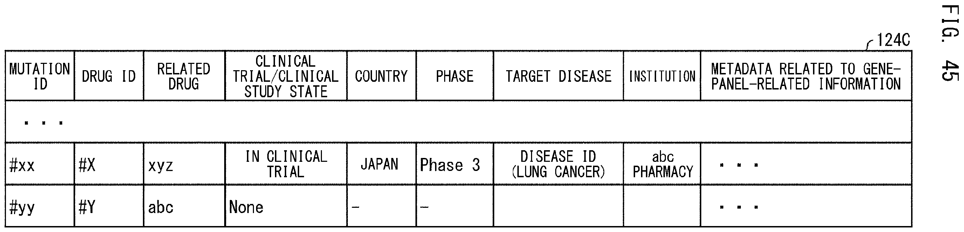

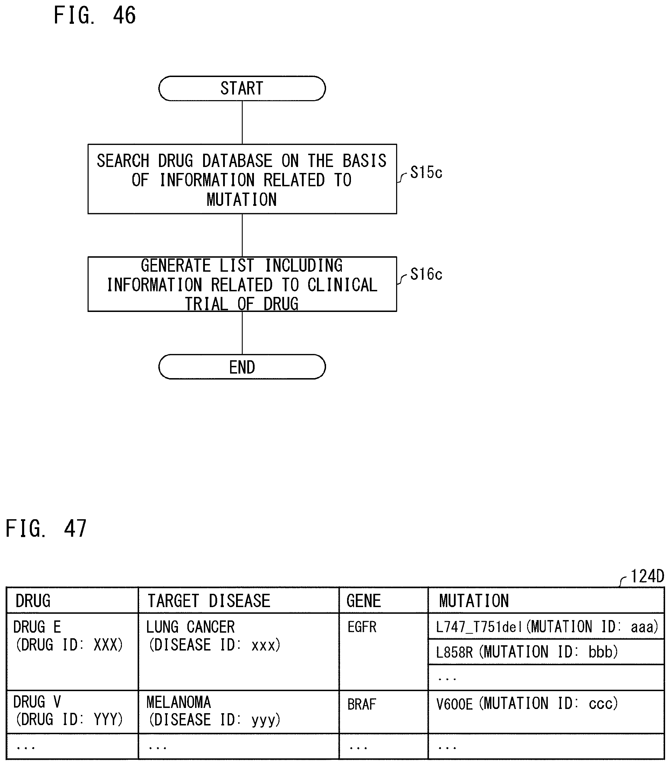

The drug search unit 118 may search the drug database 124 having a data structure shown in FIG. 45 and generate a list that includes information regarding clinical trials of drugs related to mutations. This is described with reference to FIG. 46. FIG. 46 is a flow chart showing one example of a process in which the drug search unit 118 generates a list that includes information regarding clinical trials of drugs.

The drug search unit 118 searches the drug database 124 which stores data 124C shown in FIG. 45, for information such as the progress of a clinical trial of a related drug. Specifically, by using a mutation ID or the like as a key, the drug search unit 118 searches for information regarding a clinical trial with respect to the mutation, such as, for example, "clinical trial/clinical study state", "country", and "institution" in which the clinical trial is being performed, as shown in FIG. 45 (step S15c in FIG. 46). On the basis of the search result, the drug search unit 118 generates a list that includes the mutation, the related drug corresponding to the mutation, and information regarding the clinical trial of the related drug (step S16c in FIG. 46).

The data 124A shown in FIG. 41, the data 124B shown in FIG. 42, and the data 124C shown in FIG. 45 may be integrated together and stored in the drug database 124, or may be discretely stored in a plurality of databases including the drug database 124.

(Report creation unit 115)

The report creation unit 115 creates a report (corresponding to step S111 in FIG. 4, and FIG. 1) on the basis of the information outputted by the mutation identification unit 114, the gene panel information provided from the information selection unit 112, and the drug list generated by the drug search unit 118. The information put on the created report includes gene panel information, information related to the identified gene mutation, and information of the drug related to the detected gene mutation. When the test institution 120 has concluded a contract of a "CDx usage" plan, the report creation unit 115 can create a report that includes auxiliary information related to the efficacy of the drug applicable to CDx, on the subject having a predetermined disease.

On the basis of the gene panel information from the information selection unit 112, the report creation unit 115 may select the target to be put on the report, and may delete, from the report, information that has not been selected. Alternatively, the information selection unit 112 may control the report creation unit 115 such that information related to genes that correspond to the gene panel information inputted through the input unit 17 is selected as the target to be put on the report and information that has not been selected is deleted from the report.

(Creation of report for CDx usage)

The report creation unit 115 creates a report that includes auxiliary information related to the efficacy of a predetermined drug, on the basis of CDx information database 124D shown in FIG. 47 and at least one of the information outputted by the mutation identification unit 114 and the drug list generated by the drug search unit 118.

Specifically, the report creation unit 115 confirms the disease specified by the information selection unit 112, and then, confirms whether or not the disease corresponds to the gene mutation specified by the mutation identification unit 114. When the disease corresponds to the gene mutation specified by the mutation identification unit 114, the report creation unit 115 may create a report indicating this gene mutation as a gene mutation to be used for CDx. Alternatively, after confirming the gene mutation specified by the mutation identification unit 114, the report creation unit 115 confirms the disease specified by the information selection unit 112, and when the gene mutation corresponds to the disease specified by the information selection unit 112, the report creation unit 115 may create a report indicating this gene mutation as a gene mutation to be used for CDx.

<CDx information database 124D>

Each test institution 120 that uses the gene analysis system 100 is provided with a CDx information database 124D. FIG. 47 is a diagram showing an example of a data structure of the CDx information database. The CDx information database 124D may be stored in the drug database 124 as in the case of the data 124A to 124C, or may be stored as a separate database.

In the CDx information database 124D, for each drug applicable to CDx, drug name, drug ID, target disease, gene name (or gene ID), mutation, mutation ID, and the like are associated with one another.

By using the mutation ID, or the disease name or the disease ID of the subject as a key, the report creation unit 115 searches the CDx information database 124D for information related to CDx, and creates a report that includes auxiliary information related to the efficacy for CDx.

(Example of report)

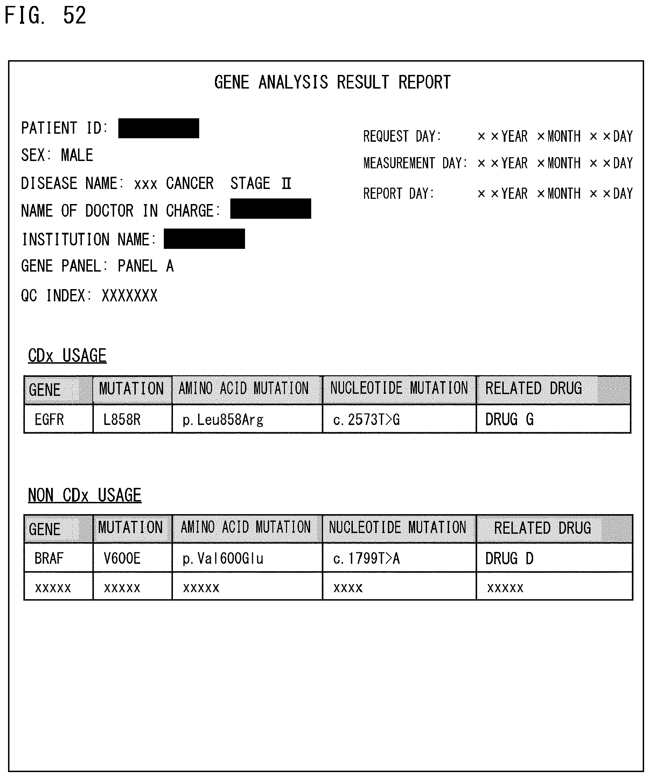

Next, some specific examples of the report created by the report creation unit 115 are described with reference to FIG. 52 to FIG. 60. FIG. 52 to FIG. 60 are each a diagram showing one example of the report to be created.

In the upper left part of the example of the report shown in FIG. 52 to FIG. 60, "patient ID" indicating the subject ID, "sex of patient", "disease name of patient", "name of doctor in charge" which is the name of the doctor who is in charge of the subject in the medical institution 210, and "institution name" indicating the medical institution name are described.

Below these items, the gene panel name such as "Panel A" is also indicated as the gene panel information. Further, the quality evaluation index "QC index" obtained from the process using the quality control sample, the result of analysis thereof, and the like is also outputted in the report.

As shown in FIG. 52, the report creation unit 115 may create a report in which information related to a predetermined gene mutation associated with auxiliary information and information related to a gene mutation different from the predetermined gene mutation are distinct from each other. Specifically, the report creation unit 115 may create a report in which an analysis result for "CDx usage" and an analysis result for "non CDx usage" are distinct from each other. The analysis result for "CDx usage" may be displayed in a color different from that for the analysis result for "non CDx usage" such that the analysis result for "CDx usage" is displayed in red, for example, and the analysis result for "non CDx usage" is displayed in black, for example. In the report created by the report creation unit 115, the heading "CDx usage" is included in auxiliary information related to the efficacy of drugs that are applicable to CDx.

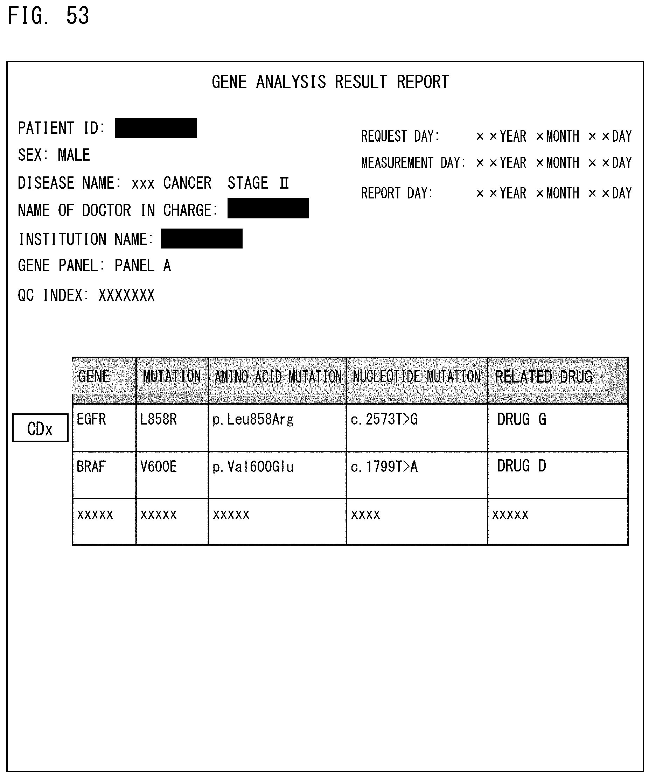

As shown in FIG. 53, the report creation unit 115 may create a report in which information related to a predetermined gene mutation having been detected in a nucleic acid sequence of the sample is provided with an indication (for example, an icon) meaning that this is a gene mutation related to auxiliary information. Specifically, the report creation unit 115 may create a report in which an icon such as "CDx" is provided to the analysis result for "CDx usage". In a case where the analysis result is electronically displayed on the input unit 17 of the information processing apparatus 1 (for example, when the analysis result is displayed on a Web browser), an icon such as "CDx" may be displayed when a cursor is at a position corresponding to a predetermined gene mutation in the list of detected gene mutations.

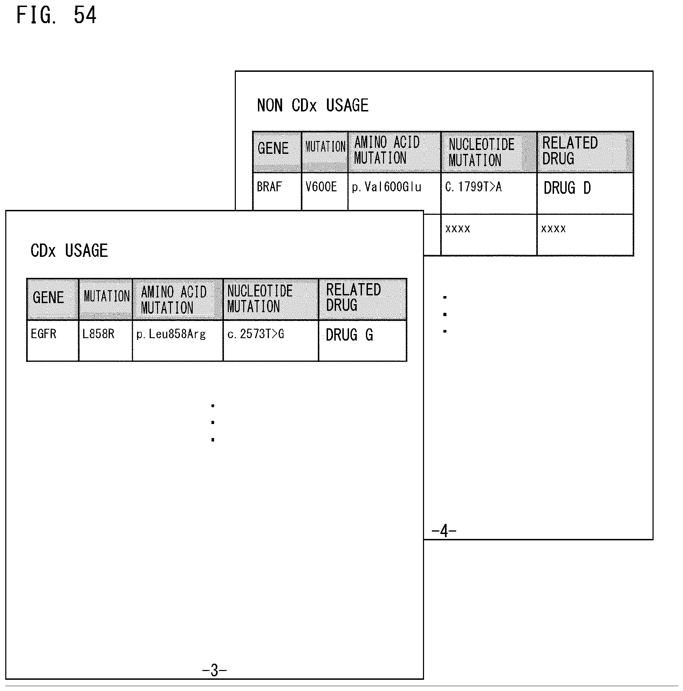

As shown in FIG. 54, the report creation unit 115 may create a report that includes: a first region which includes information related to a predetermined gene mutation and auxiliary information; and a second region which includes information related to a gene mutation different from the predetermined gene mutation. Specifically, the report creation unit 115 may create a report such that the analysis result for "CDx usage" and the analysis result for "non CDx usage" are shown in different regions (for example, on different pages) of the report. In the example shown in FIG. 54, the analysis result for "CDx usage" is shown on the third page, and the analysis result and the like for "non CDx usage" are shown on the fourth page and the subsequent pages.

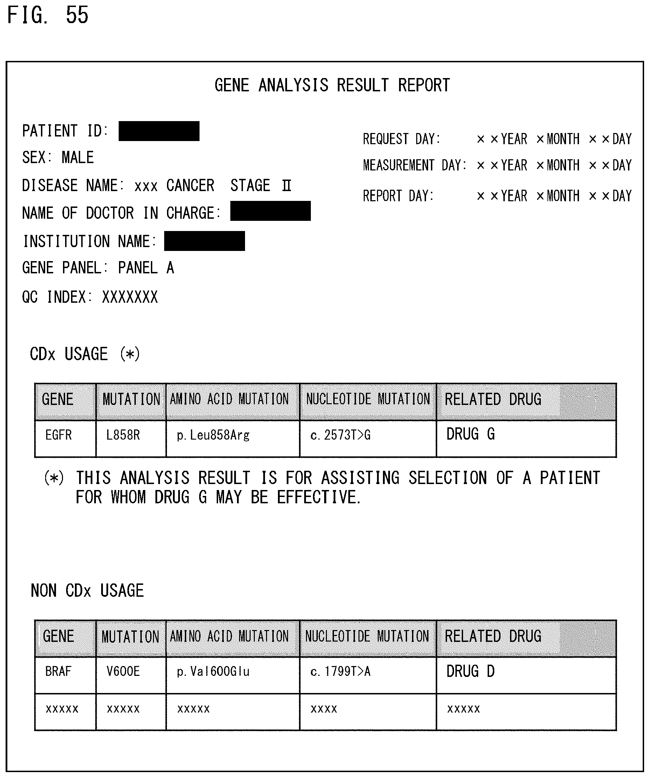

As shown in FIG. 55, on the basis of the result of analysis of the nucleic acid sequence of the sample, the report creation unit 115 may create a report that includes auxiliary information indicating that the subject from whom the sample has been collected is selectable as a subject for whom a predetermined drug may be effective to the disease. Specifically, the report creation unit 115 may add "*" to the analysis result for "CDx usage" and display, as auxiliary information, a comment such as "this analysis result is for assisting selection of a patient for whom drug G may be effective."

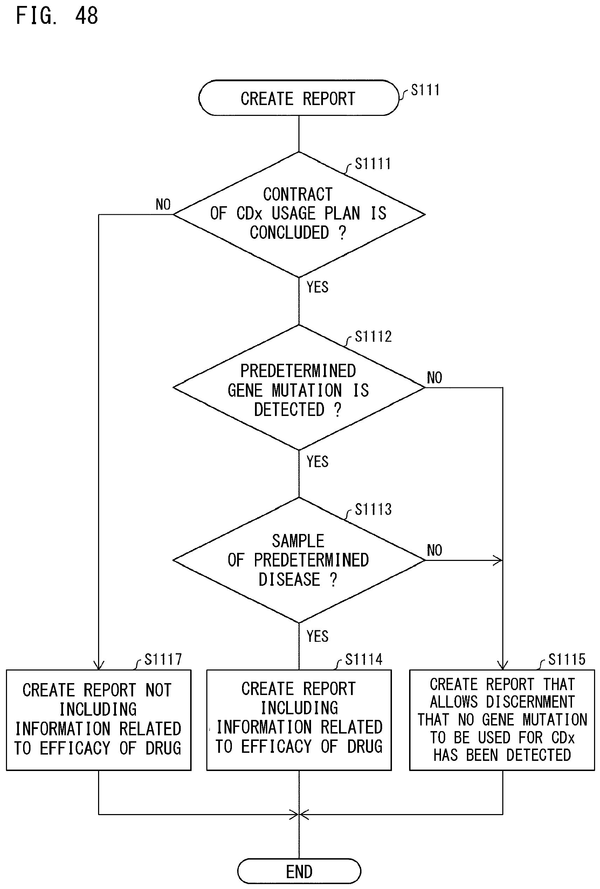

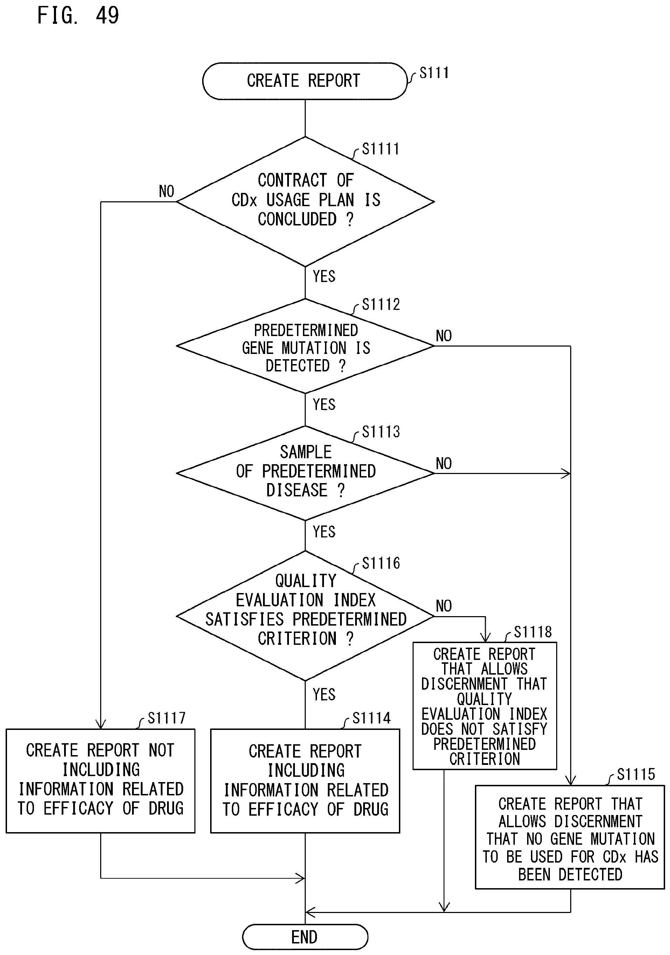

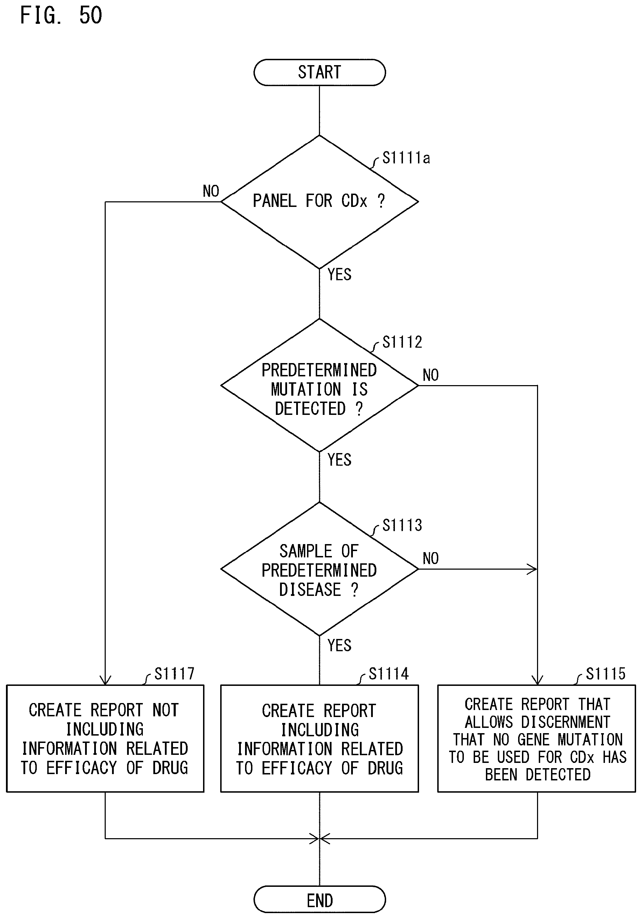

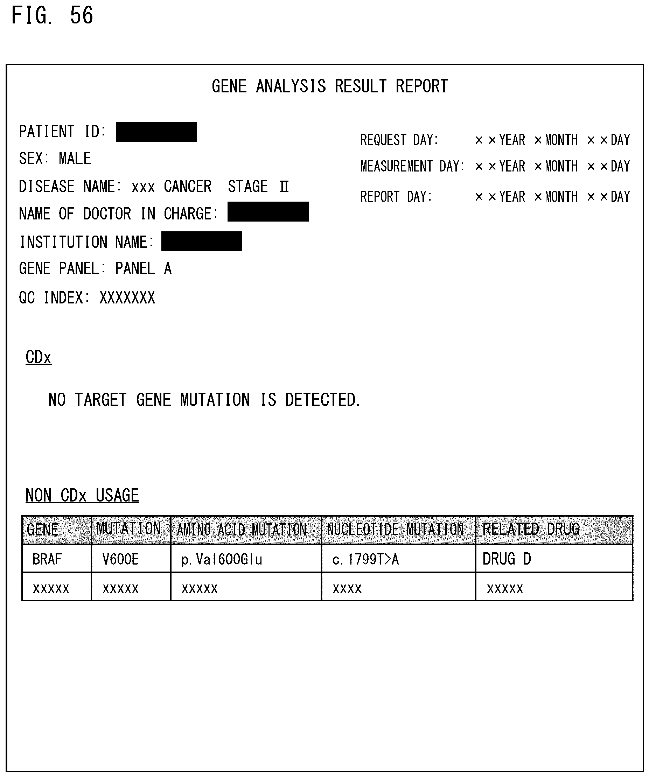

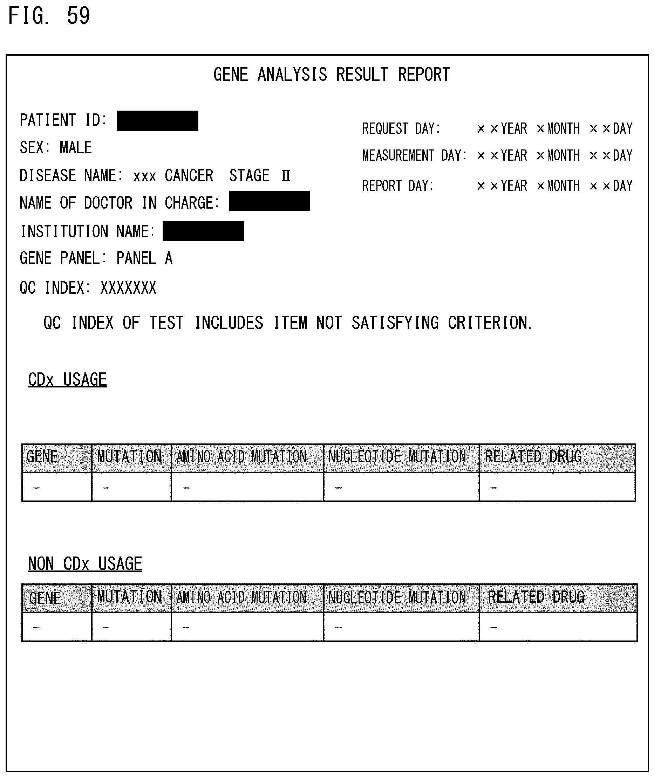

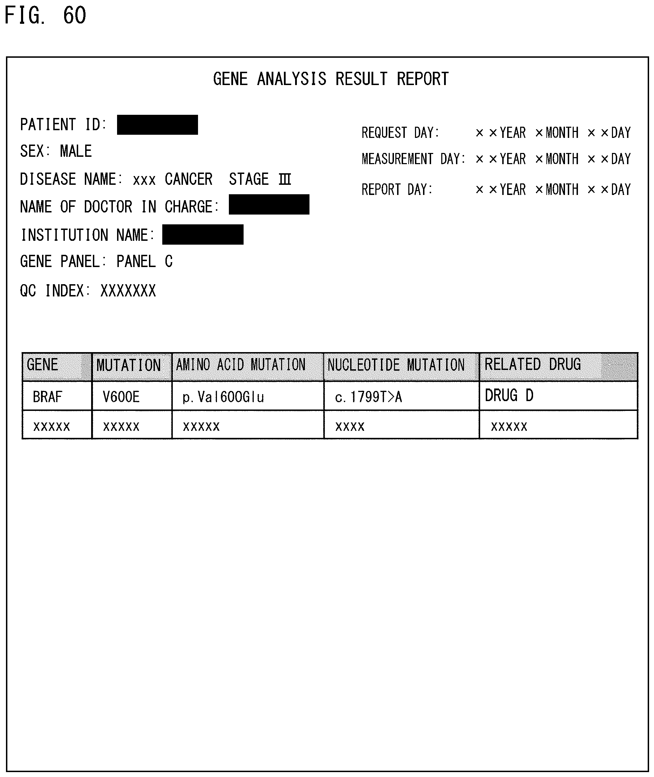

FIGS. 56 to 58 each show an example of the report created by the report creation unit 115 in step S1115 in FIG. 48 described later, and FIG. 59 shows an example of the report created by the report creation unit 115 in step S1115 in FIG. 49. The report in FIG. 60 shows an example of the report created by the report creation unit 115 in step S1117 in FIG. 50.

As described above, the report creation unit 115 creates a report in which the display of gene mutations for "CDx usage" and the display of gene mutations for "non CDx usage" are clearly distinct from each other. Therefore, as for an analysis result obtained through a gene panel test, it is possible to avoid the display of gene mutations for "CDx usage" from being undistinguishable among a plurality of gene mutations for a plurality of diseases, for example. In addition, the report created in this manner can provide a medical institution and the like with gene mutations applicable to CDx and auxiliary information related to the efficacy of drugs in an easily understandable manner. Accordingly, usability of the report is improved, and the use of CDx based on detection of a predetermined gene mutation and individualized medical care can be made more effective.

It should be noted that only the information processing apparatus 1 installed in the test institution that has concluded a contract of a CDx usage plan may be permitted to create the report as shown in FIG. 52 to FIG. 59.

For realizing this, for example, only when a contract of a CDx usage plan (for example, "Plan 3" or "Plan 4" shown in 3B in FIG. 5) has been concluded, the CDx information database 124D may be provided from the management server 3 to the information processing apparatus 1.

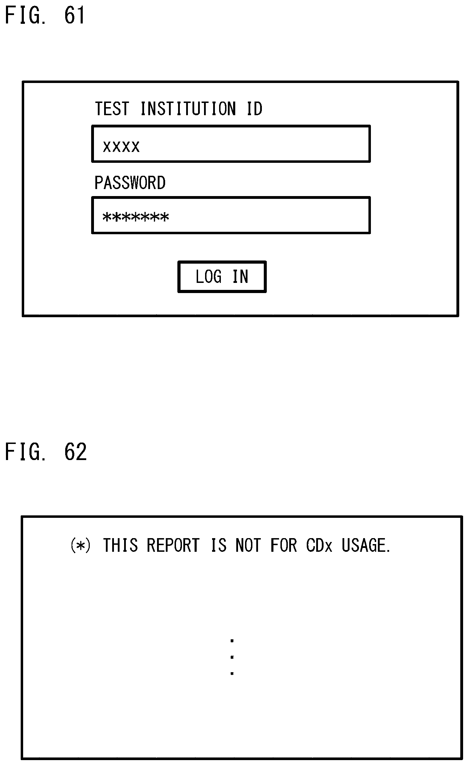

Alternatively, only in the case of a user who is allowed to use CDx in a test institution or the like that has concluded a contract of a CDx usage plan, the report creation unit 115 may be permitted to create a report as shown in FIG. 52 to FIG. 59. This configuration is described with reference to FIG. 61 and FIG. 62. FIG. 61 is one example of a GUI displayed when a test institution logs in the gene analysis system. FIG. 62 is diagram showing one example of a report to be created when the contract of the test institution is of a type that does not include CDx usage.

As shown in FIG. 61, when starting using the gene analysis system 100, each test institution 120 is required to input a test institution ID and a password. The management server 3 having received the inputted test institution ID confirms the plan contracted by the test institution 120. When the plan contracted by the test institution 120 includes CDx usage, the report creation unit 115 of the information processing apparatus 1 of the test institution 120 is permitted to create a report as shown in FIG. 52 to FIG. 59. Meanwhile, when the plan contracted by the test institution 120 does not include CDx usage, the report creation unit 115 of the information processing apparatus 1 of the test institution 120 creates a report as shown in FIG. 62, for example, and is not permitted to create a report as shown in FIG. 52 to FIG. 59. In the example shown in FIG. 62, a message "This report is not for CDx usage." indicating that this report is not for CDx usage is included in the report.

In the present configuration, confirmation of the contracted plan is not mandatory, and the analysis system management institution 130 may determine whether or not the user is allowed to use CDx, on the basis of the test institution ID inputted at the time of start of the use of the gene analysis system 100. In this case, when the test institution ID is of a user that is allowed to use CDx, the report creation unit 115 of the information processing apparatus 1 of the test institution 120 is permitted to create a report as shown in FIG. 52 to FIG. 59. Meanwhile, when the test institution ID is of a user that is not allowed to use CDx, the report creation unit 115 of the information processing apparatus 1 of the test institution 120 creates a report as shown in FIG. 60, for example, and is not permitted to create a report as shown in FIG. 52 to FIG. 59.

That is, the report creation unit 115 can create a first report which shows a gene mutation related to the efficacy of a drug, and a second report which shows a gene mutation not related to the efficacy of the drug. Specifically, the report creation unit 115 performs the following.

(1) Create a report in accordance with a disease that corresponds to a sample.
(2) Create a report in accordance with a disease that corresponds to a sample, and a gene mutation specified in a nucleic acid sequence of the sample.
(3) Create a report in which a gene mutation associated with information related to the efficacy of a drug (for example, CDx usage) is shown in a different manner, in accordance with a disease that corresponds to the sample and a gene mutation specified in a nucleic acid sequence of the sample.

In the following, the flow of a process in which the report creation unit 115 creates a report with reference to the CDx information database 124D shown in FIG. 47 is described with reference to FIG. 48. FIG. 48 is a flow chart showing one example of a process in which the report creation unit 115 creates a report that includes auxiliary information related to the efficacy for CDx.

When the test institution having the information processing apparatus 1 installed therein has not concluded a contract of a CDx usage plan (for example, "Plan 3" or "Plan 4" shown in 3B in FIG. 5) (NO in step S1111), the report creation unit 115 creates a report that does not include information related to the efficacy of a drug (step S1117). This determination of the plan is not mandatory, and auxiliary information related to the efficacy of a drug applicable to CDx may be uniformly provided.

When the test institution has concluded a contract of a CDx usage plan (YES in step S1111), the process is advanced to step S1112. In step S1112, the report creation unit 115 confirms whether or not the information (for example, mutation ID, or the like) related to a gene mutation notified of from at least one of the mutation identification unit 114 and the drug search unit 118 exists in the CDx information database 124D shown in FIG. 47. When the gene mutation exists in the CDx information database 124D (YES in step S1112), the process is advanced to step S1113. Meanwhile, when the predetermined gene mutation does not exist in the CDx information database 124D (NO in step S1112), the process is advanced to step S1115.

Subsequently, in step S1113, the report creation unit 115 confirms whether or not the identification information of the disease of the subject obtained by the information selection unit 112 (for example, target disease name, disease ID, and the like) matches a target disease that corresponds to the gene mutation confirmed in the CDx information database 124D. Specifically, by using the mutation ID of the gene mutation included in the analysis result as a key, the report creation unit 115 searches the CDx information database 124D and obtains target diseases that correspond to the mutation ID. When the disease of the subject exists in the obtained target diseases (YES in step S1113), the report creation unit 115 advances to step S1114, and creates a report that includes information related to the efficacy of the drug. Meanwhile, when the disease of the subject does not exist in the obtained target diseases (NO in step S1113), the report creation unit 115 advances to step S1115, and creates a report that allows discernment that no gene mutation to be used for CDx has been detected.

In step S1115, the report creation unit 115 creates a report as shown in (1) to (2) below.

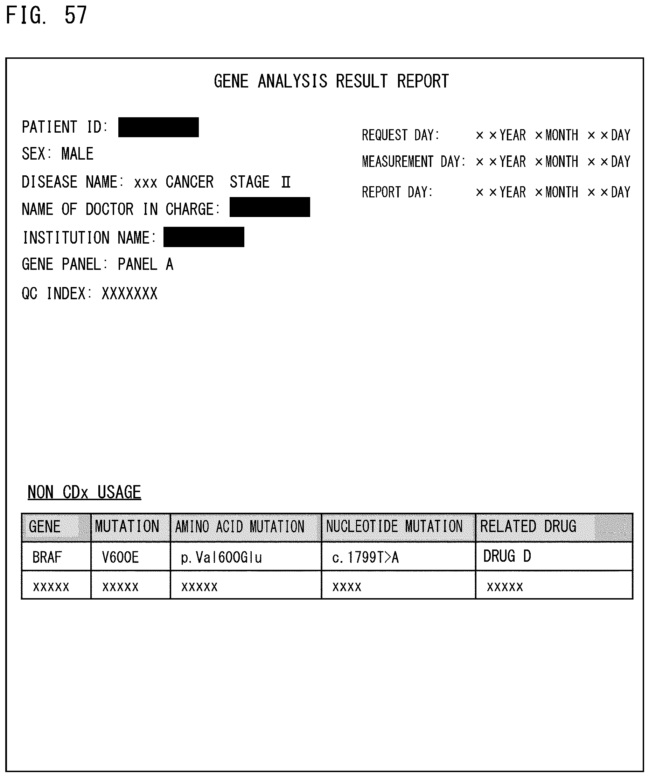

(1) The report creation unit 115 creates a report in which an indication that no gene mutation to be used for CDx has been detected is provided near information indicating "CDx usage" (for example, an indication of "CDx usage"). For example, as shown in FIG. 56, the report creation unit 115 creates a report that includes a message such as "no gene mutation to be used for CDx has been detected", or an indication of "None" or "Not detected", which indicates that no gene mutation to be used for CDx has been detected.
(2) The report creation unit 115 creates a report that does not include the information indicating "CDx usage" itself, as shown in FIG. 57.

Here, the disease identification information can be inputted through the input unit 17 by an operator or the like when performing gene analysis. The report creation unit 115 may obtain the inputted disease identification information from the information selection unit 112 and confirm whether or not the disease identification information exists in the CDx information database 124D.

As described above, when a predetermined gene mutation has been detected in a nucleic acid sequence of the sample associated with identification information of a predetermined disease, the report creation unit 115 creates a report that includes auxiliary information related to the efficacy of a predetermined drug. Thus, a report that includes auxiliary information related to the efficacy of the predetermined drug can be appropriately created.

<Modification 1 of creation of report for CDx usage>

The quality-control unit 117 generates a quality control index for evaluating the quality of a gene test, on the basis of the sequence information obtained by measuring a quality evaluation sample. When the result of a gene panel test is to be used for CDx usage, the reliability, the accuracy, and the like of the analysis result obtained in the gene panel test are required to satisfy predetermined criteria.

Therefore, as shown in FIG. 49, the report creation unit 115 may confirm whether or not at least one of a plurality of quality evaluation indexes generated by the quality-control unit 117 satisfies a predetermined criterion stored in the quality evaluation criteria 126 shown in FIG. 6 (step S1116). That is, when the quality evaluation index satisfies a predetermined criterion (YES in step S1116), the process is advanced to S1114, and the report creation unit 115 creates a report that includes information related to the efficacy of a drug. Meanwhile, when the quality evaluation index does not satisfy the predetermined criterion (NO in step S1116), the process is advanced to S1118, and the report creation unit 115 creates a report that allows discernment that the quality evaluation index does not satisfy the predetermined criterion. It should be noted that, when the quality evaluation index does not satisfy the predetermined criterion (NO in step S1116), the report creation unit 115 may create a report that includes information indicating that the analysis result included in the created report is reference information whose quality level is less than a normal quality level. The report creation unit 115 may be configured not to create a report when the quality evaluation index does not satisfy the predetermined criterion (NO in step S1116).

When the quality evaluation index does not satisfy the predetermined criterion (NO in step S1116), the report creation unit 115 may create a report, as described in (1) or (2) below, that allows discernment that no gene mutation to be used for CDx has been detected.

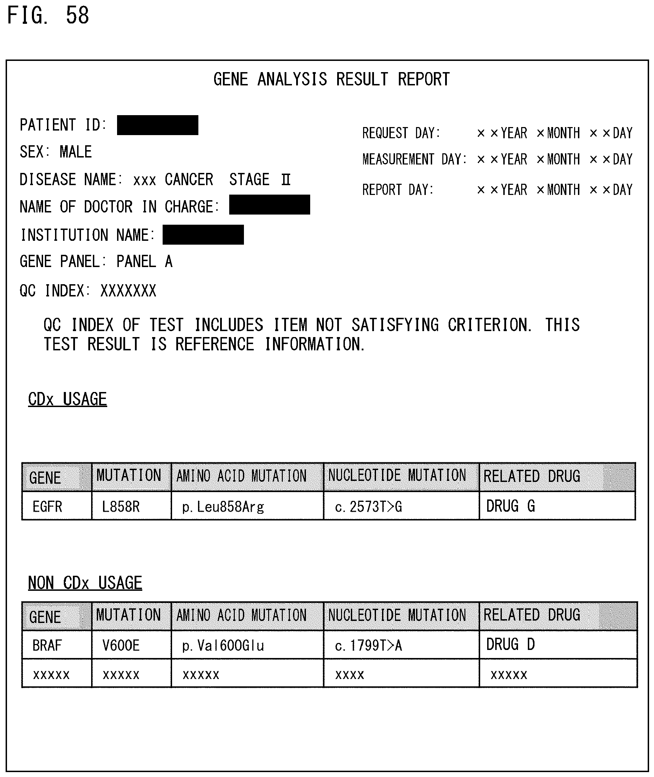

(1) As shown in FIG. 58, gene mutations for "CDx usage" and "non CDx usage" are shown, but near the gene mutations, a comment is provided such as "QC indexes of the test include an item that does not satisfy a criterion. This test result is reference information." which indicates that the analysis result included in the created report is reference information whose quality level is less than a normal quality level.
(2) As shown in FIG. 59, a comment is provided such as "QC indexes of the test include an item that does not satisfy a criterion." which indicates that the quality level of the analysis result is less than a normal quality level, and no analysis results for "CDx usage" and "non CDx usage" are shown.

As described above, even when the quality evaluation index does not satisfy a predetermined criterion, the report creation unit 115 creates a report that does not include auxiliary information related to the efficacy of a drug applicable to CDx, or a report that includes information indicating that the analysis result included in the report is reference information whose quality level is less than a normal quality level. That is, in accordance with the fact that the quality evaluation index does not satisfy a predetermined criterion, the report creation unit 115 may create a report that does not include information related to the efficacy of a drug for the subject having the gene mutation, and that allows discernment that no gene mutation to be used for CDx has been detected.

<Modification 2 of creation of report for CDx usage>

The report creation unit 115 may create a different report depending on whether or not the panel test has used a gene panel for analyzing gene mutations in genes to be used for CDx. FIG. 50 is a flow chart showing one example of a process in which the report creation unit 115 creates a report that includes auxiliary information related to the efficacy for CDx in accordance with whether or not the used gene panel is a gene panel for CDx. The steps already described are denoted by the same step numbers, and description thereof is omitted.

On the basis of the data 121B shown in FIG. 9, the information selection unit 112 specifies whether "CDx flag" of the obtained gene panel name (or gene panel ID) is "1" or "0". When the gene panel specified by the information selection unit 112 is a gene panel for CDx (YES in step S1111a), the report creation unit 115 advances to step S1112. When the gene panel specified by the information selection unit 112 is not a gene panel for CDx (NO in step S1111a), the report creation unit 115 advances to S1117, and creates a report that does not include information related to the efficacy of a drug.

According to this configuration, as a report of the result of a panel test using a gene panel (not a gene panel for CDx) whose genes to be analyzed do not include gene mutations to be used for CDx, a report that does not include information related to the efficacy of a drug is created. For example, in a panel test using a gene panel that is not for CDx, even if a predetermined gene mutation to be used for CDx is detected in a nucleic acid sequence of a sample and a disease corresponding to the sample is a predetermined disease targeted in CDx, a report that does not include information related to the efficacy of a drug is created as shown in FIG. 60.

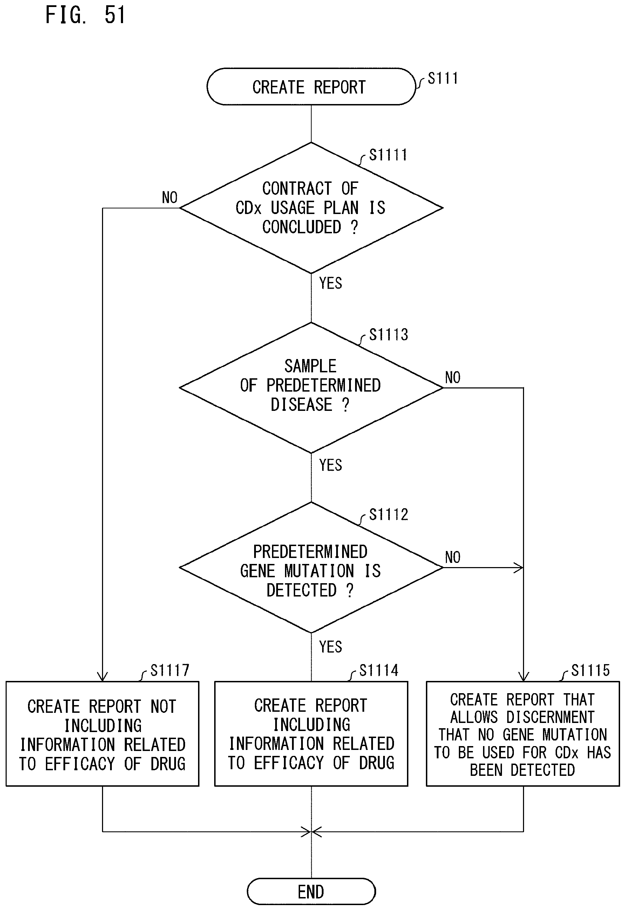

The order of the processes of step S1112 and step S1113 in FIGS. 48 to 50 is not limited thereto. For example, as shown in FIG. 51, step S1112 may be performed after step S1113. FIG. 51 is a flow chart showing another example of a process in which the report creation unit 115 creates a report that includes auxiliary information related to the efficacy of a predetermined drug.

In this case, first, in step S1113, the report creation unit 115 confirms whether or not the identification information of the disease of the subject obtained by the information selection unit 112 (for example, target disease name, disease ID, and the like) matches a target disease that corresponds to a gene mutation confirmed in the CDx information database 124D. Specifically, by using the mutation ID of the gene mutation included in the analysis result as a key, the report creation unit 115 searches the CDx information database 124D and obtains target diseases that correspond to the mutation ID. When the disease of the subject exists in the obtained target diseases (YES in step S1113), the report creation unit 115 advances to step S1112. Meanwhile, when the disease of the subject does not exist in the obtained target diseases (NO in step S1113), the report creation unit 115 advances to step S1115.

Subsequently, in step S1112, the report creation unit 115 confirms whether or not information (for example, mutation ID, or the like) related to a gene mutation notified of from at least one of the mutation identification unit 114 and the drug search unit 118 is associated with the target disease confirmed to exist in step S1113. When the target disease and the gene mutation are associated with each other in the CDx information database 124D (YES in step S1112), the report creation unit 115 advances to step S1114 and creates a report that includes information related to the efficacy of a drug. Meanwhile, when the target disease and the gene mutation are not associated with each other in the CDx information database 124D (NO in step S1112), the report creation unit 115 advances to step S1115 and creates a report that allows discernment that no gene mutation to be used for CDx has been detected.

(Output unit 13)

The report created by the report creation unit 115 may be transmitted in the form of data, from the output unit 13 to the communication terminal 5 installed in the medical institution 210, as the analysis result of the read sequence information (corresponding to step S112 in FIG. 4). Alternatively, the report may be transmitted to a printer (not shown) connected to the information processing apparatus 1, to be printed by the printer, and then, may be sent in the form of a paper medium, from the test institution 120 to the medical institution 210.

<Quality control index>

Examples of the quality evaluation index include the following.

- Index (i): quality evaluation index indicating the quality of reading of read sequence information performed by the sequencer 2.
- Index (ii): quality evaluation index indicating the proportion of bases read by the sequencer 2, to bases included in a plurality of genes to be analyzed.
- Index (iii): quality evaluation index indicating the depth of read sequence information.

- Index (iv): quality evaluation index indicating variation in the depth of read sequence information.
- Index (v): quality evaluation index indicating whether or not all the mutations in standard genes included in the quality control sample have been detected.

Index (i) can include

index (i-1): quality score, and
index (i-2): cluster concentration.

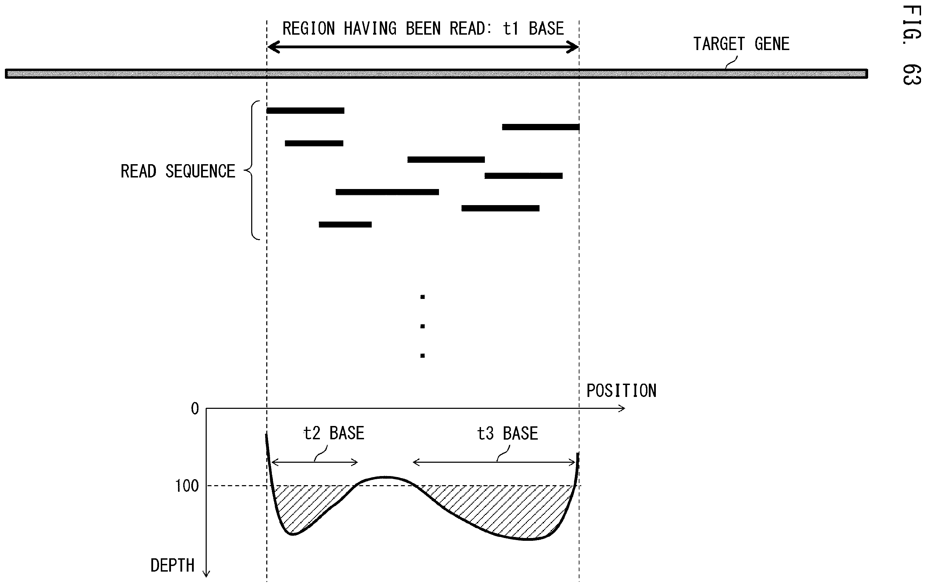

The above-described quality evaluation indexes are described with reference to FIG. 63.

Index (i-1): quality score

The quality score is an index indicating the accuracy of each base in the gene sequence read by the sequencer 2.

For example, when the read sequence information is outputted as a FASTQ file from the sequencer 2, the quality score is also included in the read sequence information (see FIG. 30). Since the quality score has already been described in detail, the description thereof is omitted here.

Index (i-2): cluster concentration

The sequencer 2 locally amplifies and immobilizes a large number of single-stranded DNA fragments on a flow cell to form a cluster (see "9" in FIG. 27). An image of the cluster group on the flow cell is taken by using a fluorescence microscope, and fluorescent colors (that is, fluorescences having different wavelengths) respectively corresponding to A, C, G, T are detected to read the sequence. The cluster density is an index indicating a degree to which the clusters of each gene formed on the flow cell are close to each other when the sequencing is performed.

For example, in a case where the cluster density becomes excessively high, and the clusters are excessively close to each other or overlap each other, the contrast of the taken image of the flow cell, i.e., the S/N ratio, is lowered, whereby focusing by the fluorescence microscope becomes difficult. Therefore, fluorescence cannot be accurately detected. As a result, the sequence cannot be accurately read.

Index (ii): quality evaluation index indicating the proportion of bases in a target region read by the sequencer 2, to bases read by the sequencer 2.

This index indicates how many bases in the target region have been read, among bases (also including bases other than those in the target region) read by the sequencer 2. This index can be calculated as a ratio between the total number of bases in the target region and the total number of bases having been read.

Index (iii): quality evaluation index indicating the depth of read sequence information.

This index is an index based on the total number of pieces of the read sequence information obtained by reading the bases included in a gene to be analyzed. This index can be calculated as a ratio between the total number of bases having depths greater than or equal to a predetermined value among the bases having been read, and the total number of bases having been read.

The depth means the total number of pieces of read sequence information having been read for one base.

FIG. 63 shows a graph indicating the depth for each base having been read in a case where T base represents the entire length of the gene to be analyzed and t1 base represents the base in the read region. In this graph, the horizontal axis represents the position of each base, and the vertical axis represents the depth of each base. In the example shown in FIG. 63, in the t1 base in the region having been read, the total number of bases in the region in which the depth is greater than or equal to a predetermined value (for example, 100) is (t2+t3) bases. In this case, index (iii) is generated as a value of (t2+t3)/t1.

Index (iv): quality evaluation index indicating variation in the depth of read sequence information.

This index is an index indicating the uniformity of the depth. When the number of pieces of the read sequence information having been read in a certain portion in the region having been read is extremely great, uniformity of the depth is low. When the read sequence information is relatively uniform over the region having been read, the uniformity of the depth is high. The uniformity of the depth is not limited thereto. For example, the uniformity can be expressed as a number by using the interquartile range (IQR). The greater the IQR is, the lower the uniformity is. The lower the IQR is, the higher the uniformity is.

Index (v): quality evaluation index indicating whether or not all the mutations in standard genes included in the quality control sample have been detected.

This index is an index indicating that the mutations in standard genes included in the quality control sample have been detected and accurately identified. For example, mutations (see the column of "Variant") in standard genes included in the quality control sample A shown in FIG. 21A and the quality control sample B shown in FIG. 21B are known mutations. The index for evaluating whether or not the position of the mutation, the type of the mutation, and the like have been accurately identified is used as the quality evaluation index.

The present disclosure is not limited to the above-described embodiments, and various modifications can be made without departing from the scope of the claims. Embodiments obtained by combining as appropriate technological means disclosed in different embodiments are also included in the technological scope of the present disclosure.

The information processing apparatus 1 is a computer which performs commands of a program which is software that realizes each function. This computer includes one or more processors, for example, and also includes a computer-readable storage medium having the program stored therein. In the computer, the processor reads the program from the storage medium and performs the program, whereby the object of the present disclosure is achieved. As the processor, a CPU (Central Processing Unit) can be used, for example. As the storage medium, a "non-transitory and tangible medium", such as ROM (Read Only Memory), tape, disk, card, semiconductor memory, or programmable logical circuit, can be used. The computer may further include a RAM (Random Access Memory) or the like onto which the program is developed. The program may be supplied to the computer via a desired transmission medium (communication network, broadcast wave, or the like) that can transmit the program. One aspect of the present disclosure can also be realized in the form of a data signal which is realized by electronic transmission of the program and which is embedded in a carrier wave.

**Claims**

1. An analysis method for analyzing a nucleic acid sequence of a sample by using a computer, the analysis method comprising:

   detecting a predetermined mutation on the basis of sequence information having been read from the nucleic acid sequence; and
   creating, in accordance with a disease that corresponds to the sample, a report that includes information related to efficacy of a predetermined drug that corresponds to the predetermined mutation.

2. The analysis method of claim 1, further comprising identifying the disease that corresponds to the sample, wherein
   the report that includes the information related to the efficacy of the predetermined drug that corresponds to the predetermined mutation is created in accordance with identification of a predetermined disease.

3. The analysis method of claim 1, further comprising identifying the disease that corresponds to the sample, wherein
   the report that includes the information related to the efficacy of the predetermined drug that corresponds to the predetermined mutation is created in accordance with detection of the predetermined mutation in a predetermined disease.

4. The analysis method of any one of claims 1 to 3, further comprising
   determining presence or absence of the predetermined mutation related to the efficacy of the predetermined drug and another mutation not related to the efficacy of the predetermined drug, on the basis of the sequence information.

5. The analysis method of any one of claims 1 to 4, wherein
   the report is created such that the predetermined mutation related to the efficacy of the predetermined drug and another mutation not related to the efficacy of the predetermined drug are distinct from each other.

6. The analysis method of any one of claims 1 to 5, wherein
   the report is created such that the predetermined mutation and the information related to the efficacy of the predetermined drug are associated with each other.

7. The analysis method of any one of claims 1 to 6, wherein
   the report includes a first region for showing the predetermined mutation related to the efficacy of the predetermined drug and a second region for showing another mutation not related to the efficacy of the predetermined drug.

8. The analysis method of any one of claims 1 to 7, wherein
   the report includes a first report that includes the predetermined mutation related to the efficacy of the predetermined drug and a second report that includes another mutation not related to the efficacy of the predetermined drug.

9. The analysis method of any one of claims 1 to 8, wherein
   the report is created such that an icon that indicates presence of relation to the efficacy of the predetermined drug is associated with the predetermined mutation.

10. The analysis method of any one of claims 1 to 9, wherein
    the report includes information indicating that the predetermined mutation is usable in selection of a subject to whom the predetermined drug has a possibility of being effective.

11. The analysis method of any one of claims 1 to 10, wherein
    the report is created in accordance with a quality evaluation index satisfying a predetermined criterion.

12. The analysis method of claim 11, wherein
    the quality evaluation index indicates at least one of:

accuracy of reading of each base in the sequence information performed by a sequencer; a depth of the sequence information having been read from the nucleic acid sequence; and variation in a depth of the sequence information having been read from the nucleic acid sequence.

13. The analysis method of claim 11, wherein the sequence information is information read from a cluster group of the nucleic acid sequence having been amplified on a flow cell, and the quality evaluation index indicates a degree of closeness between clusters in the cluster group.

14. The analysis method of any one of claims 11 to 13, wherein the report that includes information indicating that an analysis result of the nucleic acid sequence is reference information is created, or the report that does not include the information related to the efficacy of the predetermined drug is created, or the report that includes information indicating that the detected predetermined mutation is not usable in selection of a subject to whom the predetermined drug has a possibility of being effective is created, in accordance with the quality evaluation index not satisfying the predetermined criterion.

15. The analysis method of any one of claims 1 to 14, further comprising reading the sequence information from at least one of (i) the sample corresponding to a predetermined disease and (ii) the sample corresponding to a disease other than the predetermined disease, wherein a presence of the predetermined mutation in the sample corresponding to the predetermined disease indicates an effectiveness of treatment by the predetermined drug.

16. The analysis method of any one of claims 1 to 15, wherein in case where the predetermined mutation is detected in the sample corresponding to a predetermined disease, the report indicating that the detected predetermined mutation is indicative of an effectiveness of treatment by the predetermined drug is created.

17. The analysis method of claim 16, wherein in case where the predetermined mutation is detected in the sample corresponding to a disease other than the predetermined disease, the report indicating that the detected predetermined mutation is irrelevant to an effectiveness of treatment by the predetermined drug is created.

18. An information processing apparatus for analyzing a nucleic acid sequence of a sample, the information processing apparatus comprising a controller programmed to:

obtain sequence information having been read from the nucleic acid sequence; detect a predetermined mutation in the nucleic acid sequence on the basis of the sequence information; identify a disease that corresponds to the sample; and create, in accordance with identification of a predetermined disease, a report that includes information related to efficacy of a predetermined drug that corresponds to the predetermined mutation.

19. A non-transitory computer readable medium storing programs executable by a processor to:

detect a predetermined mutation in a nucleic acid sequence of a sample, on the basis of sequence information having been read from the nucleic acid sequence; and create, in accordance with a disease that corresponds to the sample, a report that includes information related to efficacy of a predetermined drug that corresponds to the predetermined mutation.

20. An analysis method for analyzing a nucleic acid sequence of a sample by using a computer, the analysis method comprising:

detecting presence of a predetermined mutation in a predetermined set of genes on the basis of sequence information having been read from the nucleic acid sequence, wherein at least one of the genes correspond to the predetermined mutation to be used for indicating an effectiveness of treatment by a predetermined drug; and creating, if one or more of the predetermined mutations are detected in the sample corresponding to a predetermined disease, a report that includes information related to the effectiveness of treatment by the predetermined drug.

21. An analysis method for analyzing a nucleic acid sequence of a sample by using a computer, the analysis method comprising:

detecting presence of a predetermined mutation in a predetermined set of genes on the basis of sequence information having been read from the nucleic acid sequence, wherein at least one of the genes correspond to the predetermined mutation to be used for indicating an effectiveness of treatment by a predetermined drug; and creating a report selectively indicating (i) information of the presence of the predetermined mutation with a relation to the effectiveness of treatment by the predetermined drug, or (ii) information of the presence of the predetermined mutation without the relation to the effectiveness of treatment by the predetermined drug.

FIG. 1

```
                    ┌─────────────┐
                    │    START    │
                    └──────┬──────┘
                           │
                         ⌐ S1
                     ╱───────────╲
                    ╱ PREDETERMINED ╲        NO
                   ╱  GENE MUTATION IS ╲───────────────────┐
                   ╲    DETECTED ?   ╱                      │
                    ╲───────────╱                           │
                         │                                  │
                        YES                                 │
                         │                                  │
                       ⌐ S2                                 │
                     ╱───────────╲                          │
                    ╱   SAMPLE     ╲        NO               │
                   ╱ OF PREDETERMINED ╲──────────────────┐  │
                   ╲   DISEASE ?    ╱                     │  │
                    ╲───────────╱                         │  │
                         │                                │  │
                        YES                               ▼  ▼
                         │                      
         ⌐ S3                              ⌐ S4
  ┌────────────────────────┐         ┌────────────────────────┐
  │  CREATE REPORT INCLUDING │       │  CREATE REPORT THAT ALLOWS │
  │  INFORMATION RELATED TO  │       │  DISCERNMENT THAT NO GENE  │
  │    EFFICACY OF DRUG      │       │  MUTATION TO BE USED FOR   │
  │                          │       │   CDx HAS BEEN DETECTED    │
  └────────────┬─────────────┘       └────────────┬─────────────┘
               │                                   │
               │◄──────────────────────────────────┘
               │
        ┌──────┴──────┐
        │     END     │
        └─────────────┘
```

# FIG. 2

INFORMATION PROCESSING APPARATUS ⌐1

SEQUENCER ⌐2

# FIG. 3

TEST INSTITUTION ID,
GENE PANEL ID, GENE ID,
ANALYSIS RECORD,
QUALITY EVALUATION INDEX

⌐4

⌐130

TEST INSTITUTION ID,
GENE PANEL ID, GENE ID,
ANALYSIS RECORD,
QUALITY EVALUATION INDEX

MANAGEMENT SERVER ⌐3

ANALYSIS SYSTEM
MANAGEMENT INSTITUTION

ANALYSIS REQUEST
⌐210 (GENE PANEL INFORMATION) ⌐120

MEDICAL INSTITUTION | SAMPLE SAMPLE ID | TEST INSTITUTION

⌐5

COMMUNICATION TERMINAL

⌐100

⌐1
INFORMATION PROCESSING APPARATUS

SAMPLE ID
READ SEQUENCE INFORMATION,
QUALITY EVALUATION INDEX,
(QUALITY SCORE,
CLUSTER CONCENTRATION)

⌐2
SEQUENCER

SAMPLE ID
REPORT
(ANALYSIS RESULT, AUXILIARY INFORMATION)

EP 3 588 508 A1

FIG. 4

**MEDICAL INSTITUTION** (210)

COMMUNICATION TERMINAL 5

**TEST INSTITUTION** (120)

SEQUENCER 2    INFORMATION PROCESSING APPARATUS 1

**ANALYSIS SYSTEM MANAGEMENT INSTITUTION** (130)

MANAGEMENT SERVER 3

S101 — FILE APPLICATION FOR USE OF GENE ANALYSIS SYSTEM

→ TEST INSTITUTION ID VARIOUS SERVICES →

S102 — SPECIFY CONTRACT TYPE

S104 — RECEIVE VARIOUS SERVICES

← VARIOUS SERVICES / CDx INFORMATION ←

S103 — START PROVIDING VARIOUS SERVICES

ANALYSIS REQUEST (GENE PANEL INFORMATION), SAMPLE ID

S105 — TRANSMIT ANALYSIS REQUEST

SAMPLE, SAMPLE ID ⇒

S106 — RECEIVE ANALYSIS REQUEST

READ SEQUENCE INFORMATION, QUALITY EVALUATION INDEX

S107 — RECEIVE INPUT OF GENE PANEL INFORMATION

S108 — SEQUENCING

S109 — ANALYZE GENE SEQUENCE

(QUALITY SCORE, CLUSTER CONCENTRATION)

S110 — GENERATE QUALITY EVALUATION INDEX BASED ON ANALYSIS RESULT OF QUALITY CONTROL SAMPLE

SAMPLE ID, REPORT (ANALYSIS RESULT, AUXILIARY INFORMATION)

S111 — CREATE REPORT

S113 — RECEIVE REPORT

S112 — TRANSMIT REPORT

CHARGE ANALYSIS FEE ⇐

S114 — MAKE NOTIFICATION OF GENE PANEL INFORMATION OF GENE PANEL USED IN ANALYSIS, INFORMATION RELATED TO ANALYZED GENE, ANALYSIS RECORD, AND QUALITY EVALUATION INDEX

→ TEST INSTITUTION ID, GENE PANEL ID, GENE ID, ANALYSIS RECORD, QUALITY EVALUATION INDEX →

S115 — STORE ANALYSIS RECORD AND QUALITY EVALUATION INDEX

CHARGE SYSTEM USAGE FEE ⇐

S116 — DETERMINE SYSTEM USAGE FEE

36

# FIG. 5

3

### 3A

| TEST INSTITUTION NAME | TEST INSTITUTION ID |
|---|---|
| INSTITUTION P | ppppp |
| INSTITUTION Q | qqqqq |
| INSTITUTION R | rrrrr |
| ... | |

### 3C

| GENE-PANEL-RELATED INFORMATION | THE NUMBER OF TIMES OF OPERATION | PERIOD |
|---|---|---|
| PANEL ID: AAA | 5 | 2017/8/1 - 2017/8/31 |
| PANEL ID: BBB | 10 | 2017/8/1 - 2017/8/31 |
| PANEL ID: CCC | 0 | 2017/8/1 - 2017/8/31 |
| ... | ... | ... |

### 3D

| GENE-PANEL-RELATED INFORMATION | THE NUMBER OF ANALYZED GENES | PERIOD |
|---|---|---|
| PANEL ID: AAA | 4178 | 2017/8/1 - 2017/8/31 |
| PANEL ID: BBB | 2734 | 2017/8/1 - 2017/8/31 |
| PANEL ID: CCC | 153 | 2017/8/1 - 2017/8/31 |
| ... | ... | ... |

### 3E

| GENE-PANEL-RELATED INFORMATION | TOTAL NUMBER OF IDENTIFIED MUTATIONS | PERIOD |
|---|---|---|
| PANEL ID: AAA | 2610 | 2017/8/1 - 2017/8/31 |
| PANEL ID: BBB | 518 | 2017/8/1 - 2017/8/31 |
| PANEL ID: CCC | 94 | 2017/8/1 - 2017/8/31 |
| ... | ... | ... |

### 3F

| NAME OF COMPANY PROVIDING GENE PANEL | PANEL ID | NECESSITY OF SYSTEM USAGE FEE |
|---|---|---|
| COMPANY A | AAA, ... | UNNECESSARY |
| COMPANY B | BBB, ... | NECESSARY |
| COMPANY C | CCC, ... | NECESSARY |
| ... | ... | ... |

### 3B

| CONTRACT TYPE | USABLE GENE PANEL | SYSTEM USAGE FEE |
|---|---|---|
| PLAN 1 | PANEL A PANEL B PANEL C ... | DETERMINE ACCORDING TO THE NUMBER OF TIMES OF OPERATION |
| PLAN 2 | PANEL A PANEL B PANEL C ... | DETERMINE ACCORDING TO THE NUMBER OF ANALYZED GENES |
| CDx USAGE PLAN 3 | PANEL A PANEL B PANEL C ... | DETERMINE ACCORDING TO THE NUMBER OF TIMES OF OPERATION |
| CDx USAGE PLAN 4 | PANEL A PANEL B PANEL C ... | DETERMINE ACCORDING TO THE NUMBER OF ANALYZED GENES |
| ... | ... | |

FIG. 6

EP 3 588 508 A1

SEQUENCER — 2

READ SEQUENCE INFORMATION
QUALITY EVALUATION INDEX
(QUALITY SCORE, CLUSTER
CONCENTRATION)

INPUT UNIT — 17

INFORMATION
PROCESSING APPARATUS — 1

GENE PANEL INFORMATION

CONTROLLER — 11

STORAGE UNIT — 12

SEQUENCE DATA
READING UNIT — 111

INFORMATION
SELECTION UNIT — 112

GENE-PANEL-RELATED
INFORMATION
DATABASE — 121

DATA ADJUSTMENT UNIT — 113

QUALITY-CONTROL
UNIT — 117

REFERENCE
SEQUENCE DATABASE — 122

MUTATION
IDENTIFICATION UNIT — 114

MUTATION DATABASE — 123

DRUG SEARCH UNIT — 118

DRUG DATABASE — 124

OUTPUT UNIT — 13

REPORT CREATION UNIT — 115

QUALITY EVALUATION
CRITERIA — 126

ANALYSIS EXECUTION UNIT — 110

GENE PANEL ID
GENE ID
ANALYSIS RECORD
QUALITY EVALUATION INDEX

MANAGEMENT SERVER — 3

COMMUNICATION
UNIT — 14

MANAGEMENT UNIT — 116

ANALYSIS RECORD
LOG — 151

FIG. 7

```
      ( RECEIVE INPUT OF GENE )  ─ S107
      (   PANEL INFORMATION   )
                 │
        ┌────────────────┐
        │ OBTAIN GENE PANEL │  ─ S201
        │   INFORMATION     │
        └────────────────┘
                 │
              ◇ S202
         ◇  REGISTERED  ◇  ──── NO ───┐
         ◇ GENE PANEL ? ◇             │
              ◇                       │
              YES                     │
               │                      │
              ◇ S203                  │
         ◇  DESIGNATED  ◇  ── NO ──►  │
         ◇ GENE PANEL ? ◇             │
              ◇                       │
              YES                     │
               │                      │
    ┌────────────── S204    ┌────────────── S205
    │ INDICATE THAT GENE PANEL │    │ INDICATE THAT GENE PANEL │
    │     CAN BE USED          │    │    CANNOT BE USED        │
    └──────────────────────┘    └──────────────────────┘
               │                       │
               │◄──────────────────────┘
               │
          (  RETURN  )
```

FIG. 8

| | |
|---|---|
| ◯ | GENE PANEL 1 (GENE PANEL NAME: "xxxxx") |
| ◉ | GENE PANEL 2 (GENE PANEL NAME: "yyyyy") |
| ◯ | GENE PANEL 3 (GENE PANEL NAME: "zzzzzz") |
| | ⋮ |
| ◯ | UNREGISTERED PANEL |

## FIG. 9

121

121A

| GENE NAME | GENE ID |
|-----------|---------|
| . . . | . . . |
| EGFR | aaa |
| BRAF | bbb |
| Ros1 | ccc |
| . . . | . . . |

121B

| GENE PANEL NAME | GENE PANEL ID | RELATED GENE ID | CDx FLAG |
|-----------------|---------------|-----------------|----------|
| . . . | . . . | . . . | . . . |
| PANEL A | AAA | aaa, bbb, ccc, · · · | 1 |
| PANEL B | BBB | aaa, bbb, ccc, ddd, · · · | 1 |
| PANEL C | CCC | aaa, ccc, · · · | 0 |
| . . . | . . . | . . . | . . . |

## FIG. 10

| ☑ AKT1 | ☑ EGFR | ☐ JAK3 | · · · |
|--------|--------|--------|-------|
| ☑ APC | ☑ ESR1 | ☑ KIT | · · · |
| ☑ ALK | ☐ EML4 | ☑ MET | · · · |
| ☑ BRAF | ☐ FBXW7 | ☑ PIK3CA | · · · |
| · · · | · · · | · · · | · · · |

FIG. 11

○ LUNG CANCER

● COLON CANCER

○ BREAST CANCER

⋮

FIG. 12

REGISTRATION FILE NAME

| GENE PANEL TARGET GENE.csv | REGISTER |

| RET |
| CHEK2 |
| PTEN |
| MEK1 |

FIG. 13

GENE NAME

| FBXW7 | REGISTER |

FIG. 14

START

PRETREATMENT — S31

SEQUENCING — S32

ANALYZE GENE SEQUENCE — S33

EVALUATE QUALITY OF PANEL TEST — S34

CREATE REPORT — S35

END

FIG. 15

```
        ┌─────────────────────┐
        │        START        │
        └─────────────────────┘
                   │
   ┌───────────────────────────────────┐
   │ EXTRACT DNA OF SAMPLE AND QUALITY  │   S300
   │ CONTROL SAMPLE CORRESPONDING TO GENE│
   │        PANEL TO BE USED            │
   └───────────────────────────────────┘
                   │
   ┌───────────────────────────────────┐
   │      FRAGMENT EXTRACTED DNA        │   S301
   └───────────────────────────────────┘
                   │
   ┌───────────────────────────────────┐
   │      PROVIDE ADAPTER SEQUENCE      │   S302
   └───────────────────────────────────┘
                   │
   ┌───────────────────────────────────┐
   │            HYBRIDIZE               │   S303
   └───────────────────────────────────┘
                   │
   ┌───────────────────────────────────┐
   │       COLLECT DNA FRAGMENT         │   S304
   └───────────────────────────────────┘
                   │
        ┌─────────────────────┐
        │         END         │
        └─────────────────────┘
```

# FIG. 16

```
        ┌──────────────────────┐
        │        START         │
        └──────────────────────┘
                   │
   ┌───────────────────────────────────┐
   │ EXTRACT DNA OF QUALITY CONTROL SAMPLE │ ╭ S300a
   └───────────────────────────────────┘
                   │
   ┌───────────────────────────────────┐
   │      FRAGMENT EXTRACTED DNA        │ ╭ S301
   └───────────────────────────────────┘
                   │
   ┌───────────────────────────────────┐
   │      PROVIDE ADAPTER SEQUENCE      │ ╭ S302
   └───────────────────────────────────┘
                   │
   ┌───────────────────────────────────┐
   │            HYBRIDIZE               │ ╭ S303
   └───────────────────────────────────┘
                   │
   ┌───────────────────────────────────┐
   │       COLLECT DNA FRAGMENT         │ ╭ S304
   └───────────────────────────────────┘
                   │
        ┌──────────────────────┐
        │         END          │
        └──────────────────────┘
```

## FIG. 17

```
              ┌─────────────────────┐
              │        START        │
              └─────────────────────┘
                         │
    ┌─────────────────────────────────────────┐
    │  EXTRACT DNA OF QUALITY CONTROL SAMPLE   │  ⌐ S300b
    │  INCLUDING MUTATION AND QUALITY CONTROL  │
    │     SAMPLE NOT INCLUDING MUTATION        │
    └─────────────────────────────────────────┘
                         │
    ┌─────────────────────────────────────────┐
    │          FRAGMENT EXTRACTED DNA          │  ⌐ S301
    └─────────────────────────────────────────┘
                         │
    ┌─────────────────────────────────────────┐
    │          PROVIDE ADAPTER SEQUENCE        │  ⌐ S302
    └─────────────────────────────────────────┘
                         │
    ┌─────────────────────────────────────────┐
    │              HYBRIDIZE                    │  ⌐ S303
    └─────────────────────────────────────────┘
                         │
    ┌─────────────────────────────────────────┐
    │          COLLECT DNA FRAGMENT            │  ⌐ S304
    └─────────────────────────────────────────┘
                         │
              ┌─────────────────────┐
              │         END         │
              └─────────────────────┘
```

FIG. 18

```
         ┌──────────────────┐
         │      START       │
         └──────────────────┘
                  │
  ┌───────────────────────────────────┐
  │ EXTRACT DNA OF SAMPLE AND QUALITY  │  ⌠ S300c
  │         CONTROL SAMPLE             │
  │ (INCLUDING/NOT INCLUDING MUTATION) │
  └───────────────────────────────────┘
                  │
  ┌───────────────────────────────────┐
  │      FRAGMENT EXTRACTED DNA        │  ⌠ S301
  └───────────────────────────────────┘
                  │
  ┌───────────────────────────────────┐
  │       PROVIDE ADAPTER SEQUENCE     │  ⌠ S302
  └───────────────────────────────────┘
                  │
  ┌───────────────────────────────────┐
  │            HYBRIDIZE               │  ⌠ S303
  └───────────────────────────────────┘
                  │
  ┌───────────────────────────────────┐
  │       COLLECT DNA FRAGMENT         │  ⌠ S304
  └───────────────────────────────────┘
                  │
         ┌──────────────────┐
         │       END        │
         └──────────────────┘
```

FIG. 19A

| PIK3CA | EGFR | IDH1 | ROS1 | · · · |
|--------|------|------|------|-------|
| ESR1 | KRAS | IDH2 | ALK | · · · |
| BRAF | PDGFRA | cKIT | AKT1 | · · · |
| MEK | RET | · · · | · · · | · · · |
| S492R | · · · | · · · | · · · | · · · |
| · · · | · · · | · · · | · · · | · · · |
| · · · | · · · | · · · | · · · | · · · |

FIG. 19B

| Gene Valiant Type in Profiling | | | |
|------|-------|-----|--------|
| SNV | Indel | CNV | Fusion |

FIG. 19C

| Mutation Type | Gene | Mutation |
|---------------|------|----------|
| SNV | AAA | aaa |
| InDel | BBB | bbb |
| CNV | CCC | ccc |
| Fusion | DDD | ddd |

FIG. 19D

QUALITY CONTROL
SAMPLE A

| Variant Type | Chromosome Number | Gene | Variant | Expected Allele Freq. |
|--------------|-------------------|------|---------|------------------------|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

FIG. 20

<table>
<tr><td colspan="3" style="text-align:right">⌐121<br>⌐121D</td></tr>
<tr><th>GENE PANEL</th><th>QUALITY CONTROL SAMPLE<br>INCLUDING MUTATION</th><th>QUALITY CONTROL SAMPLE<br>NOT INCLUDING MUTATION</th></tr>
<tr><td>PANEL A</td><td>STANDARD SAMPLE A1<br>STANDARD SAMPLE A2<br>.<br>.<br>.</td><td>STANDARD SAMPLE M1<br>STANDARD SAMPLE M2<br>.<br>.<br>.</td></tr>
<tr><td>PANEL B</td><td>STANDARD SAMPLE B1<br>STANDARD SAMPLE B2<br>.<br>.<br>.</td><td>STANDARD SAMPLE N1<br>STANDARD SAMPLE N2<br>.<br>.<br>.</td></tr>
</table>

# FIG. 21A

QUALITY CONTROL
SAMPLE A

| Variant Type | Chromosome Number | Gene | Variant | Expected Allele Freq. |
|---|---|---|---|---|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

a1
a2
a3

# FIG. 21B

QUALITY CONTROL
SAMPLE B

| Variant Type | Chromosome Number | Gene | Variant | Expected Allele Freq. |
|---|---|---|---|---|
| Verified Mutations | | | | |
| SNV High GC | chr.19 | GNA11 | R183C | 5.9 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.16 | TSC2 | ΔH1746-R1751 | 5.5 |
| Fusion | chr.4:chr.6 | ROS1 | SLC34A2/ROS1 fusion | 5.6 |
| Fusion | chr.10 | RET | CCDC6/RET fusion | 5.0 |
| Fusion | chr.9 | ABL1 | BCR/ABL1 fusion | 5.1 copies |
| CNV | chr.2 | MYC-N | amplification | 9.5 copies |
| CNV | chr.8 | MYC-C | amplification | 9.8 copies |

b1
b2
b3

# FIG. 22A

## FRAGMENTATION OF DNA

### DNA OF SAMPLE AND QUALITY CONTROL SAMPLE

FRAGMENTATION

# FIG. 22B

## PROVISION OF ADAPTER SEQUENCE

DNA FRAGMENT

ADAPTER 1 SEQUENCE          ADAPTER 2 SEQUENCE

DNA FRAGMENT

ADAPTER 1 SEQUENCE          ADAPTER 2 SEQUENCE

INDEX SEQUENCE

FIG. 23

FIG. 24

COLLECT DNA FRAGMENT TO BE ANALYZED

STREPTAVIDIN MAGNETIC BEAD

STREPTAVIDIN

BIOTIN

MAGNETIC BEAD

WASH AND COLLECT

WASH AND REMOVE DNA FRAGMENTS
THAT WERE NOT HYBRIDIZED

COLLECT HYBRIDIZED DNA FRAGMENT
BY MAGNET

MAGNET

FIG. 25

```
            ┌─────────────────┐
            │      START       │
            └─────────────────┘
                     │
    ┌────────────────────────────────┐
    │      APPLY TO FLOW CELL          │⌇ S305
    └────────────────────────────────┘
                     │
    ┌────────────────────────────────┐
    │ AMPLIFY DNA FRAGMENT TO BE ANALYZED │⌇ S306
    └────────────────────────────────┘
                     │
    ┌────────────────────────────────┐
    │        READ SEQUENCE             │⌇ S307
    └────────────────────────────────┘
                     │
            ┌─────────────────┐
            │       END        │
            └─────────────────┘
```

FIG. 26

# FIG. 27

AMPLIFY DNA FRAGMENT TO BE ANALYZED (Bridge PCR)

# FIG. 28

FIG. 29

```
          ┌─────────────────┐
          │      START      │
          └────────┬────────┘
                   │
    ┌──────────────┴──────────────┐  ┌ S11
    │   READ READ SEQUENCE DATA   │
    └──────────────┬──────────────┘
                   │
    ┌──────────────┴──────────────┐  ┌ S12
    │      PERFORM ALIGNMENT       │
    └──────────────┬──────────────┘
                   │
    ┌──────────────┴──────────────┐  ┌ S13
    │  COMPARE ALIGNMENT SEQUENCE OF │
    │  BLOOD SAMPLE WITH ALIGNMENT   │
    │  SEQUENCE OF TUMOR SAMPLE      │
    └──────────────┬──────────────┘
                   │
    ┌──────────────┴──────────────┐  ┌ S14
    │   EXTRACT DIFFERENCE BETWEEN  │
    │     SEQUENCES AS MUTATION     │
    └──────────────┬──────────────┘
                   │
    ┌──────────────┴──────────────┐  ┌ S15
    │    SEARCH MUTATION DATABASE,  │
    │  BASED ON EXTRACTED MUTATION  │
    └──────────────┬──────────────┘
                   │
    ┌──────────────┴──────────────┐  ┌ S16
    │ PROVIDE ANNOTATION TO EXTRACTED │
    │ MUTATION, BASED ON SEARCH RESULT│
    └──────────────┬──────────────┘
                   │
          ┌────────┴────────┐
          │       END       │
          └─────────────────┘
```

FIG. 30

```
┌────────────────────────────────────────────────────────┐
│ • SEQUENCE NAME: "Read Seq 1"                          │
│ • SEQUENCE:      ···GTAAGGCACGTCATA···                  │
│ • QUALITY SCORE:              ···xxxxxyzxxyzzxxx···     │
└────────────────────────────────────────────────────────┘
```

FIG. 31A

REFERENCE
SEQUENCE

MAPPING

READ SEQUENCE

EP 3 588 508 A1

FIG. 31B

· REFERENCE SEQUENCE INFORMATION: REFERENCE SEQUENCE NAME (REFERENCE SEQUENCE ID), SEQUENCE LENGTH OF REFERENCE SEQUENCE, etc.

· READ SEQUENCE NAME: NAME (READ SEQUENCE ID) OF EACH READ SEQUENCE HAVING BEEN SUBJECTED TO ALIGNMENT

· POSITION INFORMATION: LEFT MOST MAPPING POSITION.  POSITION ON REFERENCE SEQUENCE AT WHICH LEFTMOST BASE OF READ SEQUENCE WAS MAPPED

· MAP QUALITY: MAPPING QUALITY CORRESPONDING TO THE READ SEQUENCE

· SEQUENCE: BASE SEQUENCE CORRESPONDING TO EACH READ SEQUENCE (EXAMPLE: ···GTAAGGCACGTCATA···)

# FIG. 32

r-122

| WILD TYPE SEQUENCE | REFERENCE SEQUENCE HAVING KNOWN MUTATION SEQUENCE INCORPORATED |

GENOME SEQUENCE OF CHROMOSOME #1

CHROMOSOME #2

CHROMOSOME #3

CHROMOSOME #23

MUTATION SEQUENCE

MUTATION SEQUENCE

# FIG. 33

| GENE NAME | CHROMOSOME POSITION | MUTATION |
|-----------|---------------------|----------|
| AKT1 | 14q32. 33 | p. E17K |
| BRAF | 7q34 | p. V600E, p. V600K, p. V600, p. V600R, p. K601E, p. D594G, p. G469A, p. D594N, p. V600M, p. V600_K601>E, p. G466V, p. V600D, p. L597R, p. G469V, p. N581S, p. V600L, p. G469R, p. G469E, p. G466E, p. L597Q, p. L597S, p. V600A |
| EGFR | 7p11. 2 | p. L858R, p. E746_A750del, p. E746_A750delELREA, p. T790M, p. L861Q, p. G719, p. S768I, p. L747_P753>S, p. G719A, p. L747_T751del, p. L747_A750>P, p. G719S, p. L747_T751delLREAT, p. E746_S752>V, c. 3633+294delA, p. V769_D770insASV, p. L747_S752del, p. G719C, p. L747_S752delLREATS, p. G598V, p. A289V, c. 3633+293_3633+294delAA, p. L747_P753del, p. D770_N771insSVD, p. E746_T751del, p. L747_T751>P, p. E709K, p. E746_T751>A, p. L833V, p. N158N, p. K745_A750del, p. H773_V774insNPH, p. C797S |
| . . . | . . . | . . . |

FIG. 34

START

COMPARE READ SEQUENCE WITH
REFERENCE SEQUENCE — S401

SPECIFY POSITION ON REFERENCE
SEQUENCE AT WHICH DEGREE OF
MATCHING WITH READ SEQUENCE
SATISFIES PREDETERMINED CRITERION — S402

MATCH
AT PLURALITY OF
POSITIONS ? — S403

YES

NO

ALIGN READ SEQUENCE TO POSITION
OF HIGHEST DEGREE OF MATCHING — S404

ALL READ
SEQUENCES HAVE BEEN
ALIGNED ? — S405

NO

YES

END

FIG. 35A

```
REFERENCE SEQUENCE:  · · · GCCATGGACAGAAGGCGCAGGGC · · ·
READ SEQUENCE R1:          GCCATGGACAGAA
READ SEQUENCE R2:          GCCATGCACAGAA
```

FIG. 35B

```
REFERENCE SEQUENCE:  · · · GCCATGGACAGAAGGCGCAGGGC · · ·
READ SEQUENCE R3:          GCCATGGACAG**GGCG
READ SEQUENCE R4:          GCCATGGACAGAAGGCG
                                        CGGT
```

FIG. 36

EP 3 588 508 A1

· POSITION INFORMATION: POSITION INFORMATION ON REFERENCE GENOME.  FOR EXAMPLE, SPECIFIED BY
  CHROMOSOME NUMBER AND POSITION ON THE CHROMOSOME.
· REFERENCE BASE: REFERENCE BASE (A, T, C, G, etc.) IN THE POSITION INFORMATION
· MUTATION BASE: BASE OF REFERENCE BASE AFTER MUTATION

| CHROM | POS | REF | ALT | |
|---|---|---|---|---|
| 20 | 3 | C | G | |
| 21 | 4 | C | CTAG | ← EXAMPLE OF INSERTION MUTATION |
| 19 | 10 | TCG | T | ← EXAMPLE OF DELETION MUTATION |
| 2 | 321681 | G | G]17:198982] | ← REVERSE COMPLEMENT SEQUENCE OF SEQUENCE EXTENDING AT LEFT OF 198982 POSITION OF CHROMOSOME 17 IS BOUND AFTER "G" |
| 2 | 321682 | T | ]13:123456]T | ← SEQUENCE AT LEFT OF 123456 POSITION OF CHROMOSOME 13 IS BOUND BEFORE "T" |
| 13 | 123456 | C | C[2:321682[ | ← SEQUENCE AT RIGHT OF 321682 POSITION OF CHROMOSOME 2 IS BOUND AFTER "C" |
| 13 | 123457 | A | [17:198983[A | ← REVERSE COMPLEMENT SEQUENCE OF SEQUENCE EXTENDING AT RIGHT OF 198983 POSITION OF CHROMOSOME 17 IS BOUND AFTER "C" |

FIG. 37

123

| MUTATION ID | CHROM | POS | REF | ALT | Annotation | METADATA RELATED TO GENE-PANEL-RELATED INFORMATION |
|---|---|---|---|---|---|---|
| #1 | 20 | 3 | C | G | abc | GENE ID: a |
| #2 | 19 | 4 | A | T | xyz | GENE ID: b |
| #3 | xx | yy | C | G | EGFR L858R | GENE ID: c |
| #4 | aa | bb | T | A | BRAF V600E | GENE ID: d |
| ・・・ | | | | | | |
| #xx | abc | ABC | G | G]p] | ALK-EML4 FUSION | GENE ID: e, GENE ID: f |
| #yy | xyz | XYZ | T | ]p]T | ROS1-CD74 FUSION | GENE ID: g, GENE ID: h |
| ・・・ | | | | | | |

EP 3 588 508 A1

FIG. 38

- MUTATION ID: IDENTIFIER FOR IDENTIFYING MUTATION
- MUTATION POSITION INFORMATION:
  - ➢ CHROM: CHROMOSOME NUMBER
  - ➢ POS: POSITION AT CHROMOSOME NUMBER
- REF: WILD TYPE BASE
- ALT : BASE AFTER MUTATION
- Annotation: INFORMATION RELATED TO MUTATION

FIG. 39A                    FIG. 39B

| GENE | ID | CHROM | POS |
|------|-----|-------|-----|
| A | aaa | 19 | 10 |
| B | bbb | 20 | 3 |
| C | ccc | 21 | 4 |

⟹

```
Ex)
CHROM    POS        REF      ALT
20       3          C        G
21       4          C        CTAG
19       10         TCG      T
2        321681     G        G]17:198982]
2        321682     T        ]13:123456]T
13       123456     C        C[2:321682[
13       123457     A        [17:198983[A
```

FIG. 40

```
                    ┌──────────────┐
                    │    START     │
                    └──────┬───────┘
                           │
         ┌─────────────────┴─────────────────┐
         │ SEARCH DRUG DATABASE ON THE BASIS │ ⌐S15a
         │     OF INFORMATION RELATED TO     │
         │             MUTATION              │
         └─────────────────┬─────────────────┘
                           │
         ┌─────────────────┴─────────────────┐
         │ GENERATE LIST OF DRUG RELATED TO  │ ⌐S16a
         │             MUTATION              │
         └─────────────────┬─────────────────┘
                           │
                    ┌──────┴───────┐
                    │     END      │
                    └──────────────┘
```

FIG. 41

⌐124A

| MUTATION ID | DRUG ID | RELATED DRUG | METADATA RELATED TO GENE-PANEL-RELATED INFORMATION |
|---|---|---|---|
| . . . | | | |
| #3 | #a | DRUG A | GENE ID: a |
| #3 | #b | DRUG B | GENE ID: b |
| #4 | #d | DRUG C | GENE ID: c |
| . . . | | | |

FIG. 42

EP 3 588 508 A1

124B

| MUTATION ID | DRUG ID | RELATED DRUG | APPROVAL STATE | CDx FLAG | APPROVED COUNTRY | TARGET DISEASE ID | METADATA CORRESPONDING TO GENE-PANEL-RELATED INFORMATION |
|---|---|---|---|---|---|---|---|
| ・・・ | | | | | | | |
| #3 | #a | DRUG A | APPROVED | 1 | USA, JAPAN | #A (LUNG CANCER) | ・・・ |
| #3 | #b | DRUG B | APPROVED | 1 | USA, JAPAN | #A (LUNG CANCER) | ・・・ |
| ・・・ | | | | | | | |
| #xx | #X | xyz | UNAPPROVED | 0 | - | - | |
| #yy | #Y | abc | UNAPPROVED | 0 | - | - | |

FIG. 43

```
┌─────────────────────────┐
│          START          │
└─────────────────────────┘
             │
┌─────────────────────────┐
│ SEARCH DRUG DATABASE ON THE BASIS │ ⌐ S15b
│   OF INFORMATION RELATED TO       │
│           MUTATION                │
└─────────────────────────┘
             │
┌─────────────────────────┐
│   GENERATE LIST INCLUDING         │ ⌐ S16b
│ INFORMATION RELATED TO APPROVAL   │
│           OF DRUG                 │
└─────────────────────────┘
             │
┌─────────────────────────┐
│           END           │
└─────────────────────────┘
```

FIG. 44

START

S15b — SEARCH DRUG DATABASE ON THE BASIS OF INFORMATION RELATED TO MUTATION

S21 — SEARCHED DRUG HAS BEEN APPROVED ?

YES

S22 — PATIENT DISEASE AND TARGET DISEASE OF APPROVED DRUG MATCH EACH OTHER ?

NO

S25 — ASSOCIATE THE DRUG AS DRUG HAVING POSSIBILITY OF OFF-LABEL USE WITH MUTATION

YES

S24 — ASSOCIATE THE DRUG AS APPROVED DRUG WITH MUTATION

NO

S23 — ASSOCIATE THE DRUG AS UNAPPROVED DRUG WITH MUTATION

S16a — GENERATE LIST OF DRUG RELATED TO MUTATION

END

FIG. 44A

SAMPLE

FIG. 44B

SAMPLE

FIG. 45

124C

| MUTATION ID | DRUG ID | RELATED DRUG | CLINICAL TRIAL/CLINICAL STUDY STATE | COUNTRY | PHASE | TARGET DISEASE | INSTITUTION | METADATA RELATED TO GENE-PANEL-RELATED INFORMATION |
|---|---|---|---|---|---|---|---|---|
| ... | | | | | | | | |
| #xx | #X | xyz | IN CLINICAL TRIAL | JAPAN | Phase 3 | DISEASE ID (LUNG CANCER) | abc PHARMACY | ... |
| #yy | #Y | abc | None | - | - | | | ... |

FIG. 46

```
          ┌─────────────────┐
          │      START       │
          └─────────────────┘
                   │
   ┌───────────────────────────────────┐
   │ SEARCH DRUG DATABASE ON THE BASIS │ ┌ S15c
   │   OF INFORMATION RELATED TO        │
   │           MUTATION                 │
   └───────────────────────────────────┘
                   │
   ┌───────────────────────────────────┐
   │      GENERATE LIST INCLUDING       │ ┌ S16c
   │  INFORMATION RELATED TO CLINICAL   │
   │         TRIAL OF DRUG              │
   └───────────────────────────────────┘
                   │
          ┌─────────────────┐
          │       END        │
          └─────────────────┘
```

FIG. 47

┌ 124D

| DRUG | TARGET DISEASE | GENE | MUTATION |
|------|----------------|------|----------|
| DRUG E (DRUG ID: XXX) | LUNG CANCER (DISEASE ID: xxx) | EGFR | L747_T751del (MUTATION ID: aaa) |
| | | | L858R (MUTATION ID: bbb) |
| | | | ... |
| DRUG V (DRUG ID: YYY) | MELANOMA (DISEASE ID: yyy) | BRAF | V600E (MUTATION ID: ccc) |
| ... | ... | ... | ... |

FIG. 48

CREATE REPORT  ⌐S111

CONTRACT
OF CDx USAGE PLAN IS
CONCLUDED ?  ⌐S1111

NO

YES

PREDETERMINED
GENE MUTATION IS
DETECTED ?  ⌐S1112

NO

YES

SAMPLE
OF PREDETERMINED
DISEASE ?  ⌐S1113

NO

YES

⌐S1117
CREATE REPORT NOT
INCLUDING
INFORMATION RELATED
TO EFFICACY OF DRUG

⌐S1114
CREATE REPORT
INCLUDING
INFORMATION RELATED
TO EFFICACY OF DRUG

⌐S1115
CREATE REPORT THAT
ALLOWS DISCERNMENT
THAT NO GENE MUTATION
TO BE USED FOR CDx
HAS BEEN DETECTED

END

FIG. 49

```
          ( CREATE REPORT )  ⌐S111
                  │
                  ▼
              ╱S1111
            ╱        ╲
  NO      ╱ CONTRACT OF ╲
◄────────╱ CDx USAGE PLAN IS ╲
          ╲ CONCLUDED ?  ╱
            ╲        ╱
                │ YES
                ▼
              ╱S1112
            ╱        ╲
          ╱ PREDETERMINED ╲    NO
         ╱ GENE MUTATION IS ╲────────►
          ╲ DETECTED ?   ╱
            ╲        ╱
                │ YES
                ▼
              ╱S1113
            ╱      ╲
          ╱ SAMPLE  ╲        NO
         ╱ OF PREDETERMINED ╲────────►
          ╲  DISEASE ? ╱
            ╲      ╱
                │ YES
                ▼
              ╱S1116
            ╱    QUALITY    ╲
          ╱ EVALUATION INDEX ╲   NO
         ╱ SATISFIES PREDETERMINED ╲────┐
          ╲  CRITERION ? ╱              │
            ╲          ╱                ▼
                │ YES            ┌──────────────┐
                                 │  ⌐S1118      │
                                 │ CREATE REPORT│
                                 │ THAT ALLOWS  │
                                 │ DISCERNMENT THAT│
                                 │   QUALITY    │
                                 │ EVALUATION INDEX│
                                 │ DOES NOT SATISFY│
                                 │ PREDETERMINED│
                                 │  CRITERION   │
                                 └──────────────┘
```

| S1117 | S1114 | S1115 |
|---|---|---|
| CREATE REPORT NOT INCLUDING INFORMATION RELATED TO EFFICACY OF DRUG | CREATE REPORT INCLUDING INFORMATION RELATED TO EFFICACY OF DRUG | CREATE REPORT THAT ALLOWS DISCERNMENT THAT NO GENE MUTATION TO BE USED FOR CDx HAS BEEN DETECTED |

( END )

# FIG. 50

```
                            ┌──────────┐
                            │  START   │
                            └────┬─────┘
                                 │
                            ╱─────────╲  ⌐S1111a
                    NO    ╱             ╲
            ┌────────────╱  PANEL FOR CDx ?╲
            │            ╲                 ╱
            │             ╲               ╱
            │               ╲───────────╱
            │                    │ YES
            │                    │
            │               ╱─────────────╲  ⌐S1112
            │             ╱  PREDETERMINED  ╲    NO
            │            ╱   MUTATION IS     ╲─────────────┐
            │            ╲   DETECTED ?      ╱             │
            │             ╲                 ╱              │
            │               ╲─────────────╱                │
            │                    │ YES                     │
            │                    │                         │
            │               ╱─────────────╲  ⌐S1113        │
            │             ╱   SAMPLE OF      ╲   NO         │
            │            ╱  PREDETERMINED     ╲─────────────│──────►
            │            ╲    DISEASE ?       ╱             │
            │             ╲                 ╱              │
            │               ╲─────────────╱                │
            │                    │ YES                     │
   ⌐S1117   │            ⌐S1114   │                 ⌐S1115  │
┌───────────────────┐  ┌───────────────────┐  ┌──────────────────────┐
│  CREATE REPORT NOT│  │   CREATE REPORT   │  │   CREATE REPORT THAT  │
│    INCLUDING      │  │     INCLUDING     │  │  ALLOWS DISCERNMENT   │
│INFORMATION RELATED│  │INFORMATION RELATED│  │ THAT NO GENE MUTATION │
│TO EFFICACY OF DRUG│  │TO EFFICACY OF DRUG│  │ TO BE USED FOR CDx HAS│
│                   │  │                   │  │      BEEN DETECTED    │
└─────────┬─────────┘  └─────────┬─────────┘  └──────────┬───────────┘
          │                      │                       │
          └──────────────────────┼───────────────────────┘
                                 │
                            ┌────┴─────┐
                            │   END    │
                            └──────────┘
```

# FIG. 51

FIG. 52

### GENE ANALYSIS RESULT REPORT

PATIENT ID: ▓▓▓▓▓▓

SEX: MALE

DISEASE NAME: xxx CANCER STAGE II

NAME OF DOCTOR IN CHARGE: ▓▓▓▓▓▓

INSTITUTION NAME: ▓▓▓▓▓

GENE PANEL: PANEL A

QC INDEX: XXXXXXX

REQUEST DAY: × ×YEAR ×MONTH × ×DAY

MEASUREMENT DAY: × ×YEAR ×MONTH × ×DAY

REPORT DAY: × ×YEAR ×MONTH × ×DAY

#### CDx USAGE

| GENE | MUTATION | AMINO ACID MUTATION | NUCLEOTIDE MUTATION | RELATED DRUG |
|------|----------|---------------------|---------------------|--------------|
| EGFR | L858R | p. Leu858Arg | c. 2573T>G | DRUG G |

#### NON CDx USAGE

| GENE | MUTATION | AMINO ACID MUTATION | NUCLEOTIDE MUTATION | RELATED DRUG |
|------|----------|---------------------|---------------------|--------------|
| BRAF | V600E | p. Val600Glu | c. 1799T>A | DRUG D |
| XXXXX | XXXXX | XXXXX | XXXX | XXXXX |

FIG. 53

GENE ANALYSIS RESULT REPORT

PATIENT ID: ▮▮▮▮▮▮

SEX: MALE

DISEASE NAME: xxx CANCER  STAGE Ⅱ

NAME OF DOCTOR IN CHARGE: ▮▮▮▮▮

INSTITUTION NAME: ▮▮▮▮▮

GENE PANEL: PANEL A

QC INDEX: XXXXXXX

REQUEST DAY:      × ×YEAR ×MONTH × ×DAY

MEASUREMENT DAY: × ×YEAR ×MONTH × ×DAY

REPORT DAY:      × ×YEAR ×MONTH × ×DAY

| | GENE | MUTATION | AMINO ACID MUTATION | NUCLEOTIDE MUTATION | RELATED DRUG |
|---|---|---|---|---|---|
| CDx | EGFR | L858R | p. Leu858Arg | c. 2573T>G | DRUG G |
| | BRAF | V600E | p. Val600Glu | c. 1799T>A | DRUG D |
| | XXXXX | XXXXX | XXXXX | XXXX | XXXXX |

FIG. 54

NON CDx USAGE

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | C.1799T>A | DRUG D |
| | | | xxxx | xxxx |

CDx USAGE

| GENE | MUTATION | AMINO ACID MUTATION | NUCLEOTIDE MUTATION | RELATED DRUG |
|------|----------|---------------------|---------------------|--------------|
| EGFR | L858R | p.Leu858Arg | c.2573T>G | DRUG G |

−4−

−3−

## FIG. 55

GENE ANALYSIS RESULT REPORT

PATIENT ID: ▮▮▮▮▮▮

SEX: MALE

DISEASE NAME: xxx CANCER   STAGE Ⅱ

NAME OF DOCTOR IN CHARGE: ▮▮▮▮▮▮

INSTITUTION NAME: ▮▮▮▮▮▮

GENE PANEL: PANEL A

QC INDEX: XXXXXXX

REQUEST DAY:       × ×YEAR ×MONTH × ×DAY

MEASUREMENT DAY: × ×YEAR ×MONTH × ×DAY

REPORT DAY:        × ×YEAR ×MONTH × ×DAY

CDx USAGE (*)

| GENE | MUTATION | AMINO ACID MUTATION | NUCLEOTIDE MUTATION | RELATED DRUG |
|------|----------|---------------------|---------------------|--------------|
| EGFR | L858R | p.Leu858Arg | c.2573T>G | DRUG G |

(*) THIS ANALYSIS RESULT IS FOR ASSISTING SELECTION OF A PATIENT
FOR WHOM DRUG G MAY BE EFFECTIVE.

NON CDx USAGE

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | c.1799T>A | DRUG D |
| xxxxx | xxxxx | xxxxx | xxxx | xxxxx |

FIG. 56

## GENE ANALYSIS RESULT REPORT

PATIENT ID: ███████

SEX: MALE

DISEASE NAME: xxx CANCER  STAGE Ⅱ

NAME OF DOCTOR IN CHARGE: ████████

INSTITUTION NAME: ███████

GENE PANEL: PANEL A

QC INDEX: XXXXXXX

REQUEST DAY:　　×× YEAR × MONTH ×× DAY

MEASUREMENT DAY: ×× YEAR × MONTH ×× DAY

REPORT DAY:　　　×× YEAR × MONTH ×× DAY

<u>CDx</u>

NO TARGET GENE MUTATION IS DETECTED.

<u>NON CDx USAGE</u>

| GENE | MUTATION | AMINO ACID MUTATION | NUCLEOTIDE MUTATION | RELATED DRUG |
|------|----------|---------------------|---------------------|--------------|
| BRAF | V600E | p. Val600Glu | c. 1799T>A | DRUG D |
| XXXXX | XXXXX | XXXXX | XXXX | XXXXX |

# FIG. 57

GENE ANALYSIS RESULT REPORT

PATIENT ID: ▮▮▮▮▮▮

SEX: MALE

DISEASE NAME: xxx CANCER  STAGE Ⅱ

NAME OF DOCTOR IN CHARGE: ▮▮▮▮▮▮

INSTITUTION NAME: ▮▮▮▮▮

GENE PANEL: PANEL A

QC INDEX: XXXXXXX

REQUEST DAY:      ××YEAR ×MONTH ××DAY

MEASUREMENT DAY: ××YEAR ×MONTH ××DAY

REPORT DAY:       ××YEAR ×MONTH ××DAY

## NON CDx USAGE

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | c.1799T>A | DRUG D |
| xxxxx | xxxxx | xxxxx | xxxx | xxxxx |

FIG. 58

GENE ANALYSIS RESULT REPORT

PATIENT ID: ████████

SEX: MALE

DISEASE NAME: xxx CANCER  STAGE II

NAME OF DOCTOR IN CHARGE: ████████

INSTITUTION NAME: ██████

GENE PANEL: PANEL A

QC INDEX: XXXXXXX

REQUEST DAY:      × ×YEAR ×MONTH × ×DAY

MEASUREMENT DAY: × ×YEAR ×MONTH × ×DAY

REPORT DAY:       × ×YEAR ×MONTH × ×DAY

QC INDEX OF TEST INCLUDES ITEM NOT SATISFYING CRITERION. THIS TEST RESULT IS REFERENCE INFORMATION.

CDx USAGE

| GENE | MUTATION | AMINO ACID MUTATION | NUCLEOTIDE MUTATION | RELATED DRUG |
|------|----------|---------------------|---------------------|--------------|
| EGFR | L858R | p.Leu858Arg | c.2573T>G | DRUG G |

NON CDx USAGE

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | c.1799T>A | DRUG D |
| XXXXX | XXXXX | XXXXX | XXXX | XXXXX |

# FIG. 59

## GENE ANALYSIS RESULT REPORT

PATIENT ID: ███████

SEX: MALE

DISEASE NAME: xxx CANCER  STAGE II

NAME OF DOCTOR IN CHARGE: ███████

INSTITUTION NAME: ███████

GENE PANEL: PANEL A

QC INDEX: XXXXXXX

REQUEST DAY:      × ×YEAR ×MONTH × ×DAY

MEASUREMENT DAY: × ×YEAR ×MONTH × ×DAY

REPORT DAY:      × ×YEAR ×MONTH × ×DAY

QC INDEX OF TEST INCLUDES ITEM NOT SATISFYING CRITERION.

### CDx USAGE

| GENE | MUTATION | AMINO ACID MUTATION | NUCLEOTIDE MUTATION | RELATED DRUG |
|------|----------|---------------------|---------------------|--------------|
| – | – | – | – | – |

### NON CDx USAGE

| GENE | MUTATION | AMINO ACID MUTATION | NUCLEOTIDE MUTATION | RELATED DRUG |
|------|----------|---------------------|---------------------|--------------|
| – | – | – | – | – |

FIG. 60

GENE ANALYSIS RESULT REPORT

PATIENT ID: ███████

SEX: MALE

DISEASE NAME: xxx CANCER   STAGE Ⅲ

NAME OF DOCTOR IN CHARGE: ████████

INSTITUTION NAME: ██████

GENE PANEL: PANEL C

QC INDEX: XXXXXXX

REQUEST DAY:     × ×YEAR ×MONTH × ×DAY

MEASUREMENT DAY: × ×YEAR ×MONTH × ×DAY

REPORT DAY:      × ×YEAR ×MONTH × ×DAY

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | c.1799T>A | DRUG D |
| xxxxx | xxxxx | xxxxx | xxxx | xxxxx |

FIG. 61

```
┌──────────────────────────────────────────────┐
│                                                │
│     TEST INSTITUTION ID                        │
│     ┌──────────────────────────────────┐       │
│     │ XXXX                             │       │
│     └──────────────────────────────────┘       │
│     PASSWORD                                    │
│     ┌──────────────────────────────────┐       │
│     │ *******                          │       │
│     └──────────────────────────────────┘       │
│                                                │
│                    ┌──────────┐                │
│                    │  LOG IN  │                │
│                    └──────────┘                │
│                                                │
└──────────────────────────────────────────────┘
```

FIG. 62

```
┌──────────────────────────────────────────────┐
│                                                │
│   (*) THIS REPORT IS NOT FOR CDx USAGE.        │
│                                                │
│                                                │
│                                                │
│                        .                       │
│                        .                       │
│                        .                       │
│                                                │
│                                                │
│                                                │
└──────────────────────────────────────────────┘
```

FIG. 63

Europäisches
Patentamt

European
Patent Office

Office européen
des brevets

# EUROPEAN SEARCH REPORT

Application Number

EP 19 18 2564

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/049250 A1 (CUREMATCH INC [US]) 15 March 2018 (2018-03-15) | 1-14,16, 18-21 | INV. G16H15/00 |
| Y | * claims 40-98; examples 1,2,4 and 5; Figures 1A-1C, 9B, 10, 12 and 13; paragraphs 42 and 175 * | 12-14 | G16H20/10 G16H70/40 |
| X | WO 2010/028288 A2 (AUEON INC [US]; STEPHAN DIETRICH [US] ET AL.) 11 March 2010 (2010-03-11) | 1-3,7,8, 16 | |
| Y | * whole document, in particular claims, Figures and paragraphs 31 and 103 * | 12-14 | |
| X,P | EP 3 476 946 A1 (SYSMEX CORP [JP]) 1 May 2019 (2019-05-01) * whole document, in particular Figures 2, 3, 28; paragraphs [0215]-[0216], [0231]-[0233] * | 1-21 | |
| X,P | CN 109 063 420 A (MAQGEN GENE TECH CO LTD) 21 December 2018 (2018-12-21) * the whole document * | 1-21 | |

TECHNICAL FIELDS
SEARCHED (IPC)

G16H
G06F

The present search report has been drawn up for all claims

| Place of search | Date of completion of the search | Examiner |
|---|---|---|
| Munich | 14 November 2019 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................
& : member of the same patent family, corresponding document

EPO FORM 1503 03.82 (P04C01)

## EP 3 588 508 A1

### ANNEX TO THE EUROPEAN SEARCH REPORT
### ON EUROPEAN PATENT APPLICATION NO.

EP 19 18 2564

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2019

| Patent document cited in search report | | Publication date | Patent family member(s) | | Publication date |
|---|---|---|---|---|---|
| WO 2018049250 | A1 | 15-03-2018 | EP | 3510175 A1 | 17-07-2019 |
| | | | US | 2019233895 A1 | 01-08-2019 |
| | | | WO | 2018049250 A1 | 15-03-2018 |
| WO 2010028288 | A2 | 11-03-2010 | EP | 2318552 A2 | 11-05-2011 |
| | | | EP | 3216874 A1 | 13-09-2017 |
| | | | GB | 2467691 A | 11-08-2010 |
| | | | US | 2011230360 A1 | 22-09-2011 |
| | | | US | 2014080733 A1 | 20-03-2014 |
| | | | US | 2014141426 A1 | 22-05-2014 |
| | | | US | 2014141980 A1 | 22-05-2014 |
| | | | US | 2016115553 A1 | 28-04-2016 |
| | | | US | 2016115554 A1 | 28-04-2016 |
| | | | US | 2016122830 A1 | 05-05-2016 |
| | | | US | 2016362749 A1 | 15-12-2016 |
| | | | US | 2019271044 A1 | 05-09-2019 |
| | | | WO | 2010028288 A2 | 11-03-2010 |
| EP 3476946 | A1 | 01-05-2019 | CN | 109722470 A | 07-05-2019 |
| | | | EP | 3476946 A1 | 01-05-2019 |
| | | | JP | 2019080501 A | 30-05-2019 |
| | | | US | 2019127807 A1 | 02-05-2019 |
| CN 109063420 | A | 21-12-2018 | NONE | | |

EPO FORM P0459

For more details about this annex : see Official Journal of the European Patent Office, No. 12/82

**REFERENCES CITED IN THE DESCRIPTION**

**Patent documents cited in the description**

- JP 2015200678 A